# EUROPÄISCHE PATENTSCHRIFT

(11) **EP 0 842 945 B1**
(45) Veröffentlichungstag und Bekanntmachung des Hinweises auf die Patenterteilung: **19.02.2003**
(21) Anmeldenummer: 97119636.5
(22) Anmeldetag: 10.11.1997
(51) Int. Cl.: C07K 5/117, A61K 38/07

(54) **Heterocyclen als Inhibitoren der Leukozytenadhäsion und VLA-4-Antagonisten**
Heterocycles as inhibitors of leukocyte adhesion and as antagonists of VLA-4
Hétérocycles à titre d'inhibiteurs d'adhésion des leucocytes et d'antagonistes de VLA-4

(30) Priorität: 15.11.1996 DE 19647381
(43) Veröffentlichungstag der Anmeldung: 20.05.1998
(73) Patentinhaber: HOECHST AKTIENGESELLSCHAFT, 65929 Frankfurt am Main (DE)
(72) Erfinder: Stilz, Hans Ulrich, Dr., 65929 Frankfurt (DE); Wehner, Volkmar, Dr., 97657 Sandberg (DE); Hüls, Christoph, Dr., 55263 Wackernheim (DE); Seiffge, Dirk, Dr., 55246 Mainz-Kostheim (DE)

(56) Entgegenhaltungen:
- EP-A- 0 580 008
- WO-A-95/14008
- WO-A-95/15973
- WO-A-96/22966

## Beschreibung

Gegenstand der vorliegenden Erfindung sind Verbindungen der Formel I, 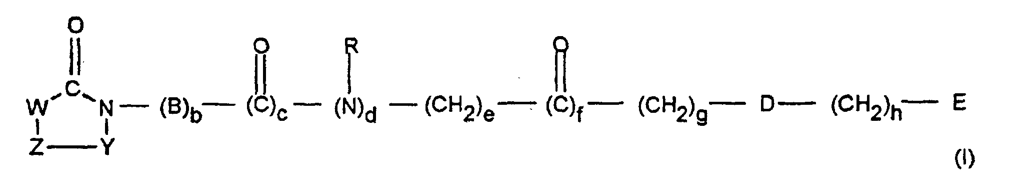 die Inhibitoren der Adhäsion und Migration von Leukozyten und/oder Antagonisten des zur Gruppe der Integrine gehörenden Adhäsionsrezeptors VLA-4 sind. Die Erfindung betrifft auch Verfahren zur Herstellung, die Verwendung von Verbindungen der Formel I zur Behandlung oder Prophylaxe von Krankheiten, die durch ein unerwünschtes Ausmaß an Leukozytenadhäsion und/oder Leukozytenmigration verursacht werden oder die damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen, der rheumatoiden Arthritis oder von allergischen Erkrankungen, sowie die Verwendung von Verbindungen der Formel I zur Herstellung von Arzneimitteln zur Anwendung bei solchen Krankheiten und pharmazeutische Zubereitungen, die Verbindungen der Formel I enthalten.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine αß-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertetern LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin II-Bindungsfragment des Plasmafibronektins beruht vor allem auf einer Interaktion mit einer LDVP-Sequenz. Im Unterschied zum Fibrinogen- oder Vitronectin-Rezeptor ist VLA-4 kein typisches RGD-bindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1; auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osborn et al., Cell 1989, 59, 1203). VCAM-1 ist, wie ICAM-1, ein Mitglied der Immunglobulin-Gen-Überfamilie. Identifiziert wurde VCAM-1 (zuerst bekannt als INCAM-110) als ein Adhäsionsmolekül, daß auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligand-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1/VLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 (α4β1) tragen, wie Eosinophile, T- und B-Lymphozyten, Monozyten oder auch Neutrophile, kommt ihm und dem VCAM-1/ VLA-4-Mechanismus die Funktion zu, derartige Zellen aus dem Blutstrom in Infektionsgebiete und Entzündungsherde zu rekrutieren (Elices et al., Cell 1990, 60, 577; Osborn, Cell 1990, 62, 3; Issekutz et al., J. Exp. Med. 1996, 183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertem Endothel unter anderem noch von den folgenden Zellen exprimiert: Myoblasten, lymphoiden dendritischen Zellen und Gewebsmakrophagen, rheumatoidem Synovium, Zytokin-stimulierten Neuralzellen, parietalen Epithelzellen der Bowmans Kapsel, dem renalen Tubularepithel, entzündetem Gewebe bei Herz- und Nieren-Transplantat-Abstoßung und von Intestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 auf follikulären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1/VLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben Immunglobulin-Domänen. Die Domänen 4, 5 und 6 ähneln in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995, 16, 379.

Aufgrund der Rolle des VCAM-1/VLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osborn et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen.

Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die α4-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. Immunol. 1991, 147, 4207).

In vivo-Versuche haben gezeigt, daß eine experimentelle autoimmune Enzephalomyelitis durch anti-α4 mAK gehemmt werden kann. Die Wanderung von Leukozyten in einen Entzündungsherd wird ebenfalls durch einen monoklonalen Antikörper gegen die α4-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (WO-A-93/13798; EP-A-626 861). Die Gabe von anti-VLA-4-Antikörpern inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpern eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176, 1183), Arteriosklerose (Obrien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osborn, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4A/CAM-1-Adhäsionsmechanismen (Springer, Cell 1994, 76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. Immunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93/13798, WO-A-93/15764, WO-A-94/16094, WO-A-94/17828 und WO-A-95/19790 beschrieben. In den Patentanmeldungen WO-A-94/15958, WO-A-95/15973, WO-A-96/00581, WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung, und es besteht somit Bedarf nach VLA-4-Antagonisten mit einem günstigen Eigenschaftsprofil für einen Einsatz in der Therapie und Prophylaxe. Bestimmte nicht-peptidische Verbindungen mit VLA-4-inhibitorischer Wirkung sind bereits in der WO-A-96122966 beschrieben.

In der EP-A-580 008 sind Phenylimidazolidin-Derivate und in der WO-A-95/14008 substituierte 5-Ring-Heterocyclen beschrieben, die am N-terminalen Ende des Moleküls eine Amino-, Amidino- oder Guanidinofunktion aufweisen und die thrombozytenaggregationshemmende Wirkungen zeigen. In der DE-A-19635522 sind Heterocyclen beschrieben, die Inhibitoren der Knochenresorption sind. In der WO-A-96/33976 und der DE-A-19515177 werden bestimmte Hydantoinderivate mit einem 4-Cyanophenylrest in der 4-Position der Hydantoinrings beschrieben, die Zwischenprodukte für die Herstellung von Wirkstoffen sind, die in der WO-A-95/14008 beschrieben sind. Pharmakologische Wirkungen dieser Cyanophenylhydantoinderivate werden aber nicht offenbart. Die vorliegende Erfindung betrifft weitere heterocyclische Verbindungen, die VLA-4-Antagonisten und/oder Inhibitoren der Leukozytenadhäsion sind.

Gegenstand der vorliegenden Anmeldung sind Verbindungen der Formel I, 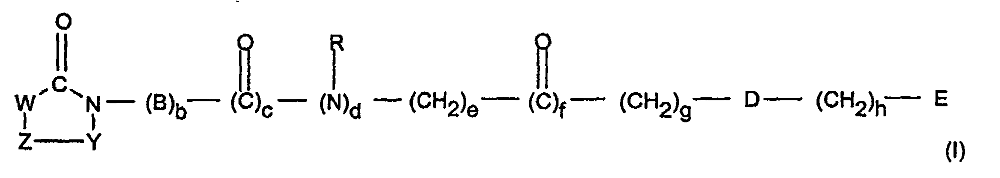 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor ein- oder mehrfach substituiert sein können;
- R⁰: für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 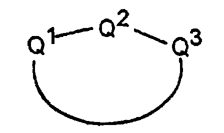 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)(C₁-C₁₈)-Alkoxycarbonyl, Het-CO, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: für Wasserstoff, R^{12a}, R^{12a}-CO, H-CO, R^{12a}-O-CO, R^{12b}-CO, R^{12b}-CS, R^{12a}-S(O)₂ oder R^{12b}-S(O)₂ steht;
- R^{12a}: (C₁-C₁₈)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
- R^{12b}: Amino, Di-((C₁-C₁₈)-alkyl)-amino oder R^{12a}-NH bedeutet;
- R¹³: Wasserstoff, (C₁-C₆)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder (C₃-C₈)-Cycloalkyl-(C₁-C₈)-alkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
- R²⁸: für einen der Reste R²¹-, R²¹N(R²¹)-, R²¹C(O)-, R²²O-C(O)-, R²¹N(R²¹)-C(O)- oder R²¹N(R²¹)-C=(N(R²¹))- steht;
- R²⁹: für einen der Reste R²²-, R²¹N(R²¹)-, R²¹C(O)-, R²²O-C(O)-, R²¹N(R²¹)-C(O)- oder R²¹N(R²¹)-C(=N(R²¹))- steht;
- Het: für den Rest eines über ein Stickstoffatom gebundenen 5- bis 10-gliedrigen, monocyclischen oder polycyclischen Heterocyclus steht, der aromatisch oder teilweise ungesättigt oder gesättigt sein kann und der ein, zwei, drei oder vier gleiche oder verschiedene zusätzliche Ring-Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring-Stickstoffatomen gleiche oder verschiedene Reste R^{h}, R^{h}CO oder R^{h}O-CO als Substituenten stehen können und R^{h} für Wasserstoff, (C₁-C₈)-Alkyl, (C₃-C₈)-Cycloalkyl, (C₃-C₈)-Cycloalkyl-(C₁-C₈)-alkyl, gegebenenfalls substituiertes (C₆-C₁₄)-Aryl oder im Arylrest gegebenenfalls substituiertes (C₆-C₁₄)-Aryl-(C₁-C₈)-alkyl steht;
b, c, d und f unabhängig voneinander für 0 oder 1 stehen, aber nicht alle gleichzeitig
0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze;
wobei aber, wenn gleichzeitig W für 4-Cyanophenyl-C(R¹³) steht, Y für eine Carbonylgruppe steht, Z für NR^{0a} steht, B für eine unsubstituierte Methylengruppe steht, R für R^{a} steht, b, c und d für 1 stehen und e, f und g für 0 stehen, dann nicht
D für C(R^{2a})(R^{3a}) stehen kann, wobei
- R^{0a}, R^{a} und R^{2a}: unabhängig voneinander für Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl stehen und
- R^{3a}: für Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder 2-, 3- oder 4-Pyridyl steht.

Alkylreste können geradkettig oder verzweigt sein. Dies gilt auch, wenn sie Substituenten tragen oder als Substituenten anderer Reste auftreten, beispielsweise in Alkoxy-, Alkoxycarbonyl- oder Aralkylresten. Entsprechendes gilt für Alkylenreste. Beispiele für geeignete (C₁-C₂₈)-Alkylreste sind: Methyl, Ethyl, Propyl, Butyl, Pentyl, Hexyl, Heptyl, Octyl, Decyl, Undecyl, Dodecyl, Tridecyl, Pentadecyl, Hexadecyl, Heptadecyl, Nonadecyl, Eicosyl, Docosyl, Tricosyl, Pentacosyl, Hexacosyl, Heptacosyl, Octacosyl, Isopropyl, Isobutyl, Isopentyl, Neopentyl, Isohexyl, 3-Methylpentyl, 2,3,5-Trimethylhexyl, sec-Butyl, tert-Butyl, tert-Pentyl. Bevorzugte Alkylreste sind Methyl, Ethyl, Propyl, Isopropyl, Butyl, Isobutyl, sec-Butyl und tert-Butyl. Beispiele für Alkylenreste sind Methylen, Ethylen, Tri-, Tetra-, Penta- und Hexamethylen oder durch einen Alkylrest substituiertes Methylen, zum Beispiel Methylen, das durch eine Methylgruppe, eine Ethylgruppe, eine Isopropylgruppe, eine Isobutylgruppe oder eine tert-Butylgruppe substituiert ist.

Auch Alkenyl- und Alkenylenrest sowie Alkinylreste können geradkettig oder verzweigt sein. Beispiele für Alkenylreste sind Vinyl, 1-Propenyl, Allyl, Butenyl, 3-Methyl-2-butenyl, für Alkenylenreste Vinylen oder Propenylen, für Alkinylreste Ethinyl, 1-Propinyl oder Propargyl.

Cycloalkylreste sind insbesondere Cyclopropyl, Cyclobutyl, Cyclopentyl, Cyclohexyl, Cycloheptyl, Cyclooctyl, Cyclononyl, Cyclodecyl, Cycloundecyl und Cyclododecyl, die aber auch durch beispielsweise durch (C₁-C₄)-Alkyl substituiert sein können. Als Beispiele für substituierte Cycloalkylreste seien 4-Methylcyclohexyl und 2,3-Dimethylcyclopentyl genannt. Analoges gilt für Cycloalkylenreste.

Die für R¹⁶ stehenden 6- bis 24-gliedrigen bicyclischen und tricyclischen Reste werden formal durch Abstraktion eines Wasserstoffatoms aus Bicyclen bzw. Tricyclen erhalten. Die zugrunde liegenden Bicyclen und Tricyclen können als Ringglieder nur Kohlenstoffatome enthalten, es kann sich also um Bicycloalkane oder Tricycloalkane handeln, sie können aber auch ein, zwei, drei oder vier gleiche oder verschiedene Heteroatome aus der Reihe Stickstoff, Sauerstoff und Schwefel enthalten, es kann sich also um Aza-, Oxa- und Thiabicyclo- und -tricycloalkane handeln. Sind Heteroatome enthalten, so sind bevorzugt ein oder zwei Heteroatome, insbesondere Stickstoff- oder Sauerstoffatome, enthalten. Die Heteroatome können beliebige Positionen im bi- bzw. tricyclischen Gerüst einnehmen, sie können sich in den Brücken oder im Falle von Stickstoffatomen auch an den Brückenköpfen befinden. Sowohl die Bicyclo- und Tricycloalkane als auch ihre Hetero-Analoga können vollständig gesättigt sein oder eine oder mehrere Doppelbindungen enthalten; bevorzugt enthalten sie eine oder zwei Doppelbindungen oder sind insbesondere vollständig gesättigt. Sowohl die Bicyclound Tricycloalkane als auch die Hetero-Analoga und sowohl die gesättigten als auch die ungesättigten Vertreter können unsubstituiert sein oder in beliebigen geeigneten Positionen durch eine oder mehrere Oxogruppen und/oder eine oder mehrere gleiche oder verschiedene (C₁-C₄)-Alkylgruppen, zum Beispiel Methyloder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Beispiele für Grundkörper bicyclischer Ringsysteme, von denen sich ein bicyclischer Rest ableiten kann, sind das Norbornan (= Bicyclo[2.2.1]heptan), das Bicyclo[2.2.2]octan und das Bicyclo[3.2.1]octan, Beispiele für Heteroatome enthaltende Systeme, ungesättigte oder substituierte Systeme sind das 7-Azabicylo[2.2.1]heptan, das Bicyclo[2.2.2.]oct-5-en und der Campher (= 1,7,7-Trimethyl-2-oxoblcyclo[2.2.1]heptan).

Beispiele für Systeme, von denen sich ein tricyclischer Rest ableiten kann, sind das Twistan (= Tricyclo[4.4.0.0^{3,8}]decan), das Adamantan (= Tricyclo[3.3.1.1^{3,7}]decan), das Noradamantan (= Tricyclo[3.3.1.0^{3,7}]nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Bevorzugt leiten sich für R¹⁶ stehende bicyclische oder tricyclische Reste von verbrückten Bicyclen bzw. Tricyclen ab, also von Systemen, in denen Ringe zwei oder mehr als zwei Atome gemeinsam haben. Bevorzugt sind weiterhin auch bicyclische oder tricyclische Reste mit 6 bis 18 Ringgliedern, besonders bevorzugt solche mit 7 bis 12 Ringgliedern.

Im einzelnen besonders bevorzugte bi- und tricyclische Reste sind der 2-Norbornylrest, sowohl derjenige mit der freien Bindung in der exo-Position als auch derjenige mit der freien Bindung in der endo-Position, der 2-Bicyclo[3.2.1]octylrest, der 1-Adamantylrest, der 2-Adamantylrest und der Noradamantylrest, zum Beispiel der 3-Noradamantylrest. Darüber hinaus bevorzugt sind der 1- und der 2-Adamantylrest.

(C₆-C₁₄)-Arylgruppen sind beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei- oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Ethylendioxy, Cyano, Hydroxycarbonyl, Aminocarbonyl, (C₁-C₄)-Alkoxycarbonyl, Phenyl, Phenoxy, Benzyl, Benzyloxy, (R⁸O)₂P(O), (R⁸O)₂P(O)-O-, Tetrazolyl substituiert sein. Entsprechendes gilt beispielsweise für Reste wie Aralkyl oder Arylcarbonyl. Aralkylreste sind insbesondere Benzyl sowie 1- und 2-Naphthylmethyl, 2-, 3- und 4-Biphenylylmethyl und 9-Fluorenylmethyl, die auch substituiert sein können. Substituierte Aralkylreste sind beispielsweise durch einen oder mehrere (C₁-C₈)-Alkylreste, insbesondere (C₁-C₄)-Alkylreste, im Arylteil substituiertes Benzyl und Naphthylmethyl, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere (C₁-C₈)-Alkoxyreste, insbesondere (C₁-C₄)-Alkoxyreste, im Arylteil substituiertes Benzyl und Naphthylmethyl, zum Beispiel 4-Methoxybenzyl, 4-Neopentyloxybenzyl, 3,5-Dimethoxybenzyl, 3,4-Methylendioxybenzyl, 2,3,4-Trimethoxybenzyl, weiter 2-, 3- und 4-Nitrobenzyl, Halobenzyl, zum Beispiel 2-, 3- und 4-Chlor- und 2-, 3-, und 4-Fluorbenzyl, 3,4-Dichlorbenzyl, Pentafluorbenzyl, Trifluormethylbenzyl, zum Beispiel 3- und 4-Trifluormethylbenzyl oder 3,5-Bis(trifluormethyl)benzyl. Substituierte Aralkylreste können aber auch unterschiedliche Substitutenten aufweisen. Beispiele für Pyridyl sind 2-Pyridyl, 3-Pyridyl und 4-Pyridyl.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Zweifach substituiertes Phenyl kann also in der 2,3-Position, der 2,4-Position, der 2,5-Position, der 2,6-Position, der 3,4-Position oder der 3,5-Position, bezogen auf die Verknüpfungstelle, substituiert sein. Bevorzugt sind in zweifach substituierten Phenylresten die beiden Substituenten in der 3-Position und der 4-Position, bezogen auf die Verknüpfungsstelle, angeordnet. In dreifach substituierten Phenylresten können sich die Substituenten beispielsweise in der 2,3,4-Position, der 2,3,5-Position, der 2,4,5-Position, der 2,4,6-Position, der 2,3,6-Position oder der 3,4,5-Position befinden. Entsprechendes gilt für Phenylenreste, die zum Beispiel als 1,4-Phenylen oder als 1,3-Phenylen vorliegen können.

Phenylen-(C₁-C₆)-alkyl ist insbesondere Phenylenmethyl (-C₆H₄-CH₂-) und Phenylenethyl, (C₁-C₆)-Alkylen-phenyl insbesondere Methylenphenyl (-CH₂-C₆H₄-). Phenylen-(C₂-C₆)-alkenyl ist insbesondere Phenylenethenyl und Phenylenpropenyl.

Heteroaryl steht für einen mono- oder polycyclischen aromatischen Rest mit 5 bis 14 Ringgliedern, der 1 bis 5 Heteroatome als Ringglieder enthält. Beispiele für Heteroatome sind N, O und S. Sind mehrere Heteroatome enthalten, können diese gleich oder verschieden sein. Heteroarylreste können ebenfalls ein- oder mehrfach, bevorzugt ein-, zwei- oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Ethylendioxy, Cyano, Hydroxycarbonyl, Aminocarbonyl, (C₁-C₄)-Alkoxycarbonyl, Phenyl, Phenoxy, Benzyl, Benzyloxy, (R⁸O)₂P(O), (R⁸O)₂P(O)-O-, Tetrazolyl substituiert sein. Bevorzugt steht Heteroaryl für einen mono- oder bicyclischen aromatischen Rest, der 1, 2, 3, oder 4, insbesondere 1 bis 3, gleiche oder verschiedene Heteroatome aus der Reihe N, O und S enthält und der durch 1, 2, 3 oder 4, insbesondere 1 bis 3, gleiche oder verschiedene Substituenten aus der Reihe (C₁-C₆)-Alkyl, (C₁-C₆)-Alkoxy, Fluor, Chlor, Nitro, Amino, Trifluormethyl, Hydroxy, (C₁-C₄)(C₁-C₄)-Alkoxycarbonyl, Phenyl, Phenoxy, Benzyloxy und Benzyl substituiert sein kann. Besonders bevorzugt steht Heteroaryl für einen mono- oder bicyclischen aromatischen Rest mit 5 bis 10 Ringgliedern, insbesondere einen 5-bis 6-gliedrigen monocyclischen aromatischen Rest, der 1, 2 oder 3, insbesondere 1 oder 2, gleiche oder verschiedene Heteroatome aus der Reihe N, O und S enthält und durch 1 oder 2 gleiche oder verschiedene Substituenten aus der Reihe (C₁-C₄)-Alkyl, (C₁-C₄)-Alkoxy, Phenyl, Phenoxy, Benzyloxy und Benzyl substituiert sein kann.

Heterocyclische Ringe der Formel 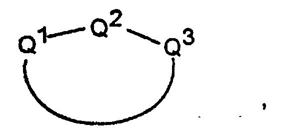 die für die Gruppe R¹ stehen können, können im Ring vollständig gesättigt sein oder eine oder mehrere Doppelbindungen enthalten. Neben den Substituenten in den Gruppen Q¹ und Q³ können diese heterocyclischen Ringe noch einen oder mehrere weitere, gleiche oder verschiedene Reste R²¹ und/oder Halogenatome als Substituenten tragen. Die in der Definition von Q³ enthaltenen Bedeutungen ―C(R²¹)(―)― und ―N(―)― sind so zu verstehen, daß zwei der drei freien Bindungen des Kohlenstoffatoms bzw. des Stickstoffatoms zu den benachbarten Ringgliedern gerichtet sind und die dritte freie Bindung zur Gruppe A gerichtet ist, so daß in diesem Fall also der heterocyclische Ring über die Gruppe Q³ an die Gruppe A gebunden ist. Ist der heterocyclische Ring über zwei benachbarte Atome an das Ringsystem in der Gruppe A anelliert, so liegt ein kondensiertes Ringsystem vor. Die Anellierung kann über die Gruppe Q³ und das benachbarte Kohlenstoffatom oder über zwei beliebige benachbarte Kohlenstoffatome erfolgen. Beispiele für derartige kondensierte Ringsysteme sind die Reste 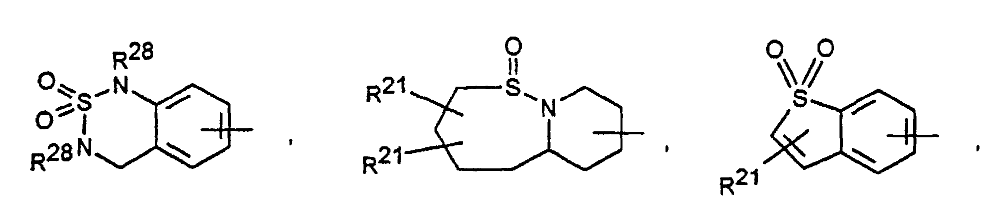 die dann für die Gruppierung R¹-A in der Formel I stehen.

Heterocyclen, die für die in der Definition von R⁵ aufgeführten mono- oder bicyclischen 5- bis 12-gliedrigen heterocyclischen Ringe stehen, können aromatisch oder teilweise oder vollständig gesättigt sein und können insbesondere an einem Stickstoffatom durch (C₁-C₇)-Alkyl, zum Beispiel Methyl oder Ethyl, Phenyl oder Phenyl-(C₁-C₄)-alkyl, zum Beispiel Benzyl, und/oder an einem oder mehreren Kohlenstoffatomen durch (C₁-C₄)-Alkyl, Halogen, Hydroxy, (C₁-C₄)-Alkoxy, zum Beispiel Methoxy, Phenyl-(C₁-C₄)-alkoxy, zum Beispiel Benzyloxy, oder Oxo substituiert sein.

Beispiele für Heterocyclen, die der Gruppe Heteroaryl oder dem mono- oder bicyclischen 5- bis 12-gliedrigen heterocyclischen Ring zugrunde liegen können, sind Pyrrol, Furan, Thiophen, Imidazol, Pyrazol, Oxazol, Isoxazol, Thiazol, Isothiazol, Tetrazol, Pyridin, Pyrazin, Pyrimidin, Indol, Isoindol, Indazol, Phthalazin, Chinolin, Isochinolin, Chinoxalin, Chinazolin, Cinnolin, β-Carbolin oder benzanellierte, cyclopenta-, cyclohexa- oder cyclohepta-anellierte Derivate dieser Heterocyclen. Stickstoffheterocyclen können auch als N-Oxide vorliegen.

Reste, die für Heteroaryl oder den Rest eines mono- oder bicyclischen 5- bis 12-gliedrigen heterocyclischen Rings stehen können, sind beispielsweise 2- oder 3-Pyrrolyl, Phenylpyrrolyl, zum Beispiel 4- oder 5-Phenyl-2-pyrrolyl, 2-Furyl, 2-Thienyl, 4-Imidazolyl, Methylimidazolyl, zum Beispiel 1-Methyl-2-, -4- oder -5-imidazolyl, 1,3-Thiazol-2-yl, 2-, 3- oder 4-Pyridyl, 2-, 3- oder 4-Pyridyl-N-oxid, 2-Pyrazinyl, 2-, 4-oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder, als Reste von teilhydrierten oder vollständig hydrierten heterocyclischen Ringen, beispielsweise auch Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl, Benzodioxolanyl.

Für den Rest Het stehende heterocylische Reste können an Kohlenstoffatomen undloder Ring-Stickstoffatomen unsubstituiert oder einfach oder mehrfach, zum Beispiel zweifach, dreifach, vierfach oder fünffach, durch gleiche oder verschiedene Substituenten substituiert sein. Kohlenstoffatome können zum Beispiel durch (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Oxo, Methylendioxy, Cyano, Hydroxycarbonyl, Aminocarbonyl, (C₁-C₄)-Alkoxycarbonyl, Phenyl, Phenoxy, Benzyl, Benzyloxy, (R⁸O)₂P(O), (R⁸O)₂P(O)-O-, Tetrazolyl substituiert sein. Schwefelatome können zum Sulfoxid oder zum Sulfon oxidiert sein. Beispiele für den Rest Het sind 1-Pyrrolyl, 1-Imidazolyl, 1-Pyrazolyl, 1-Tetrazolyl, Dihydropyridin-1-yl, Tetrahydropyridin-1-yl, 1-Pyrrolidinyl, 1-Piperidinyl, 1-Piperazinyl, 4-substituiertes 1-Piperazinyl, 4-Morpholinyl, 4-Thiomorpholinyl, 1-Oxo-4-Thiomorpholinyl, 1,1-Dioxo-4-Thiomorpholinyl, Perhydroazepin-1-yl, 2,5-Dimethyl-1-pyrrolyl, 2,6-Dimethyl-1-piperidinyl, 3,3-Dimethyl-4-morpholinyl, 4-lsopropyl-2,2,6,6-tetramethyl-1-piperazinyl, 4-Acetyl-1-piperazinyl, 4-Ethoxycarbonyl-1-piperazinyl,

Halogen steht für Fluor, Chlor, Brom oder lod, insbesondere für Fluor oder Chlor.

Natürliche oder unnatürliche Aminosäuren können in allen stereochemischen Formen vorliegen, falls chiral, beispielsweise in der D- oder L-Form oder in Form einer Mischung von Stereoisomeren, zum Beispiel in Form eines Racemats. Bevorzugt sind α-Aminosäuren und β-Aminosäuren, besonders bevorzugt α-Aminosäuren. Als in Betracht kommende Aminosäuren seien beispielsweise genannt (vgl. Houben-Weyl, Methoden der organischen Chemie, Band 15/1 und 15/2, Georg Thieme Verlag, Stuttgart, 1974):
Aad, Abu, yAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hlle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure.

Der Rest einer Aminosäure, Iminosäure oder Azaaminosäure oder eines Dipeptids wird wie in der Peptidchemie üblich aus der entsprechenden Aminosäure, Iminosäure oder Azaaminosäure oder dem Dipeptid erhalten, indem von der N-terminalen Aminogruppe oder von der Iminogruppe formal ein Wasserstoffatom entfernt wird bzw. von der Carbonsäuregruppe formal die Hydroxygruppe entfernt wird. Unter Aminosäureseitenketten werden Seitenketten von natürlichen oder unnatürlichen Aminosäuren verstanden. Azaaminosäuren sind natürliche oder unnatürliche Aminosäuren, in denen eine CH-Einheit durch ein Stickstoffatom ersetzt ist, zum Beispiel in α-Aminosäuren der Zentralbaustein 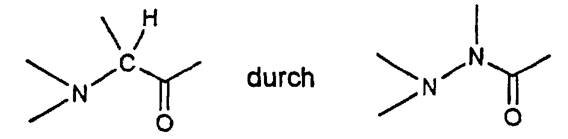 ersetzt ist.

Als Rest einer Iminosäure kommen insbesondere Reste von Heterocyclen aus der folgenden Gruppe in Betracht: Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1^{6,9}]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; lsoxazolidin-3-carbonsäure; Pyrazolidin-3-carbonsäure, Hydroxypyrrolidin-2-carbonsäure, die alle gegebenenfalls substituiert sein können (siehe folgende Formeln):

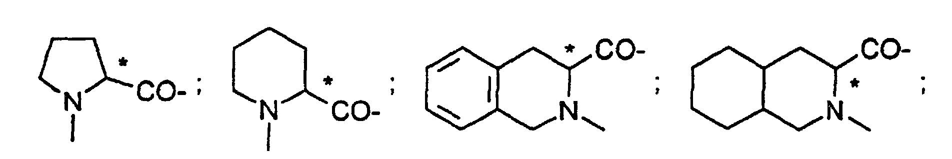

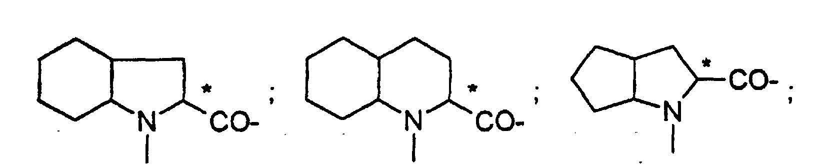

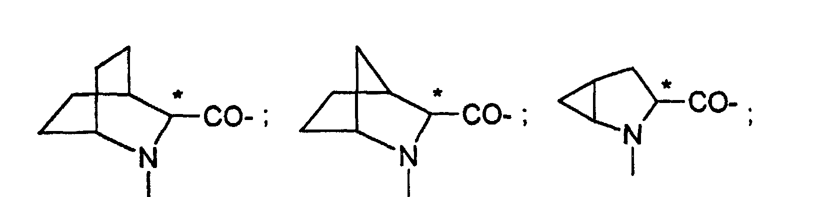

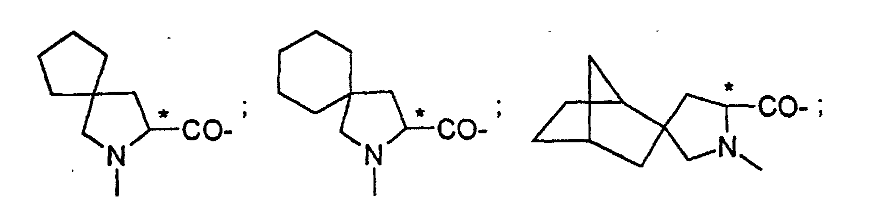

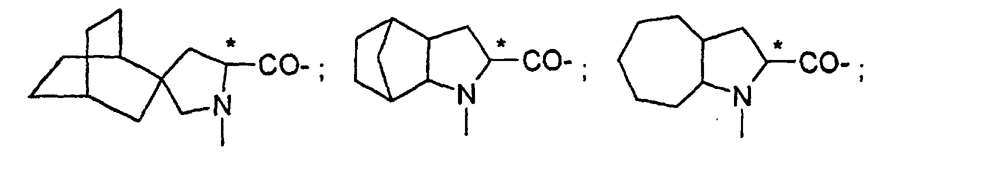

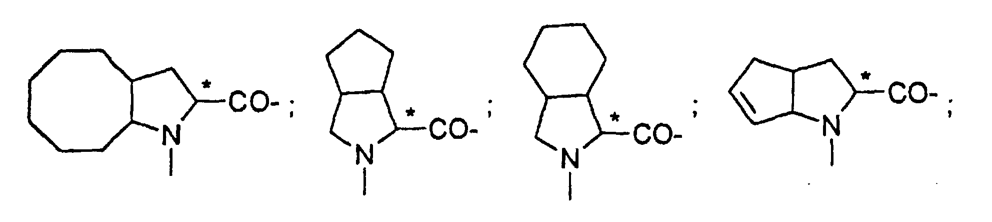

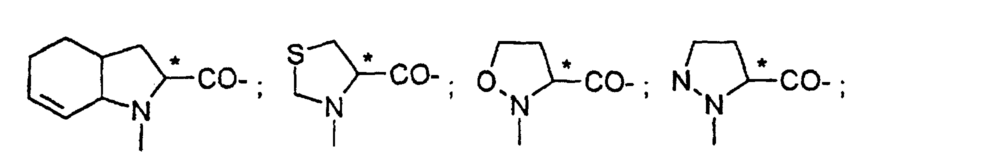

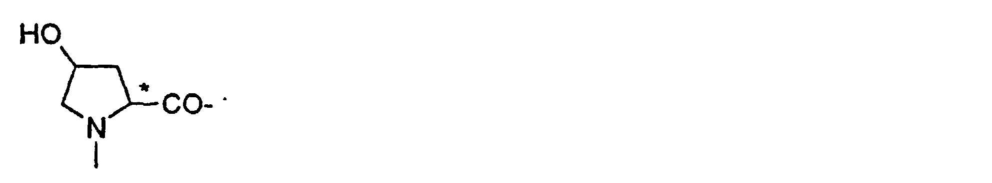

Die oben genannten Resten zugrundeliegenden Heterocyclen sind beispielsweise bekannt aus US-A-4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873, EP-A 271,865 und EP-A 344,682.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren und Dipeptide auch als Ester bzw. Amide vorliegen, wie zum Beispiel als Methylester, Ethylester, Isopropylester, Isobutylester, tert-Butylester, Benzylester, unsubstituiertes Amid, Ethylamid, Semicarbazid oder ω-Amino-(C₂-C₈)-alkylamid.

Funktionelle Gruppen der Aminosäuren, Iminosäuren und Dipeptide können geschützt vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr. 3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze.

Solche Salze werden beispielsweise von Verbindungen der Formel I, welche saure Gruppen enthalten, zum Beispiel Carbonsäure- oder Sulfonsäure- oder Phosphonsäuregruppen, mit anorganischen Basen, zum Beispiel Alkali- oder Erdalkalimetallverbindungen oder Ammoniak, gebildet. Die Salze der Verbindungen der Formel 1 können also zum Beispiel Natrium-, Kalium-, Magnesium-, Calciumoder Ammoniumsalze sein. Ebenso können Salze von Verbindungen der Formel I mit physiologisch verträglichen organischen Basen gebildet werden, zum Beispiel mit physiologisch verträglichen organischen Aminen wie Triethylamin, Ethanolamin oder Tris-(2-hydroxy-ethyl)-amin.

Verbindungen der Formel I, welche basische Gruppen enthalten, zum Beispiel eine Aminogruppe oder eine Guanidinogruppe, bilden mit anorganischen Säuren, wie zum Beispiel Salzsäure, Schwefelsäure oder Phosphorsäure, und mit organischen Carbon- oder Sulfonsäuren, wie zum Beispiel Essigsäure, Citronensäure, Benzoesäure, Maleinsäure, Fumarsäure, Weinsäure, Methansulfonsäure oder p-Toluolsulfonsäure Salze.

Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen.

Die Verbindungen der Formel I können in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als links- als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Mischungen dieser Formen Gegenstand der Erfindung.

Die erfindungsgemäßen Verbindungen der Formel I können darüber hinaus bewegliche Wasserstoffatome enthalten, also in verschiedenen tautomeren Formen vorliegen. Auch diese alle Tautomeren sind Gegenstand der vorliegenden Erfindung. Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und Metabolite, die wie die Verbindungen der Formel I wirken.

Die einzelnen Strukturelemente in der Formel I haben bevorzugt die folgenden Bedeutungen.

W steht bevorzugt für R¹-A-C(R¹³).
A steht bevorzugt für Methylen, Ethylen, Trimethylen, Tetramethylen, Pentamethylen, Cyclohexylen, Phenylen, Methylenphenyl, Methylenphenylmethyl, Phenylenmethyl oder Phenylenethyl.
Y steht bevorzugt für eine Carbonylgruppe.
Z steht bevorzugt für N(R⁰).
B steht bevorzugt für Methylen, Ethylen, Trimethylen, Tetramethylen, Vinylen, Phenylen oder für substituiertes Methylen oder Ethylen. Besonders bevorzugt steht B für einen zweiwertigen Methylenrest oder Ethylenrest (= 1,2-Ethylen) wobei jeder dieser Reste unsubstituiert oder substituiert sein kann und insbesondere substituiert ist. Ganz besonders bevorzugt steht B für einen substituierten oder unsubstituierten Methylenrest, insbesondere für einen substituierten Methylenrest. Ist ein für B stehender zweiwertiger Methylenrest oder Ethylenrest (= 1,2-Ethylen) substituiert, so ist er bevorzugt substituiert durch einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl oder im Heteroarylrest gegebenenfalls substituiertes Heteroaryl-(C₁-C₄)-alkyl, und ist er besonders bevorzugt substituiert durch (C₁-C₈)-Alkyl, also durch einen geradkettigen oder verzweigten Alkylrest mit 1, 2, 3, 4, 5, 6, 7 oder 8 Kohlenstoffatomen.
D steht bevorzugt für C(R²)(R³).
E steht bevorzugt für R¹⁰CO.

R steht bevorzugt für Wasserstoff, (C₁-C₆)-Alkyl oder Benzyl, insbesondere Wasserstoff, Methyl oder Ethyl.
R⁰ steht bevorzugt für (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl, besonders bevorzugt für gegebenenfalls substituiertes (C₆-C₁₄)-Aryl, im Arylrest gegebenenfalls substituiertes (C₆-C₁₄)-Aryl-(C₁-C₈)-alkyl oder im Heteroarylrest gegebenenfalls substituiertes Heteroaryl-(C₁-C₈)-alkyl, ganz besonders bevorzugt für im Arylrest gegebenenfalls substituiertes (C₆-C₁₄)-Aryl-(C₁-C₄)-alkyl oder im Heteroarylrest gegebenenfalls substituiertes Heteroaryl-(C₁-C₄)-alkyl, darüber hinaus bevorzugt für im Arylrest gegebenenfalls substituiertes (C₆-C₁₄)-Aryl-(C₁-C4)-alkyl. Speziell bevorzugt ist es, wenn R⁰ für unsubstituiertes oder im Arylrest einfach oder mehrfach substituiertes Biphenylylmethyl, Naphthylmethyl oder Benzyl steht.
R¹ steht bevorzugt für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano oder für den Rest eines gegebenenfalls substituierten, 5- bis 14-gliedrigen, mono- oder polycyclischen, gesättigten oder ungesättigten heterocyclischen Rings der Formel 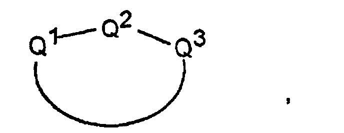 in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann.
Besonders bevorzugt steht R¹ für einen der Reste -O-C(O)-R²¹, -O-C(O)-OR²²,
-O-C(O)-N(R²¹)-R²⁸, -N(R²⁹)-C(O)-OR²², -N(R²⁸)-C(O)-N(R²¹)-R²⁸,
-N(R²⁸)-C(S)-N(R²¹)-R²⁸ oder Cyano.

R² steht bevorzugt für Wasserstoff oder (C₁-C₈)-Alkyl.
R³ steht bevorzugt für (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵, besonders bevorzugt für gegebenenfalls substituiertes (C₆-C₁₄)-Aryl, R¹¹NH, CON(CH₃)R⁴ oder CONHR⁴.
R⁴ steht bevorzugt für (C₁-C₈)-Alkyl, das gegebenenfalls wie in der Definition von R⁴ angegeben substituiert sein kann, besonders bevorzugt für (C₁-C₈)-Alkyl, das durch einen oder zwei der in der Definition von R⁴ angegebenen Reste substituiert ist;
R¹¹ steht bevorzugt für Wasserstoff, R^{12a}, R^{12a}-CO, H-CO, R^{12a}-O-CO, R^{12b}-CO, R^{12b}-CS oder R^{12a}-S(O)₂, besonders bevorzugt für Wasserstoff, R^{12a}, R^{12a}-CO, R^{12a}-O-CO, R^{12b}-CO, R^{12b}-CS oder R^{12a}-S(O)₂, insbesondere für einen Rest aus der Reihe Wasserstoff, (C₁-C₁₈)-Alkyl, R^{12c}CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ und R¹⁵, wobei R^{c} für Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- steht. Ganz besonders bevorzugt steht R¹¹ für R^{12a}, R^{12a}-CO, R^{12a}-O-CO, R^{12b}-CO, R^{12b}-CS oder
R^{12a}-S(O)₂, darüber hinaus besonders bevorzugt für R^{12a}, R^{12a}-CO, R^{12a}-O-CO,
R^{12b}-CO oder R^{12a}-S(O)₂.
R^{12a} steht bevorzugt für (C₁-C₁₀)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵.
R^{12b} steht bevorzugt für R^{12a}-NH.
R¹³ steht bevorzugt für Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl, besonders bevorzugt für Wasserstoff oder insbesondere für (C₁-C₆)-Alkyl, (C₃-C₈)-Cycloalkyl oder Benzyl, wobei ein ganz besonders bevorzugter Alkylrest, für den R¹³ steht, der Methylrest ist.
R¹⁵ steht bevorzugt für R¹⁶-(C₁-C₃)-alkyl oder für R¹⁶, besonders bevorzugt für R¹⁶-(C₁)-alkyl oder R¹⁶. Darüber hinaus bevorzugt steht R¹⁵ dann, wenn R³ für COOR¹⁵ steht, für den exo-2-Norbornylrest, den endo-2-Norbornylrest oder den Bicyclo[3.2.1]octylrest, und steht R¹⁵ dann, wenn R³ für CONHR¹⁵ steht, für den exo-2-Norbornylrest, den endo-2-Norbornylrest, den 3-Noradamantylrest und insbesondere den 1-Adamantylrest, den 2-Adamantylrest, den 1-Adamantylmethylrest oder den 2-Adamantylmethylrest.
R¹⁶ steht bevorzugt für einen 7- bis 12-gliedrigen verbrückten bicyclischen oder tricyclischen Rest, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann.
Het steht bevorzugt für den Rest eines über ein Stickstoffatom gebundenen 5- bis 10-gliedrigen, gesättigten monocyclischen oder polycyclischen Heterocyclus, der ein oder zwei gleiche oder verschiedene zusätzliche Ring-Heteratome aus der Reihe Sauerstoff, Stickstoff und Schwefel enthalten kann und an Kohlenstoffatomen und an Ring-Stickstoffatomen gegebenenfalls substituiert sein kann, wobei an zusätzlichen Ring-Stickstoffatome gleiche oder verschiedene Reste R^{h}, R^{h}CO oder R^{h}O-CO als Substituenten stehen können. Besonders bevorzugt steht Het für einen derartigen Heterocyclus, der kein zusätzliches Ring-Heteroatom enthält oder der ein zusätzliches Ring-Heteroatom aus der Reihe Stickstoff, Sauerstoff und Schwefel enthält, ganz besonders bevorzugt steht Het für den Rest eines über ein Stickstoffatom gebundenen 5-, 6- oder 7-gliedrigen, gesättigten monocyclischen Heterocyclus, der kein zusätzliches Ring-Heteroatom enthält oder der ein zusätzliches Ring-Heteroatom aus der Reihe Stickstoff, Sauerstoff und Schwefel enthält, wobei auch in diesen Fällen der Rest Het unsubstituiert sein kann oder an Kohlenstoffatomen und/oder an zusätzlichen Ring-Stickstoffatomen substituiert sein kann.
b, c und d stehen bevorzugt unabhängig voneinander für 1.
e, g und h stehen bevorzugt unabhängig voneinander für die Zahlen 0, 1, 2 oder 3.

Bevorzugte Verbindungen der Formel I sind solche, worin gleichzeitig
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R und R⁰: unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
- R¹: einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, )-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸, Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 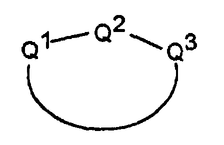 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)2, im Arylrest gegebenenfalls substituiertes (C₆-C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxyl, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- bedeutet;
- R¹³: Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Oycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
- R²⁸: für einen der Reste R²¹-, R²¹N(R²¹)-, R²¹C(O)-, R²²O-C(O)-, R²¹N(R²¹)-C(O)- oder R²¹N(R²¹)-C(=N(R²¹))- steht;
- R²⁹: für einen der Reste R²²-, R²¹N(R²¹)-, R²¹C(O)-, R²²O-C(O)-, R²¹N(R²¹)-C(O)- oder R²¹N(R²¹)-C(=N(R²¹))- steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze,
wobei, wenn gleichzeitig W für 4-Cyanophenyl-C(R¹³) steht, Y für eine Carbonylgruppe steht, Z für NR^{0a} steht, B für eine unsubstituierte Methylengruppe steht, R für R^{a} steht, b, c und d für 1 stehen und e, f und g für 0 stehen, dann nicht
D für C(R^{2a})(R^{3a}) stehen kann, wobei
- R^{0a}, R^{a} und R^{2a}: unabhängig voneinander für Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl stehen und
- R^{3a}: für Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder 2-, 3- oder 4-Pyridyl steht.

Besonders bevorzugte Verbindungen der Formel I sind solche, worin gleichzeitig W für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff, (C₁-C₆)-Alkyl oder Benzyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 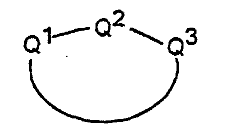 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff oder (C₁-C₈)-Alkyl steht;
R³ für (C₁-C₈)-Alkyl, gegebenenfalls substituiertes (C₆-C₁₄)-Aryl, (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹NH, R⁴CO, COOR⁴, CONHR⁴, CSNHR⁴, COOR¹⁵ und CONHR¹⁵ steht;
und e, g und h unabhängig voneinander für die Zahlen 0, 1, 2 oder 3 stehen;
in allen ihren stereoisomeren Formen und Mischungen davon in allen Verhältnissen, sowie ihre physiologisch verträglichen Salze.

Ganz besonders bevorzugt sind Verbindungen der Formel I, worin W für R¹-A-C(R¹³) und R¹³ für (C₁-C₆)-Alkyl, im Arylrest gegebenenfalls substituiertes (C₆-C₁₄)-Aryl-(C₁-C₈)-alkyl oder (C₃-C₈)-Cycloalkyl steht;
in allen ihren stereoisomeren Formen und Mischungen davon in allen Verhältnissen, sowie ihre physiologisch verträglichen Salze.

Eine Reihe speziell bevorzugter Verbindungen der Formel I bilden solche, worin R³ für gegebenenfalls substituiertes (C₆-C₁₄)-Aryl, für COOR⁴, für R¹¹NH oder für CONHR⁴ steht, wobei -NHR⁴ für den Rest einer α-Aminosäure, für deren ω-Amino-(C₂-C₈)-alkylamid, für deren (C₁-C₈)-Alkylester, für deren (C₆-C₁₄)-Aryl-(C₁-C₄)-alkylester oder für deren Derivat steht, in dem die Carbonsäuregruppe in die Gruppe Het-CO überführt ist, in allen ihren stereoisomeren Formen und Mischungen davon in allen Verhältnissen, sowie ihre physiologisch verträglichen Salze. Der für -NHR⁴ stehende Rest einer α-Aminosäure wird formal durch Abstraktion eines Wasserstoffatoms von der Aminogruppe der Aminosäure erhalten. Speziell bevorzugt ist es in dieser Reihe, wenn R³ für CONHR⁴ steht, wobei -NHR⁴ für den Rest der α-Aminosäuren Valin, Lysin, Phenylglycin, Phenylalanin oder Tryptophan oder deren (C₁-C₈)-Alkylester, (C₆-C₁₄)-Aryl-(C₁-C₄)-alkylester oder Het-CO-Derivat steht.

Darüber hinaus bevorzugte Verbindungen der Formel I in dieser Reihe sind solche, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl oder Methylenphenylmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -O-C(O)-R²¹, -O-C(O)-OR²², -O-C(O)-N(R²¹)-R²⁸,
-N(R²⁹)-C(O)-OR²², -N(R²⁸)-C(O)-N(R²¹)-R²⁸, -N(R²⁸)-C(S)-N(R²¹)-R²⁸ oder Cyano steht;
R² für Wasserstoff steht;
R³ für den Rest CONHR⁴ steht;
R⁴ für Methyl steht, das durch Hydroxycarbonyl und einen Rest aus der Reihe (C₁-C₄)-Alkyl, Phenyl und Benzyl substituiert ist, oder für Methyl steht, das durch (C₁-C₈)-Alkoxycarbonyl, bevorzugt (C₁-C₄)-Alkoxycarbonyl, und einen Rest aus der Reihe (C₁-C₄)-Alkyl, Phenyl und Benzyl substituiert ist, oder für Methyl steht, das durch Het-CO und einen Rest aus der Reihe (C₁-C₄)-Alkyl, Phenyl und Benzyl substituiert ist;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Steht -NHR⁴ für einen (C₁-C₈)-Alkylester einer α-Aminosäure bzw. enthält R⁴ einen Alkoxycarbonylrest, so ist der Methyl-, Ethyl-, Isopropyl-, Isobutyl- oder tert-Butylester bevorzugt, steht -NHR⁴ für einen (C₆-C₁₄)-Aryl-(C₁-C₄)-alkylester einer α-Aminosäure, so ist der Benzylester bevorzugt.

Eine weitere Reihe speziell bevorzugter Verbindungen der Formel I bilden solche, worin gleichzeitig
W für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff oder (C₁-C₆)-Alkyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 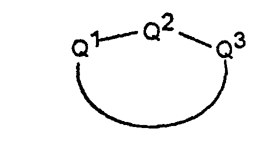 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff oder (C₁-C₈)-Alkyl steht;
R³ für CONHR¹⁵ oder CONHR⁴ steht, wobei R⁴ hierin für einen unsubstituierten oder durch einen oder mehrere (C₆-C₁₄)-Arylreste substituierten (C₁-C₈)-Alkylrest steht;
R¹⁵ für R¹⁶-(C₁-C₆)-Alkyl oder R¹⁶ steht, wobei R¹⁶ für einen 7- bis 12-gliedrigen verbrückten bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann, und insbesondere R¹⁵ für einen Adamantylrest oder einen Adamantylmethylrest steht;
und e, g und h unabhängig voneinander für die Zahlen 0, 1, 2 oder 3 stehen und b, c und d für 1 stehen;
in allen ihren stereoisomeren Formen und Mischungen davon in allen Verhältnissen, sowie ihre physiologisch verträglichen Salze.

Darüber hinaus bevorzugte Verbindungen der Formel I in dieser Reihe sind solche, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl oder Methylenphenylmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ oder Cyano steht;
R² für Wasserstoff steht;
R³ für CONHR¹⁵ oder CONHR⁴ steht, wobei R⁴ hierin für einen unsubstituierten oder durch einen oder mehrere (C₆-C₁₀)-Arylreste substituierten (C₁-C₆)-Alkylrest steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Weiterhin bilden eine Reihe speziell bevorzugter Verbindungen der Formel I solche, in denen gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl, Methylenphenylmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 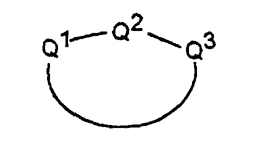 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen unsubstituierten oder substituierten Phenylrest oder Naphthylrest steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und lsopropoxy steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Schließlich bilden eine Reihe speziell bevorzugter Verbindungen der Formel I solche Verbindungen, in denen gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl, Methylenphenylmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 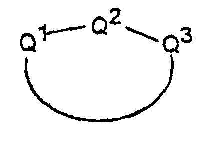 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff steht;
R³ für R¹¹NH steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c, d und e für 1 stehen und f und g für 0 stehen;
h für 0 steht;
in allen ihren stereoisomeren Formen und Mischungen davon in allen Verhältnissen, sowie ihre physiologisch verträglichen Salze.

In dieser Reihe sind darüber hinaus bevorzugt solche Verbindungen der Formel I, worin R¹¹ für R^{12a}, R^{12a}-CO, R^{12a}-O-CO, R^{12b}-CO, R^{12b}-CS oder R^{12a}-S(O)₂, insbesondere für R^{12a}, R^{12a}-CO, R^{12a}-O-CO, R^{12b}-CO oder R^{12a}-S(O)₂, steht;
R^{12a} für (C₁-C₁₀)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ steht ;
R^{12b} für R^{12a}-NH steht;
in allen ihren stereoisomeren Formen und Mischungen davon in allen Verhältnissen, sowie ihre physiologisch verträglichen Salze.

Ganz speziell bevorzugte Verbindungen der Formel I sind solche, worin ein für die Gruppe B stehender substituierter Methylenrest oder substituierter Ethylenrest als Substituenten einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl und im Heteroarylrest gegebenenfalls substituiertes Heteroaryl-(C₁-C₄)-alkyl trägt, in allen ihren stereoisomeren Formen und Mischungen davon in allen Verhältnissen, sowie ihre physiologisch verträglichen Salze. Noch spezieller bevorzugte Verbindungen der Formel I sind solche, worin B für einen unsubstituierten Methylenrest steht oder für einen Methylenrest steht, der durch einen (C₁-C₈)-Alkylrest, insbesondere durch einen (C₁-C₆)-Alkylrest, substituiert ist, in allen ihren stereoisomeren Formen und Mischungen davon in allen Verhältnissen, sowie ihre physiologisch verträglichen Salze.

Generell sind Verbindungen der Formel I bevorzugt, die an Chiralitätszentren, z. B an dem für D stehenden chiralen Kohlenstoffatom und an dem Zentrum W im 5-Ring-Heterocyclus in der Formel I, eine einheitliche Konfiguration aufweisen.

Die Verbindungen der Formel I können beispielsweise hergestellt werden durch Fragmentkondensation einer Verbindung der Formel II 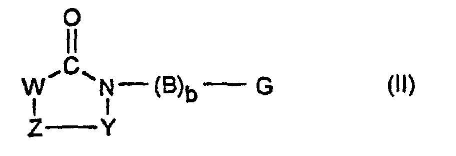 mit einer Verbindung der Formel III, 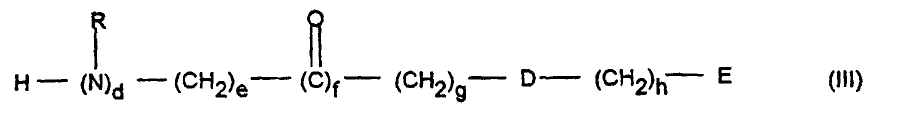 wobei W, Y, Z, B, D, E, R sowie b, d, e, f, g, und h wie oben angegeben definiert sind und G für Hydroxycarbonyl, (C₁-C₆)-Alkoxycarbonyl, aktivierte Carbonsäurederivate, wie Säurechloride oder Aktivester, oder Isocyanato steht.

Zur Kondensation der Verbindungen der Formel II mit denen der Formel III verwendet man vorteilhafterweise die an sich bekannten Kupplungsmethoden der Peptidchemie (siehe zum Beispiel Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert-Butylester vorliegen. Ein Aminogruppen-Schutz erübrigt sich, wenn die zu generierenden Aminogruppen noch als Nitro- oder Cyanogruppen vorliegen und erst nach der Kupplung zum Beispiel durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird.

Verbindungen der Formel II, in der W für R¹-A-C(R¹³), Y für eine Carbonylgruppe und Z für NR⁰ steht, können beispielsweise hergestellt werden, indem man zunächst Verbindungen der Formel IV 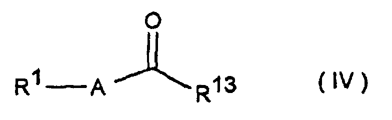 in einer Bucherer-Reaktion zu Verbindungen der Formel V, 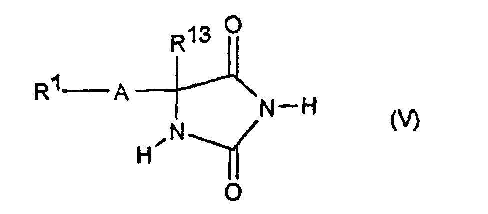 in der ebenso wie in der Formel IV R¹, R¹³ und A wie oben angegeben definiert sind, umsetzt (H. T. Bucherer, V. A. Lieb, J. Prakt. Chem. 141(1934), 5). Verbindungen der Formel VI, 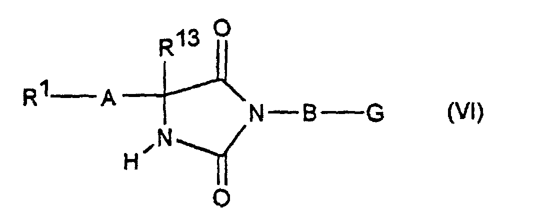 in der R¹, R¹³, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel R⁰-LG, in der R⁰ die oben angegebenen Bedeutungen hat und LG eine nucleophil substituierbare Abgangsgruppe, zum Beispiel Halogen, insbesondere Chlor oder Brom, (C₁-C₄)-Alkoxy, gegebenenfalls substituiertes Phenoxy oder eine heterocyclische Abgangsgruppe wie zum Beispiel Imidazolyl, darstellt, führt zu den entsprechenden Verbindungen der Formel II. Diese Umsetzungen können analog bekannten, dem Fachmann geläufigen Methoden durchgeführt werden. Je nach dem Einzelfall kann es hierbei, wie bei allen Schritten in der Synthese der Verbindungen der Formel I, angebracht sein, funktionelle Gruppen die zu Nebenreaktionen oder unerwünschten Reaktionen führen könnten, durch eine dem Syntheseproblem angepaßte Schutzgruppenstrategie temporär zu blockieren, was dem Fachmann bekannt ist.

Steht W für R¹-A-CH=C, so kann dieses Strukturelement beispielsweise eingeführt werden, indem analog bekannten Methoden ein Aldehyd mit einem 5-Ring-Heterocyclus kondensiert wird, der eine Methylengruppe in der der Gruppe W entsprechenden Position enthält.

Verbindungen der Formel I, in denen der 5-Ring-Heterocyclus einen Dioxo- oder Thioxo-oxo-substituierten Imidazolidinring darstellt, in dem W für R¹-A-C(R¹³) steht, können auch wie folgt erhalten werden:
Durch Reaktion von α-Aminosäuren oder N-substituierten α-Aminosäuren oder bevorzugt deren Ester, zum Beispiel der Methyl-, Ethyl-, tert-Butyl- oder Benzylester, beispielsweise einer Verbindung der Formel VII, 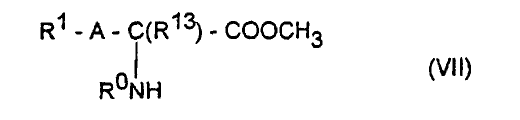 worin R⁰, R¹, R¹³ und A wie oben angegeben definiert sind, mit einem Isocyanat oder Isothiocyanat beispielsweise der Formel VIII, 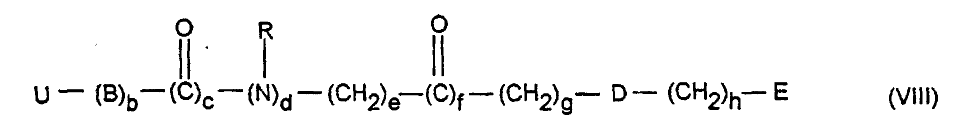 worin B, D, E und R sowie b, c, d, e, f, g und h wie oben angegeben definiert sind und U für Isocyanato oder Isothiocyanato steht, erhält man Harnstoff- oder Thioharnstoffderivate, beispielsweise der Formel IX, 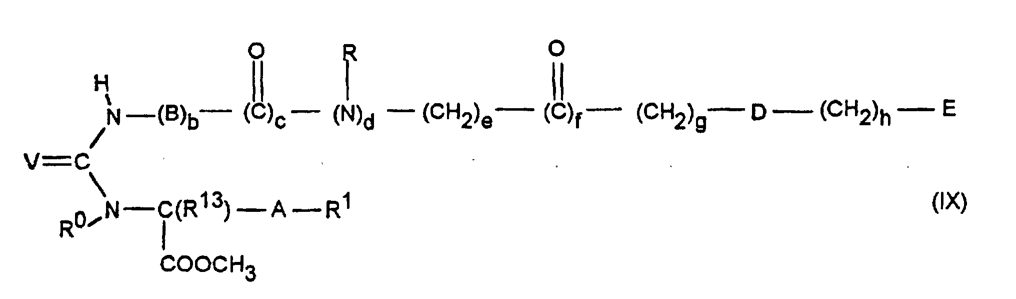 für die die oben angegebenen Definitionen gelten und in der V Sauerstoff oder Schwefel bedeutet, und die durch Erhitzen mit Säure unter Verseifung der Esterfunktionen zu Verbindungen der Formel Ia 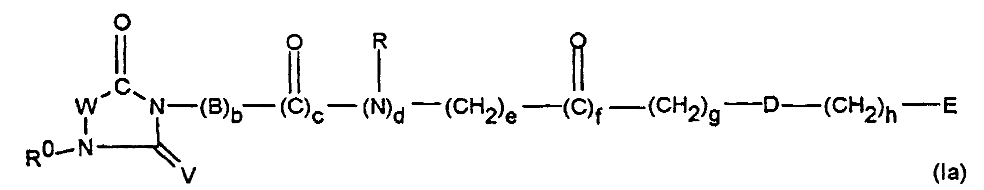 cyclisiert werden, in der V Sauerstoff oder Schwefel bedeutet, W für R¹-A-C(R¹³) steht und für die ansonsten die oben angegebenen Bedeutungen gelten. Die Cyclisierung der Verbindungen der Formel IX zu den Verbindungen der Formel Ia kann auch durch Behandlung mit Basen in inerten Lösungsmittel durchgeführt werden, zum Beispiel durch Behandlung mit Natriumhydrid in einem aprotischen Lösungsmittel wie Dimethylformamid.

Während der Cyclisierung können Guanidinogruppen durch Schutzgruppen, zum Beispiel NO₂, blockiert werden. Aminogruppen in der Seitenkette können in geschützer Form oder noch als NO₂- oder Cyanofunktion vorliegen, die später zur Aminogruppe reduziert oder im Falle der Cyanogruppe auch in die Formamidinogruppe umgewandelt werden kann.

Verbindungen der Formel I, in denen der 5-Ring-Heterocyclus einen Dioxo- oder Thioxo-oxo-substituierten Imidazolidinring darstellt, in dem W für R¹-A-C(R¹³) und c für 1 steht, können auch erhalten werden, indem man eine Verbindung der Formel VII mit einem Isocyanat oder Isothiocyanat der Formel X 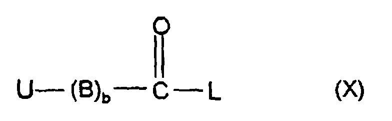 umsetzt, in der B, U und b wie oben für die Formel VIII angegeben definiert sind und L eine Alkoxygruppe, zum Beispiel eine (C₁-C₄)-Alkoxygruppe wie Methoxy, Ethoxy oder tert-Butoxy, eine (C₆-C₁₄)-Aryloxygruppe, zum Beispiel Phenoxy, oder eine (C₆-C₁₄)-Aryl-(C₁-C₄)-alkoxygruppe, zum Beispiel Benzyloxy, bedeutet. Dabei wird eine Verbindung der Formel XI 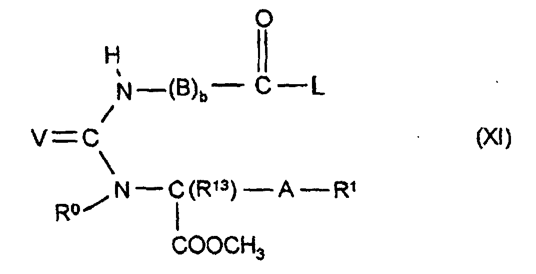 erhalten, in der A, B, V, L, R⁰, R¹, R¹³ und b wie oben für die Formeln IX und X angegeben definiert sind, die dann unter dem Einfluß einer Säure oder einer Base, wie oben für die Cyclisierung der Verbindungen der Formel IX beschrieben, zu einer Verbindung der Formel XII, 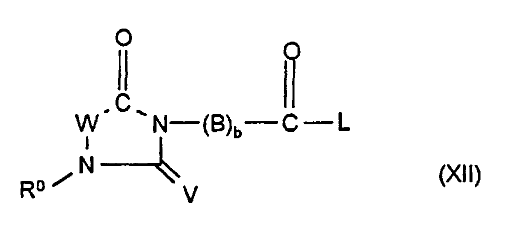 in der B, L, V, W, R⁰ und b wie oben für die Formeln Ia und X angegeben definiert sind, cyclisiert wird. Aus der Verbindung der Formel XII wird dann durch Hydrolyse der Gruppe CO-L zur Carbonsäure COOH und nachfolgende Kupplung mit einer Verbindung der Formel III, wie oben für die Kupplung der Verbindungen der Formeln II und III beschrieben, eine Verbindung der Formel Ia erhalten. Auch hier können während der Cyclisierung funktionelle Gruppen in geschützter Form vorliegen oder in Form von Vorstufen vorliegen, zum Beispiel Guanidinogruppen durch NO₂ blockiert werden oder Aminogruppen in geschützer Form oder noch als NO₂- oder Cyanofunktion vorliegen, die später zur Aminogruppe reduziert oder im Falle der Cyanogruppe auch in die Formamidinogruppe umgewandelt werden kann.

Eine weitere Methode zur Herstellung von Verbindungen der Formel Ia ist beispielsweise die Umsetzung von Verbindungen der Formel XIII,

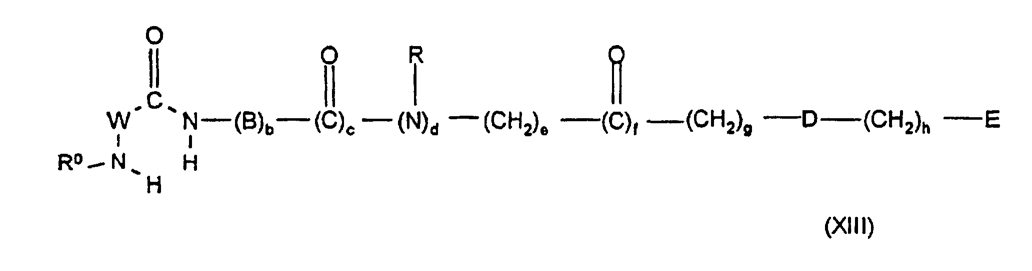

in der W für R¹-A-C(R¹³) steht und für die ansonsten die oben angegebenen Definitionen gelten, mit Phosgen, Thiophosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Eine Überführung einer Aminofunktion in eine Guanidinofunktion kann mit folgenden Reagenzien durchgeführt werden:
1. O-Methylisoharnstoff (S. Weiss und H. Krommer, Chemiker Zeitung 98 (1974), 617-618)
2. S-Methylisothioharnstoff (R.F. Borne, M.L. Forrester und I.W. Waters, J. Med. Chem. 20 (1977), 771-776)
3. Nitro-S-methylisothioharnstoff (L.S. Hafner und R.E. Evans, J. Org. Chem. 24 (1959)57)
4. Formamidinosulfonsäure (K. Kim, Y.-T. Lin und H.S. Mosher, Tetrah. Lett. 29 (1988), 3183-3186)
5. 3,5-Dimethyl-1-pyrazolyl-formamidinium-nitrat (F.L. Scott, D.G. O'Donovan und J. Reilly, J. Amer. Chem. Soc. 75 (1953), 4053-4054)
6. N,N'-Di-tert-butyloxycarbonyl-S-methyl-isothioharnstoff (R. J. Bergeron und J. S. McManis, J. Org. Chem. 52 (1987), 1700-1703)
7. N-Alkoxycarbonyl-, N,N'-Dialkoxycarbonyl-, N-Alkylcarbonyl- und N,N'dialkylcarbonyl-S-methyl-isothioharnstoff (H. Wollweber, H. Kölling, E. Niemers, A. Widdig, P. Andrews, H.-P. Schulz und H. Thomas, Arzneim. Forsch./Drug Res. 34 (1984), 531-542).

Hinsichtlich der Herstellung der Verbindungen der Formel I wird weiterhin Bezug genommen auf die Angaben zum Aufbau des Molekülgerüsts, die in der WO-A-95/14008, der DE-A-19515177 und in der ihr entsprechenden WO-A-96/33976, und in der DE-A-19635522 und den ihr entsprechenden Patentdokumenten, zum Beispiel der EP-A-796 855 (europäische Patentanmeldung 97103712.2) und der US-B-6218415 (US-Patentanmeldung 08/821253), enthalten sind und die Bestandteil der vorliegenden Offenbarung sind.

Zur Herstellung von Verbindungen der Formel 1, in der R¹ für -S-R²¹ steht, kann von den entsprechenden Verbindungen der Formel IV ausgegangen werden, in der R¹ für -S-R²¹ steht. Setzt man in die Bucherer-Reaktion Verbindungen der Formel IV ein, in der R¹ für eine geschützte SH-Gruppe steht, so gelangt man nach der Abspaltung der Schutzgruppe zu Verbindungen der Formel I, in der R¹ für -SH steht. Diese Verbindungen können wiederum durch Einführung des Restes R²¹ in Verbindungen der Formel I überführt werden, in der R¹ für -S-R²¹ (mit anderen Bedeutungen von R²¹ als Wasserstoff) steht, oder auch als Zwischenprodukte für die Herstellung anderer Verbindungen der Formel I dienen, in denen das an die Gruppe A gebundene Atom des Restes R²¹ ein Schwefelatom ist.

Verbindungen der Formel I, in der R¹ für -S(O)₂-R²¹ steht, können beispielsweise hergestellt werden, indem man Verbindungen der Formel I, in der R¹ für -S-R²¹ steht, gemäß literaturbekannten Verfahren (vgl. Houben-Weyl, Methoden der Organischen Chemie, Bd. E12/2, Georg Thieme Verlag, Stuttgart 1985, S. 1058 ff.) zu den Sulfonen oxidiert, d. h. zu den Verbindungen der Formel I, in der R¹ für -S(O)₂-R²¹ steht. Entsprechend können Verbindungen der Formel I, in der R¹ für -S-R²¹ steht, unter geeigneten, dem Fachmann geläufigen Reaktionsbedingungen gemäß an sich bekannten Verfahren (vgl. Houben-Weyl, Methoden der Organischen Chemie, Bd. E11/1, Georg Thieme Verlag, Stuttgart 1985, S. 702 ff.) zu den Sulfoxiden oxidiert werden, d. h. zu den Verbindungen der Formel I, in der R¹ für -S(O)-R²¹ steht. Falls erforderlich, werden bei den Oxidationen zu den Sulfoxiden oder zu den Sulfonen oxidationsempfindliche Gruppen im Molekül vor der Durchführung der Oxidation durch geeignete Schutzgruppen geschützt.

Verbindungen der Formel I, in der R¹ für -S(O)₂-OR²¹ oder -S(O)₂-N(R²¹)-R²⁸ steht, können beispielsweise hergestellt werden, indem man Verbindungen der Formel I, in der R¹ für -SH steht, gemäß literaturbekannten Verfahren (vgl. Houben-Weyl, Methoden der Organischen Chemie, Bd. E12/2, Georg Thieme Verlag, Stuttgart 1985, S. 1058 ff.) für die Herstellung von Sulfonsäurederivaten zu Verbindungen der Formel I, in der R¹ für -S(O)₂-OH steht, oxidiert. Aus diesen Sulfonsäuren können dann direkt oder über entsprechende Sulfonsäurehalogenide durch Veresterung oder Knüpfung einer Amidbindung die Verbindungen der Formel I erhalten werden, in der R¹ für -S(O)₂-OR²¹ oder -S(O)₂-N(R²¹)-R²⁸ steht.

Verbindungen der Formel I, in der R¹ für -S(O)-OR²¹ oder -S(O)-N(R²¹)-R²⁸ steht, können beispielsweise hergestellt werden, indem man Verbindungen der Formel I, in der R¹ für -SH steht, in die entsprechenden Sulfide überführt, d. h. in die Salze, in denen R¹ für -S⁻ steht und das Gegenion zum Beispiel ein Alkalimetallion oder ein Erdalkalimetallion ist, und diese Salze anschließend zum Beispiel mit meta-Chlorperbenzoesäure zu den Sulfinsäuren oxidiert, d. h. zu den Verbindungen der Formel I, in der R¹ für -S(O)-OH steht (vgl. Houben-Weyl, Methoden der Organischen Chemie, Bd. E11/1, Georg Thieme Verlag, Stuttgart 1985, S. 618 f.). Aus den Sulfinsäuren können nach an sich bekannten Verfahren die entsprechenden Sulfinsäureester und Sulfinsäureamide hergestellt werden, d. h. die Verbindungen der Formel I, in der R¹ für -S(O)-OR²¹ oder -S(O)-N(R²¹)-R²⁸ steht.

Falls erforderlich, werden auch bei der Herstellung von Sulfonsäurederivaten und Sulfinsäurederivaten durch eine Oxidation oxidationsempfindliche Gruppen im Molekül vor der Durchführung der Oxidation durch geeignete Schutzgruppen geschützt.

Außer den erwähnten Methoden können zur Herstellung der Verbindungen der Formel I, in der R¹ für -S(O)-R²², -S(O)₂-R²², -S(O)-OR²¹, -S(O)₂-OR²¹, -S(O)-N(R²¹)-R²⁸ oder -S(O)₂-N(R²¹)-R²⁸ steht, generell auch andere in der Literatur beschriebene Verfahren zu Herstellung solcher Verbindungstypen Anwendung finden (vgl. Houben-Weyl, Methoden der Organischen Chemie, Georg Thieme Verlag, Stuttgart, Bd. E11/1, 1985, S. 618 ff. oder Bd. E11/2, 1985, S. 1055 ff.).

Verbindungen der Formel I, in der R¹ für weitere schwefelhaltige Gruppen steht, zum Beispiel für -N(R²⁸)-C(S)-OR²², -N(R²⁸)-C(S)-SR²², -N(R²⁸)-C(S)-R²¹, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -C(S)-R²¹, -C(S)-N(R²¹)-R²⁸, können nach an sich bekannten, in der Literatur beschriebenen Verfahren (vgl. Houben-Weyl, Methoden der Organischen Chemie, Bd. E11/1 und E11/2, Georg Thieme Verlag, Stuttgart 1985) aus geeigneten Vorstufen aufgebaut werden, wobei die Anpassung der gewählten Synthesemethode an das jeweilige Zielmolekül dem Fachmann kein Probleme bereitet. Dies gilt auch für weitere Verfahren zur Herstellung von Verbindungen der Formel I, in der R¹ für -S-R²¹ steht.

Letzteres gilt auch für die Herstellung der Verbindungen der Formel I, in der R¹ für einen der in der Definition von R¹ aufgeführten phosphorhaltigen Reste steht, zum Beispiel ein Phosphonsäure-Derivat oder ein Phosphorsäure-Derivat. Diese Verbindungen können analog literaturbekannten Verfahren (vgl. Houben-Weyl, Methoden der Organischen Chemie, Bd. E1 und E2, Georg Thieme Verlag, Stuttgart 1982) für die Herstellung solcher Verbindungen aus geeigneten Vorstufen aufgebaut werden.

Verbindungen der Formel I, in der R¹ für -N(R²⁸)-C(O)-NH-R²⁸ steht, können beispielsweise hergestellt werden, indem man die entsprechenden Verbindungen der Formel I, in der R¹ für -NH-R²⁸ steht, mit Isocyanaten der Formel O=C=N-R²⁸ gemäß literaturbekannten Verfahren umsetzt (vgl. Houben-Weyl, Methoden der Organischen Chemie, Bd. VIII, Georg Thieme Verlag, Stuttgart 1952, S. 132). Analog können Verbindungen der Formel I, in R¹ für -N(R²⁸)-C(S)-NH-R²⁸ steht, hergestellt werden, indem man zum Beispiel die entsprechenden Verbindungen der Formel I, in der R¹ für -NHR²⁸ steht, mit Isothiocyanaten der Formel S=C=N-R²⁸ umsetzt. Generell können zur Herstellung von Verbindungen der Formel I, in der R¹ für -N(R²⁸)-C(O)-N(R²¹)-R²⁸ oder-N(R²⁸)-C(S)-N(R²¹)-R²⁸ steht, literaturbekannte Methoden zur Herstellung von Harnstoffen bzw. Thioharnstoffen Anwendung finden (vgl. Houben-Weyl, Methoden der Organischen Chemie, Bd. VIII, Georg Thieme Verlag, Stuttgart 1952).

Verbindungen der Formel I, in der R¹ für -N(R²⁹)-C(O)-OR²² steht, können beispielsweise hergestellt werden, indem man Verbindungen der Formel I, in der R¹ für -NHR²⁹ steht, mit Chlorkohlensäureestern der Formel Cl-C(O)-OR²² gemäß literaturbekannten Verfahren (vgl. Houben-Weyl, Methoden der Organischen Chemie, Bd. VIII, Georg Thieme Verlag, Stuttgart 1952, S. 138) umsetzt. Generell können auch andere literaturbekannte Methoden zur Herstellung von Verbindungen der Formel I, in der R¹ für -N(R²⁹)-C(O)-OR²² steht, Anwendung finden (vgl.

Houben-Weyl, Methoden der Organischen Chemie, Bd. VIII, Georg Thieme Verlag, Stuttgart 1952). Analog können Verbindungen der Formel I, in der R¹ für -N(R²⁸)-C(O)-SR²² steht, hergestellt werden.

Verbindungen der Formel I, in der R¹ für -O-C(O)-NH-R²⁸ steht, können beispielsweise hergestellt werden, indem man Verbindungen der Formel I, in der R¹ für -OH steht, mit Isocyanaten der Formel O=C=N-R²⁸ gemäß literaturbekannten Verfahren (vgl. Houben-Weyl, Methoden der Organischen Chemie, Bd. VIII, Georg Thieme Verlag, Stuttgart 1952, S. 141) umsetzt. Analog können Verbindungen der Formel I, in der R¹ für -S-C(O)-NH-R²⁸ steht, beispielsweise hergestellt werden, indem man Verbindungen der Formel I, in der R¹ für -SH steht, mit Isocyanaten der Formel O=C=N-R²⁸ umsetzt. Analog können Isothiocyanate der Formel S=C=N-R²⁸ umgesetzt werden. Generell können zur Herstellung von Verbindungen der Formel I, in der R¹ für -O-C(O)-N(R²¹)-R²⁸ oder -S-C(O)-N(R²¹)-R²⁸ steht, literaturbekannte Methoden zur Herstellung solcher Kohlensäurederivate Anwendung finden, beispielsweise Umsetzungen mit Carbamidsäurehalogeniden.

Verbindungen der Formel I, in der R¹ für -O-C(O)-R²¹ steht, können beispielsweise hergestellt werden, indem Verbindungen der Formel I, in der R¹ für -OH steht, nach an sich bekannten, in der Literatur beschriebenen Verfahren (vgl. Houben-Weyl, Methoden der Organischen Chemie, Bd. VIII, Georg Thieme Verlag, Stuttgart 1952, S. 508 ff.) umsetzt, beispielsweise mit reaktiven Carbonsäurederivaten. Entsprechend können beispielsweise Verbindungen der Formel I, in der R¹ für -O-C(O)-OR²² steht, aus Verbindungen der Formel I, in der R¹ für -OH steht, mit geeigneten Kohlensäurederivaten wie zum Beispiel Chlorkohlensäureestern erhalten werden.

Gemäß den vorstehenden Ausführungen können die Verbindungen der Formel I, in der die Reste W, Z, Y, B, R, D, E und b, c, d, e, f, g, und h die eingangs angegebenen Bedeutungen haben, auch als Zwischenprodukte für die Herstellung anderer Verbindungen, insbesondere weiterer Arzneimittelwirkstoffe, eingesetzt werden, die aus den Verbindungen der Formel I beispielsweise durch Abwandlung oder Einführung von Resten oder funktionellen Gruppen erhältlich sind.

Die Verbindungen der Formel I, in der die Reste W, Z, Y, B, R, D, E und b, c, d, e, f, g, und h die eingangs angegebenen Bedeutungen haben, sind Antagonisten des Adhäsionsrezeptors VLA-4 undloder Inhibitoren der Leukozytenadhäsion. Dies gilt in gleichem Maße auch für die bereits in der WO-A-96/33976 beschriebenen Verbindungen, die nach der eingangs angegebenen Definition derjenigen Verbindungen, die per se in der vorliegenden Anmeldung beansprucht werden, ausgeschlossen sind, für die aber in der WO-A-96/33976 keine pharmakologische Wirkung oder pharmazeutische Verwendung beschrieben ist. Die folgenden Ausführungen zur pharmakologischen Wirkung und Verwendung gelten auch für die letztgenannten Verbindungen. Zur Herstellung der letztgenannten Verbindungen wird auf die WO-A-96/33976 bzw. die deutsche Patentanmeldung 19515177.1 verwiesen, deren Inhalt insofern Bestandteil der vorliegenden Offenbarung ist. Hinsichtlich der Verwendung von Verbindungen und hinsichtlich pharmazeutischer Zubereitungen betrifft die vorliegende Erfindung damit zum einen die Verbindungen der Formel I, in der die Reste W, Z, Y, B, R, D, E und b, c, d, e, f, g, und h die eingangs angegebenen Bedeutungen haben, zum aber ebenso die nach der eingangs angegebenen Definition der beanspruchten Verbindungen ausgeschlossenen, in der WO-A-96/33976 beschriebenen Verbindungen. Hinsichtlich der im folgenden beschriebenen Verwendung und pharmazeutischer Zubereitung sind also Gegenstand der vorliegenden Erfindung Verbindungen der Formel Ib, 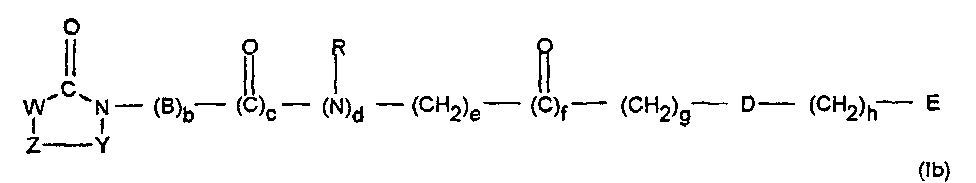 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor ein- oder mehrfach substituiert sein können;
- R⁰: für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)_{2,} -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 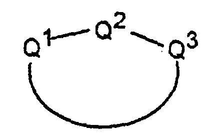 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-Amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, Het-CO, gegebenenfalls substituiertes (C₃-C₈)-Cycloalkyl, HOS(O)₂-(C₁-C₃)-alkyl, R⁹NHS(O)₂-(C₁-C₃)-alkyl, -alkyl, (R⁸O)₂P(O)-(C₁-C₃)-alkyl, -alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: für Wasserstoff, R^{12a}, R^{12a}-CO, H-CO, R^{12a}-O-CO, R^{12b}-CO, R^{12b}-CS, R^{12a}-S(O)₂ oder R^{12b}-S(O)₂ steht;
- R^{12a}: (C₁-C₁₈)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
- R^{12b}: Amino, Di-((C₁-C₁₈)-alkyl)-amino oder R^{12a}-NH bedeutet;
- R¹³: Wasserstoff, (C₁-C₆)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder (C₃-C₈)-Cycloalkyl-(C₁-C₈)-alkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein,zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
- R²⁸: für einen der Reste R²¹-, R²¹N(R²¹)-, R²¹C(O)-, R²²O-C(O)-, R²¹N(R²¹)-C(O)- oder R²¹N(R²¹)-C(=N(R²¹))- steht;
- R²⁹: für einen der Reste R²²-, R²¹N(R²¹)-, R²¹C(O)-, R²²O-C(O)-, R²¹N(R²¹)-C(O)- oder R²¹N(R²¹)-C(=N(R²¹))- steht;
- Het: für den Rest eines über ein Stickstoffatom gebundenen 5- bis 10-gliedrigen, monocyclischen oder polycyclischen Heterocyclus steht, der aromatisch oder teilweise ungesättigt oder gesättigt sein kann und der ein, zwei, drei oder vier gleiche oder verschiedene zusätzliche Ring-Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring-Stickstoffatomen gleiche oder verschiedene Reste R^{h}, R^{h}CO oder R^{h}O-CO als Substituenten stehen können und R^{h} für Wasserstoff, (C₁-C₈)-Alkyl, (C₃-C₈)-Cycloalkyl, (C₃-C₈)-Cycloalkyl-(C₁-C₈)-alkyl, gegebenenfalls substituiertes (C₆-C₁₄)-Aryl oder im Arylrest gegebenenfalls substituiertes (C₆-C₁₄)-Aryl-(C₁-C₈)-alkyl steht;
b, c, d und f unabhängig voneinander für 0 oder 1 stehen, aber nicht alle gleichzeitig
0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Alle obigen Erläuterungen zu den Verbindungen der Formel 1, zum Beispiel hinsichtlich Alkylresten, Arylresten, usw. gelten für die Verbindungen der Formel Ib entsprechend. Auch hier werden alle Stereoisomeren umfaßt. Ebenso gelten alle oben angegebenen bevorzugten Bedeutungen und bevorzugten Verbindungen ausdrücklich auch hier für die Verbindungen der Formel Ib entsprechend. Hinsichtlich der Verwendung und der pharmazeutischen Zubereitungen sind bevorzugte Verbindungen der Formel Ib zum Beispiel also wiederum solche Verbindungen, worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R und R⁰: unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
- R¹: einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-M(R²¹)-R²⁸, -O-C(S)-N(R²¹)-R²⁸, -O-S(O)₂-OR²¹, -O-S(O)-OR²¹, -O-S(O)2-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, (R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 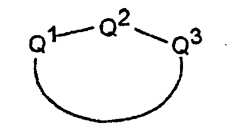 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht, wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
- R⁵: gegebenenfalls substituiertes (C₆-C₁₄)-Aryl, im Arylrest gegebenenfalls substituiertes (C₆-C₁₄)-Ayyl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert undloder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
- R¹²: Wasserstoff, (C₁-C₁₈)-Alkyl, (C₂-C₈) Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- bedeutet;
- R¹³: Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- R²¹: Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
- R²²: (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
- R²⁸: für einen der Reste R²¹-, R²¹N(R²¹)-, R²¹C(O)-, R²²O-C(O)-, R²¹N(R²¹)-C(O)- oder R²¹N(R²¹)-C(=N(R²¹))- steht;
- R²⁹: für einen der Reste R²²-, R²¹N(R²¹)-, R²¹N(R²¹)-, R²¹C(O)-, R²¹N(R²¹)-C(O)- oder R²¹N(R²¹)-C(=N(R²¹))- steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Die Verbindungen der Formel Ib gemäß der vorstehenden Definition haben die Fähigkeit, den VLA-4-Adhäsionsrezeptor zu inhibieren und Zell-Zell- und Zell-Matrix-lnteraktionsprozesse zu inhibieren, bei denen Wechselwirkungen zwischen VLA-4 mit seinen Liganden eine Rolle spielen. Die Wirksamkeit der Verbindungen der Formel Ib kann beispielsweise in einem Assay nachgewiesen werden, in dem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel Ib die Adhäsion und die Migration von Leukozyten inhibieren, etwa die Anheftung von Leukozyten an endotheliale Zellen, die - wie oben erläutert - über den VCAM-1/VLA-4-Adhäsionsmechanismus gesteuert wird.

Die Verbindungen der Formel Ib und ihre physiologisch verträglichen Salze eignen sich daher zur Behandlung und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Behandlung und Prophylaxe von Krankheiten, die zumindest teilweise durch ein unerwünschtes Ausmaß an Leukozytenadhäsion und/oder Leukozytenmigration verursacht werden oder damit verbunden sind, und zu deren Vorbeugung, Linderung oder Heilung die Adhäsion und/oder Migration von Leukozyten verringert werden soll. Sie können somit zum Beispiel bei entzündlichen Erscheinungen unterschiedlichster Ursache als Entzündungshemmer eingesetzt werden. Anwendung finden die Verbindungen der Formel Ib gemäß der vorliegenden Erfindung beispielsweise zur Behandlung oder Prophylaxe der rheumatoiden Arthritis, der inflammatorischen bowel disease (ulcerativen Colitis), des systemischen Lupus erythematosus oder zur Behandlung oder Prophylaxe von inflammatorischen Erkrankungen des zentralen Nervensystems, wie zum Beispiel der multiplen Sklerose, zur Behandlung oder Prophylaxe von Asthma oder von Allergien, z. B. Allergien vom verzögerten Typ (Typ IV-Allergie), weiterhin zur Behandlung oder Prophylaxe von cardiovaskulären Erkrankungen, der Arteriosklerose, von Restenosen, zur Behandlung oder Prophylaxe von Diabetes, zur Verhinderung der Schädigung von Organtransplantaten, zur Hemmung von Tumorwachstum oder Tumormetastasierung bei verschiedenen Malignitäten, zur Therapie der Malaria, sowie von weiteren Krankheiten, bei denen eine Blockierung des Integrins VLA-4 und/oder eine Beeinflussung der Leukozytenaktivität zur Vorbeugung, Linderung oder Heilung angebracht erscheint. Die Verbindungen der Formel Ib und ihre Salze können weiterhin für diagnostische Zwecke, zum Beispiel bei in vitro-Diagnosen, und als Hilfsmittel in biochemischen Untersuchungen eingesetzt werden, bei denen eine VLA-4-Blockierung oder eine Beeinflussung von Zell-Zell- oder Zell-Matrix-Interaktionen angestrebt wird.

Die Verbindungen der Formel Ib und ihre physiologisch verträglichen Salze können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel Ib und/oder ihrer physiologisch verträglichen Salze neben üblichen pharmazeutisch einwandfreien Trägerstoffen und/oder Zusatzstoffen enthalten. Die pharmazeutischen Zubereitungen enthalten normalerweise etwa 0,5 bis 90 Gew.-% der therapeutisch wirksamen Verbindungen der Formel Ib und/oder ihrer physiologisch verträglichen Salze.

Gegenstand der vorliegenden Erfindung sind die Verbindungen der Formel Ib und/oder ihre physiologisch verträglichen Salze zur Verwendung als Arzneimittel, die Verwendung der Verbindungen der Formel Ib und/oder ihrer physiologisch verträglichen Salze zur Herstellung von Arzneimitteln zur Hemmung der Adhäsion und/oder Migration von Leukozyten oder zur Hemmung des VLA-4-Rezeptors, also von Arzneimitteln zur Behandlung oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, insbesondere zur Herstellung von Arzneimitteln zur Entzündungshemmung, sowie die Verwendung der Verbindungen der Formel Ib und/oder ihrer physiologisch verträglichen Salze bei der Behandlung und Prophylaxe derartiger Krankheiten. Weiterhin sind Gegenstand der vorliegenden Erfindung pharmazeutische Zubereitungen, die eine wirksame Dosis mindestens einer Verbindung der Formel Ib und/oder ihrer physiologisch verträglichen Salze neben üblichen pharmazeutisch einwandfreien Trägerstoffen und/oder Zusatzstoffen enthalten.

Die Arzneimittel können oral, zum Beispiel in Form von Pillen, Tabletten, Filmtabletten, Dragees, Granulaten, Hart- und Weichgelatinekapseln, Lösungen, Sirupen, Emulsionen oder Suspensionen verabreicht werden. Die Verabreichung kann aber auch rektal, zum Beispiel in Form von Suppositorien, oder parenteral, zum Beispiel in Form von Injektions- oder Infusionslösungen, Mikrokapseln oder Rods, oder perkutan, zum Beispiel in Form von Salben oder Tinkturen, oder auf anderem Wege, zum Beispiel in Form von Nasalsprays oder Aerosolmischungen, erfolgen.

Die Herstellung der erfindungsgemäß einzusetzenden pharmazeutischen Zubereitungen erfolgt in an sich bekannter Weise, wobei neben der oder den Verbindungen der Formel Ib und/oder ihren physiologisch verträglichen Salzen pharmazeutisch inerte anorganische oder organische Trägerstoffe verwendet werden. Für die Herstellung von Pillen, Tabletten, Dragees und Hartgelatinekapseln kann man zum Beispiel Lactose, Maisstärke oder Derivate davon, Talk, Stearinsäure oder deren Salze etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Glycerin, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure.

Die pharmazeutischen Zubereitungen können neben den Wirk- und Trägerstoffen noch Zusatzstoffe, wie zum Beispiel Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel Ib und/oder deren physiologisch verträgliche Salze enthalten. Ferner können sie neben mindestens einer Verbindung der Formel Ib und/oder ihren physiologisch verträglichen Salzen noch einen oder mehrere andere therapeutisch oder prophylaktisch wirksame Stoffe enthalten, zum Beispiel Stoffe mit entzündungshemmender Wirkung.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,01 bis 100 mg/kg, vorzugsweise 0,1 bis 10 mg/kg, insbesondere 0,3 bis 2 mg/kg Körpergewicht zur Erzielung wirksamer Ergebnisse angemessen. Bei intravenöser Applikation beträgt die Tagesdosis im allgemeinen etwa 0,01 bis 50 mg/kg, vorzugsweise 0,01 bis 10 mg/kg Körpergewicht. Die Tagesdosis kann, insbesondere bei der Applikation größerer Mengen, in mehrere, zum Beispiel 2, 3, oder 4, Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Zubereitungen enthalten normalerweise 0,2 bis 500 mg, vorzugsweise 1 bis 100 mg Wirkstoff der Formel Ib und/oder dessen physiologisch verträgliche Salze pro Dosis.

### Beispiele

Die Produkte wurden über Massenspektren (MS) und/oder NMR-Spektren identifiziert. Verbindungen, die durch Chromatographie unter Verwendung eines Laufmittels gereinigt wurden, das beispielsweise Essigsäure oder Trifluoressigsäure enthielt, und anschließend gefriergetrocknet wurden, haben zum Teil je nach Durchführung der Gefriertrocknung noch die aus dem Laufmittel stammende Säure enthalten, wurden also teilweise oder vollständig in Form eines Salzes, zum Beispiel in Form des Essigsäuresalzes oder Trifluoressigsäuresalzes, erhalten.

### Es bedeuten:

| | |
|---|---|
| THF | Tetrahydrofuran |
| DMF | N,N-Dimethylformamid |
| DCC | N,N'-Dicyclohexylcarbodiimid |
| HOBt | 1-Hydroxybenzotriazol |

### Beispiel 1

### ((R,S)-4-(4-Cyanophenyl)-4-methyl-3-((2-naphthyl)-methyl)-2,5-dioxoimidazolidin-1-yl)-acetyl-L-aspartyl-L-phenylglycin

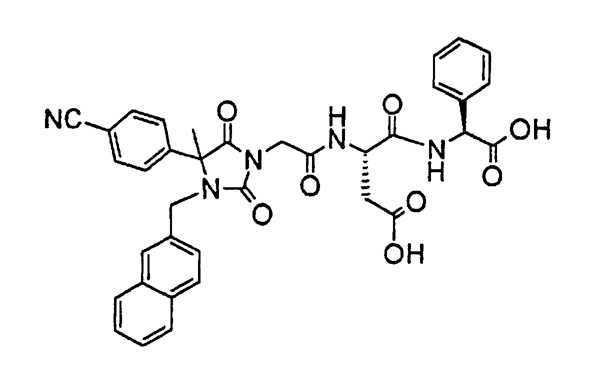

### 1a) (R,S)-4-(4-Cyanophenyl)-4-methyl-2,5-dioxoimidazolidin

20 g (138 mmol) p-Acetylbenzonitril, 115,6 g Ammoniumcarbonat (1,21 mol) und 11,6 g Kaliumcyanid (178 mmol) wurden in 600 ml einer Mischung aus 50 % Ethanol und 50 % Wasser gelöst. Das Gemisch wurde 5 Stunden bei 55 °C gerührt und über Nacht bei Raumtemperatur stehen gelassen. Die Lösung wurde mit 6 N HCI auf pH = 6,3 eingestellt und anschließend zwei Stunden bei Raumtemperatur gerührt. Der Niederschlag wurde abgesaugt, mit Wasser gewaschen und über Phosphorpentoxid im Hochvakuum getrocknet. Ausbeute: 22,33 g (75 %).
FAB-MS: 216,1 (M + H)⁺

### 1b) ((R,S)-4-(4-Cyanophenyl)-4-methyl-2,5-dioxoimidazolidin-1-yl)-essigsäuremethylester

1,068 g Natrium (46,47 mmol) wurden unter Stickstoff in 110 ml abs. Methanol gelöst. Die klare Lösung wurde mit 10 g (R,S)-4-(4-Cyanophenyl)-4-methyl-2,5-dioxoimidazolidin (46,47 mmol) versetzt und das Gemisch wurde 2 h unter Rückfluß gekocht. Man gab 7,75 g (46,68 mmol) Kaliumiodid zu und tropfte innerhalb einer Stunde eine Lösung von 4,53 ml Chloressigsäuremethylester (51,3 mmol) in 5 ml Methanol zu. Es wurde 6 Stunden zum Sieden erhitzt, über Nacht bei Raumtemperatur stehen gelassen und eingeengt. Der ölige Rückstand wurde über Kieselgel mit Methylenchlorid/Essigester (9:1) chromatographiert. Ausbeute: 8,81 g (66 %).
FAB-MS: 288 (M + H)⁺

### 1c) ((R,S)-4-(4-Cyanophenyl)-4-methyl-3-((2-naphthyl)-methyl)-2,5-dioxoimidazolidin-1-yl)-essigsäure-methylester

5 g ((R,S)-4-(4-Cyanophenyl)-4-methyl-2,5-dioxoimidazolidin-1-yl)-essigsäuremethylester (17,4 mmol) wurden unter Argon in 20 ml wasserfreiem DMF gelöst. Im Argon-Gegenstrom wurden 920 mg einer Natriumhydrid-Dispersion in Mineralöl (19,14 mmol) zugegeben. Das Reaktionsgemisch wurde 15 Minuten bei Raumtemperatur gerührt. Anschließend setzte man eine Lösung von 3,85 g 2-Brommethylnaphthalin (19,14 mmol) in 10 ml wasserfreiem DMF zu. Es wurde 4 Stunden bei Raumtemperatur gerührt und dann über Nacht bei Raumtemperatur stehen gelassen. Die Lösung wurde konzentriert. Zur Reinigung wurde die Substanz über Kieselgel mit Methylenchlorid/Essigester (9,75:0,25) chromatographiert. Die Fraktionen mit der reinen Substanz wurden eingeengt. Ausbeute: 5,15 g Öl (69 %).
FAB-MS: 428,3 (M + H)⁺

### 1d) ((R,S)-4-(4-Cyanophenyl)-4-methyl-3-((2-naphthyl)-methyl)-2,5-dioxoimidazolidin-1-yl)-essigsäure

1,1 g ((R,S)-4-(4-Cyanophenyl)-4-methyl-3-((2-naphthyl)-methyl)-2,5-dioxoimidazolidin-1-yl)-essigsäure-methylester (2,57 mmol) wurden in einer Mischung aus 20 ml 6 N HCl und 10 ml Dioxan gelöst. Die Lösung wurde 3 Stunden bei 70 °C gerührt und dann eingeengt. Ausbeute: 1,2 g Rohprodukt.
FAB-MS: 414,2 (M + H)⁺

### 1e) ((R,S)-4-(4-Cyanophenyl)-4-methyl-3-((2-naphthyl)-methyl)-2,5-dioxoimidazolidin-1-yl)-acetyl-L-aspartyl-L-phenylglycin-di-tert-butylester

Zu einer Lösung von 1,2 g ((R,S)-4-(4-Cyanophenyl)-4-methyl-3-((2-naphthyl)-methyl)-2,5-dioxoimidazolidin-1-yl)-essigsäure (Rohprodukt), 0,97 g H-Asp(OBu^{t})-Phg-OBu^{t}-Hydrochlorid (2,34 mmol) und 320 mg HOBt (2,34 mmol) in 25 ml DMF gab man bei 0 °C 515 mg DCC (2,34 mmol). Man ließ eine Stunde bei 0 °C und 3 Stunden bei Raumtemperatur rühren. Anschließend ließ man den Ansatz über Nacht bei Raumtemperatur stehen, saugte den Niederschlag ab und engte das Filtrat ein. Zur Reinigung wurde die Substanz über Kieselgel zuerst mit Methylenchlorid/Methanol/Eisessig (9,5:0,5:0,05) und dann mit Methylenchlorid/Essigester (8:2) chromatographiert. Ausbeute: 620 mg Öl (34,4 %).
FAB-MS: 774,3 (M + H)⁺

### 1f) ((R,S)-4-(4-Cyanophenyl)-4-methyl-3-((2-naphthyl)-methyl)-2,5-dioxoimidazolidin-1-yl)-acetyl-L-aspartyl-L-phenylglycin

250 mg ((R,S)-4-(4-Cyanophenyl)-4-methyl-3-((2-naphthyl)-methyl)-2,5-dioxoimidazolidin-1-yl)-acetyl-L-aspartyl-L-phenylglycin-di-tert-butylester wurden in einer Mischung aus 2,25 ml Trifluoressigsäure und 0,25 ml Wasser gelöst. Man ließ eine Stunde bei Raumtemperatur stehen und engte im Wasserstrahlvakuum ein. Zur Reinigung wurde die Substanz über Sephadex LH20 mit einer Mischung aus Eisessig, n-Butanol und Wasser chromatographiert. Die Fraktionen mit der reinen Substanz wurden eingeengt. Der Rückstand wurde in Wasser gelöst, gefriergetrocknet und zur weiteren Reinigung über Kieselgel mit Methylenchlorid/Methanol/Eisessig/Wasser (9:1:0,01:0,1) chromatographiert. Ausbeute: 78 mg (36,8 %).
FAB-MS: 662,2 (M + H)⁺

### Beispiel 2

### ((R,S)-3-Benzyl-4-methyl-4-(4-nitro-phenyl)-2,5-dioxoimidazolidin-1-yl)-acetyl-Laspartyl-L-phenylglycin

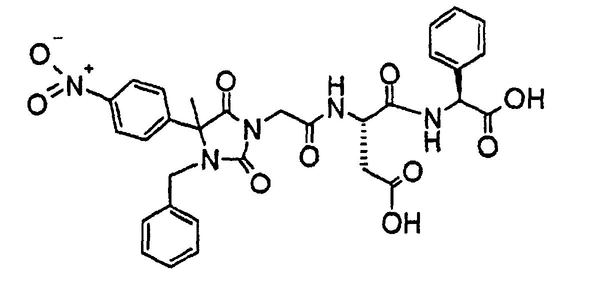

### 2a) (R,S)-4-Methyl-4-(4-nitro-phenyl)-2,5-dioxoimidazolidin

Ein Gemisch aus 20 g (121 mmol) 4-Nitroacetophenon, 101,65 g (1,06 mol) Ammoniumcarbonat und 10,2 g (156 mmol) Kaliumcyanid in 400 ml Ethanol/Wasser (1:1) wurden 5 h bei 50 °C erhitzt. Anschließend wurde die Lösung mit 6 N Salzsäure auf pH = 6,3 eingestellt und 2 h bei Raumtemperatur gerührt. Der Niederschlag wurde abgesaugt, mit Wasser gewaschen und über Phosphorpentoxid im Hochvakuum getrocknet. Ausbeute: 27,37 g (96 %) farbloser Feststoff.

### 2b) ((R,S)-3-Benzyl-4-methyl-4-(4-nitro-phenyl)-2,5-dioxoimidazolidin-1-yl)-essigsäure

Die Verbindung wurde analog Beispiel 1 durch Umsetzung von (R,S)-4-Methyl-4-(4-nitro-phenyl)-2,5-dioxoimidazolidin mit Bromessigsäuremethylester und anschließend Benzylbromid (an Stelle von 2-Brommethylnaphthalin) und Spaltung des Methylesters mit 6 N Salzsäure hergestellt.

### 2c) ((R,S)-3-Benzyl-4-methyl-4-(4-nitro-phenyl)-2,5-dioxoimidazolidin-1-yl)-acetyl-Laspartyl-L-phenylglycin

Eine Lösung von 383 mg (1 mmol) ((R,S)-3-Benzyl-4-methyl-4-(4-nitro-phenyl)-2,5-dioxoimidazolidin-1-yl)-essigsäure, 414 mg (1mmol) H-Asp(O^{t}Bu)-Phg-(O^{t}Bu) x HCl und 135 mg (1 mmol) HOBt in 5 ml absolutem DMF wurde bei 0 °C mit 220 mg (1,1 mmol) DCC versetzt. Nach 60 Minuten Rühren bei 0 °C und 60 Minuten bei Raumtemperatur wurde der Niederschlag abgesaugt, das Filtrat eingeengt und der Rückstand in Essigester aufgenommen. Die Essigester-Lösung wurde nach Filtration nacheinander mit gesättigter NaHCO₃-Lösung, KHSO₄/K₂SO₄-Lösung, gesättigter NaHCO₃-Lösung und Wasser gewaschen. Nach Phasentrennung wurde die organische Phase über Natriumsulfat getrocknet. Das Trockenmittel wurde abfiltriert, das Lösungsmittel im Vakuum entfernt und der Rückstand mit Dichlormethan/Essigester (9:1) über Kieselgel chromatographiert. Nach Einengen der Produktfraktionen wurde der Rückstand mit 10 ml 90-%iger Trifluoressigsäure versetzt. Nach 1 h bei Raumtemperatur wurde die Trifluoressigsäure im Vakuum entfernt und der Rückstand mit Dichlormethan/Methanol/Essigsäure/Wasser (9:1:0,1:0,1) über Kieselgel und anschließend mit Wasser/Butanol/Essigsäure (43:4,3:3,5) über Sephadex LH20 chromatographiert. Nach Gefriertrocknen der Produktfraktionen erhielt man 23 mg (4 %) der Titelverbindung.
ES(+)-MS: 632 (M+H)⁺

### Beispiel 3

### ((R,S)-3-Senzyl-4-methyl-4-(4-(3-(2-methyl-phenyl)-ureido)-phenyl)-2,5-dioxoimidazolidin-1-yl)-acetyl-L-aspartyl-L-phenylglycin

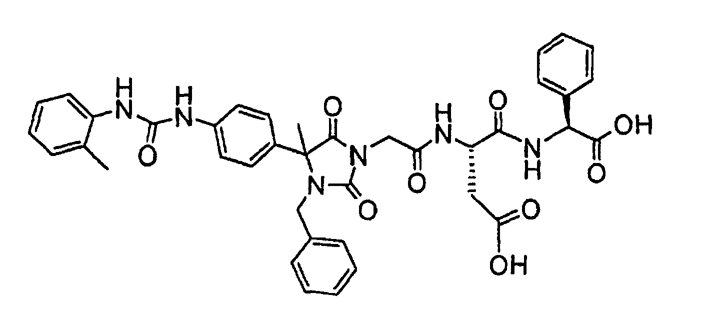

### 3a) ((R,S)-3-Benzyl-4-methyl-4-(4-nitro-phenyl)-2,5-dioxoimidazolidin-1-yl)-essigsäure-methylester

Die Verbindung wurde analog Beispiel 1 aus (R,S)-4-Methyl-(4-nitro-phenyl)-2,5-dioxoimidazolidin (synthetisiert aus 4-Nitroacetophenon wie in Beispiel 2 beschrieben) hergestellt.

### 3b) ((R,S)-4-(4-Amino-phenyl)-3-benzyl-4-methyl-2,5-dioxoimidazolidin-1-yl)-essigsäure-methylester

Eine Lösung von 8,92 g (22,45 mmol) an ((R,S)-3-Benzyl-4-methyl-4-(4-nitrophenyl)-2,5-dioxoimidazolidin-1-yl)-essigsäure-methylester in 280 ml absolutem Methanol wurde mit 17 g (90 mmol) Zinnchlorid und 3 Tropfen Essigsäure versetzt und bei 50 °C erhitzt. Nachdem laut HPLC-Kontrolle kein Edukt mehr nachweisbar war wurde das Reaktionsgemisch im Vakuum eingeengt und der Rückstand mit Methanol über Kieselgel filtriert. Nach Einengen erhielt man 6,39 g (78 %) der Titelverbindung.

### 3c) ((R,S)-3-Benzyl-4-methyl-4-(4-(3-(2-methyl-phenyl)-ureido)-phenyl)-2,5-dioxoimidazolidin-1-yl)-essigsäure-methylester

Man gab zu einer Lösung von 2,5 g (6,8 mmol) an ((R,S)-4-(4-Amino-phenyl)-3-benzyl-4-methyl-2,5-dioxoimidazolldin-1-yl)-essigsäure-methylester in 20 ml THF 0,91 g (6,8 mmol) ortho-Tolylisocyanat in 2 ml THF. Nach 5 h Erhitzen unter Rückfluß ließ man das Reaktionsgemisch über Nacht bei Raumtemperatur stehen, gab weitere 0,18 g (1,36 mmol) ortho-Tolylisocyanat zu und ließ 3 h unter Rückfluß rühren. Das Reaktionsgemisch wurde eingeengt und der Rückstand mit Heptan/Essigester (1:1) mittels MPLC über Kieselgel gereinigt. Nach Einengen der Produktfraktionen erhielt man 1,35 g (40 %) der Titelverbindung.

### 3d) ((R,S)-3-benzyl-4-methyl-4-(4-(3-(2-methyl-phenyl)-ureido)-phenyl)-2,5-dioxoimidazolidin-1-yl)-acetyl-L-aspartyl-L-phenylglycin

Die Synthese erfolgte analog Beispiel 2 durch Kupplung von ((R,S)-3-Benzyl-4-methyl-4-(4-(3-(2-methyl-phenyl)-ureido)-phenyl)-2,5-dioxoimidazolidin-1-yl)-essigsäure (hergestellt durch Spaltung von ((R,S)-3-Benzyl-4-methyl-4-(4-(3-(2-methyl-phenyl)-ureido)-phenyl)-2,5-dioxoimidazolidin-1-yl)-essigsäure-methylester mit 6 N Salzsäure analog Beispiel 1) und H-Asp(O^{t}Bu)-Phg-(O^{t}Bu) x HCl. Nach Spaltung des tert-Butylesters mit 90-%iger Trifluoressigsäure wurde das Rohprodukt durch präparative HPLC über RP-18 gereinigt.
ES(+)-MS: 735 (M+H)⁺

### Beispiel 4

### ((R,S)-3-Benzyl-4-(4-(3-benzyl-ureido-methyl)-phenyl)-4-methyl-2, 5-dioxoimidazolidin-1-yl)-acetyl-L-aspartyl-L-phenylglycin

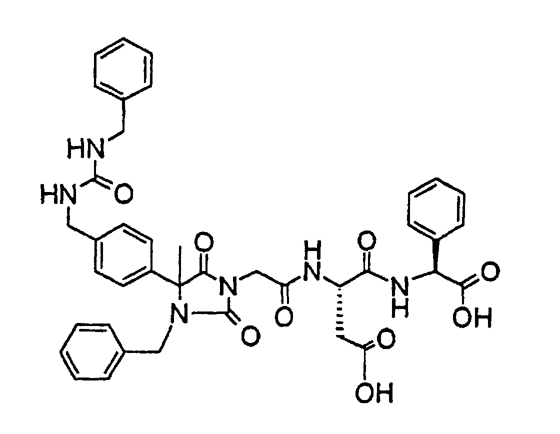

### 4a) ((R,S)-3-Benzyl-4-(4-cyanophenyl)-4-methyl-2,5-dioxoimidazolidin-1-yl)-essigsäure-methylester

28,7 g (100 mmol) ((R,S)-4-(4-Cyanophenyl)-4-methyl-2,5-dioxoimidazolidin-1-yl)-essigsäure-methylester wurden unter Argon in 160 ml wasserfreiem DMF gelöst. Bei 0 °C wurden unter Rühren portionsweise 5,28 g (110mmol) NaH zugegeben. Man rührte 30 Minuten bei 0 °C nach. Anschließend tropfte man langsam 13 ml Benzylbromid zu. Man rührte 5 Stunden bei Raumtemperatur und ließ über Nacht bei Raumtemperatur stehen. Die fast klare Lösung wurde abgesaugt und am Hochvakuum eingeengt. Der Rückstand wurde in Essigester gelöst, mit Wasser gewaschen und die wässerige Phase mit Essigester gewaschen. Die vereinigten organischen Phasen wurden mit Wasser gewaschen, mit wasserfreiem Magensiumsulfat getrocknet und eingeengt. Zur Reinigung wurde das Rohprodukt über Kieselgel (70-200 µm) in n-Heptan/Essigester (1:1) chromatographiert. Man erhielt 35,7 g (94,6 %) der Titelverbindung als Öl.

### 4b) ((R,S)-4-(4-(Aminomethyl)-phenyl)-3-benzyl-4-methyl-2,5-dioxoimidazolidin-1-yl)-essigsäure-methylester

15,2 g (40 mmol) ((R,S)-3-Benzyl-4-(4-cyanophenyl)-4-methyl-2,5-dioxoimidazolidin-1-yl)-essigsäure-methylester (4a) wurden in 160 ml eines Gemisches aus Ethanol und 50-%iger Essigsäure (8:2) gelöst, mit 3 g Pd/Kohle vesetzt und 7 Stunden im Autoklaven bei 3 bar H₂ hydriert. Der Katalysator wurde abgesaugt und das Filtrat eingeengt. Der Rückstand wurde in Dichlormethan gelöst und über Kieselgel (70-200 µm) unter Verwendung von Dichlormethan und anschließend Dichlormethan/Methanol (8:2) chromatographiert. Man erhielt 15,3 g (100 %) der Titelverbindung.

### 4c) ((R,S)-3-Benzyl-4-(4-(3-benzyl-ureido-methyl)-phenyl)-4-methyl-2,5-dloxolmldazolidin-1-yl)-essigsäure-methylester

3,80 g (10 mmol) ((R,S)-4-(4-(Aminomethyl)-phenyl)-3-benzyl-4-methyl-2,5-dioxoimidazolidin-1-yl)-essigsäure-methylester (4b) wurden unter Argon in 20 ml wasserfreiem Dichlormethan gelöst. Dazu gab man 1,85 ml (2 g; 15mmol) Benzylisocyanat und 2 Tropfen Triethylamin zu. Man rührte bei Raumtemperatur 2 Stunden und engte die Lösung etwas ein. Es wurde mit Essigester verdünnt und 2x mit 5-%iger Zitronensäure, 2x mit gesättigter NaHCO₃-Lösung und 1x mit Wasser/NaCl-Lösung gewaschen. Man trocknete über wasserfreiem Natriumsulfat und engte ein. Man erhielt 5,09 g (98,9 %) der Titelverbindung als Öl.

### 4d) ((R,S)-3-Benzyl-4-(4-(3-benzyl-ureido-methyl)-phenyl)-4-methyl-2,5-dioxoimidazolidin-1-yl)-essigsäure

1 g (1,94 mmol) ((R,S)-3-Benzyl-4-(4-(3-benzyl-ureido-methyl)-phenyl)-4-methyl-2,5-dioxoimidazolidin-1-yl)-essigsäure-methylester (4c) wurden mit 20 ml konzentrierter Salzsäure 5 Stunden zum Rückfluß erhitzt. Anschließend engte man ein. Der Rückstand wurde mit Wasser verrieben, gekühlt und abgesaugt. Man erhielt 700 mg (72 %) der Titelverbindung.

### 4e) ((R,S)-3-Benzyl-4-(4-(3-benzyl-ureido-methyl)-phenyl)-4-methyl-2,5-dioxoimidazolidin-1-yl)-acetyl-L-aspartyl-L-phenylglycin-di-tert-butylester

500 mg (1 mmol) ((R,S)-3-Benzyl-4-(4-(3-benzyl-ureido-methyl)-phenyl)-4-methyl-2,5-dioxoimidazolidin-1-yl)-essigsäure (4d), 414,9 mg (1 mmol) H-Asp(O^{t}Bu)-Phg-(O^{t}Bu) x HCl und 135 mg (1 mmol) HOBt wurden in 10 ml absolutem DMF gelöst. Bei 0°C gab man 0,13 ml (1 mmol) N-Ethylmorpholin und 220 mg (1,1 mmol) DCC zu. Es wurde 1 Stunde bei 0 °C und 3 Stunden bei Raumtemperatur gerührt. Man ließ über Nacht bei Raumtemperatur stehen, saugte den Harstoff-Niederschlag ab und engte am Hochvakuum ein. Der Rückstand wurde in Essigester aufgenommen, mit gesättigter NaHCO₃-Lösung, KHSO₄/K₂SO₄-Lösung sowie mit Wasser/NaCl-Lösung gewaschen. Die organische Phase wurde mit wasserfreiem Natriumsulfat getrocknet, eingeengt und der ölige Rückstand am Hochvakuum getrocknet. Man erhielt 800 mg (92,9 %) der Titelverbindung.

### 4f) ((R,S)-3-Benzyl-4-(4-(3-benzyl-ureido-methyl)-phenyl)-4-methyl-2,5-dioxoimidazolidin-1-yl)-acetyl-L-aspartyl-L-phenylglycin

800 mg (0,93 mmol) ((R,S)-3-Benzyl-4-(4-(3-benzyl-ureido-methyl)-phenyl)-4-methyl-2,5-dloxoimidazolidin-1-yl)-acetyl-L-aspartyl-L-phenylglycin-di-tert-butylester (4e) wurden in 8 ml 90-%iger Trifluoresssigsäure gelöst und 1 Stunde bei Raumtemperatur stehen gelassen. Anschießend wurde eingeengt und der Rückstand mit Diethylether verrieben. Das Rohprodukt wurde durch Chromatographie über Kieselgel mit Dichlormethan/Methanol/Essigsäure/Wasser (9:1:0,1:0,1) gereinigt. Man erhielt 527 mg (76 %) der Titelverbindung.
ES(+)-MS: 749,3 (M+H)⁺

### Beispiel 5

### ((R,S)-3-Benzyl-4-methyl-4-(4-(3-(phenyl-ureido)-methyl)-phenyl)-2,5-dioxoimidazolidin-1-yl)-acetyl-L-aspartyl-L-phenylglycin

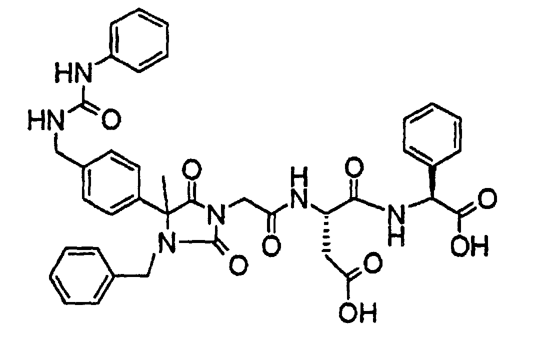

Die Synthese erfolgte analog Beispiel 4. Nach Spaltung des tert-Butylesters mit 90-%iger Trifluoressigsäure wurde das Rohprodukt durch Chromatographie über Kieselgel mit Dichlormethan/Methanol/Essigsäure/Wasser (9:1:0,1:0,1) gereinigt.
ES(+)-MS: 735,2 (M+H)⁺

### Beispiel 6

### ((R, S)-3-Benzyl-4-methyl-4-(4-(3-(2-methyl-phenyl)-ureido-methyl)-phenyl)-2,5-dioxoimidazolidin-1-yl)-acetyl-L-aspattyl-L-phenylglycin

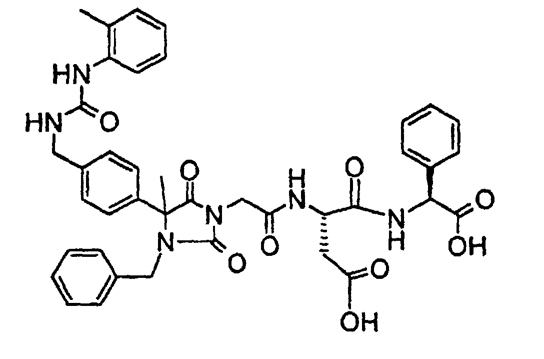

Die Synthese erfolgte analog Beispiel 4. Nach Spaltung des tert-Butylesters mit 90 %iger Trifluoressigsäure wurde das Rohprodukt durch Chromatographie über Kieselgel mit Dichlormethan/Methanol/Essigsäure/Wasser (9:1:0,1:0,1) gereinigt.
ES(+)-MS: 749,3 (M+H)⁺

### Beispiel 7

### ((R,S)-3-Benzyl-4-methyl-4-(4-(3-(2-phenylethyl)-ureido-methyl)-phenyl)--2,5-dioxoimidazolidin-1-yl)-acetyl-L-aspattyl-L-phenylglycin

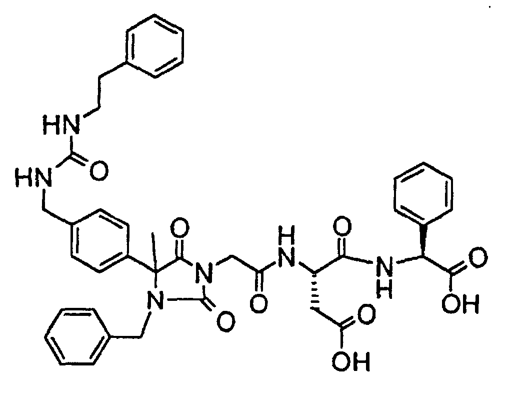

Die Synthese erfolgte analog Beispiel 4. Nach Spaltung des tert-Butylesters mit 90-%iger Trifluoressigsäure wurde das Rohprodukt durch Chromatographie über Kieselgel mit Dichlormethan/Methanol/Essigsäure/Wasser (9:1:0,1:0,1) gereinigt.
ES(+)-MS: 763,3 (M+H)⁺

### Untersuchung der biologischen Aktivität

Als Testmethode für die Wirksamkeit der Verbindungen der Formel Ib auf die Interaktion zwischen VCAM-1 und VLA-4 wird ein Assay verwendet, der für diese Interaktion spezifisch ist. Die zellulären Bindungspartner, d. h. die VLA-4-Integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

### Testmethode :

### Assay zur Messung der Adhäsion von U937-Zellen (ATCC CRL 1593) an hVCAM-1(1-3)-IgG

1. Herstellung von humanem VCAM-1(1-3)-IgG und humanem CD4-IgG
   Eingesetzt wurde ein genetisches Konstrukt zur Expression der extrazellulären Domäne des humanen VCAM-1, verbunden mit der genetischen Sequenz der schweren Kette des humanen Immunglobulins IgG1 (Hinge, CH2 und CH3 Regionen), von Dr. Brian Seed, Massachusetts General Hospital, Boston, USA. Das lösliche Fusionsprotein hVCAM-1(1-3)-IgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextran-vermittelter DNA-Transfektion in COS-Zellen (ATCC CRL 1651) gemäß Standardprozeduren exprimiert (Ausubel et al., Current protocols in molecular biology, John Wiley & Sons, Inc., 1994).
2. Assay zur Messung der Adhäsion von U937-Zellen an hVCAM-1(1-3)-IgG
   2.1 96 well-Mikrotitertestplatten (Nunc Maxisorb) wurden mit 100 µl/well einer Ziege-anti-human-fgG-Antikörperlösung (10 µg/ml in 50 mM Tris, pH 9,5) 1 Stunde bei Raumtemperatur inkubiert. Nach Entfernen der Antikörperlösung wurde einmal mit PBS gewaschen.
   2.2 150 µl/well eines Blockierungspuffers (1 % BSA in PBS) wurde 0,5 Stunden bei Raumtemperatur auf den Platten inkubiert. Nach Entfernen des Blockierungspuffers wurde einmal mit PBS gewaschen.
   2.3 100 µl pro well eines Zellkulturüberstandes von transfektierten COS-Zellen wurde für 1,5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem IgG₁ (hVCAM-1(1-3)-IgG), codiert. Der Gehalt an hVCAM-1(1-3)-IgG betrug ca. 0,5 - 1 µg/ml. Nach Entfernen des Kulturüberstandes wurde einmal mit PBS gewaschen.
   2.4 Die Platten wurden mit 100 µl/well Fc-Rezeptor-Blockpuffer (1 mg/ml γ-Globulin, 100 mM NaCl, 100 µM MgCl₂, 100 µM MnCl₂, 100 µM CaCl₂, 1 mg/ml BSA in 50 mM HEPES, pH 7,5) für 20 Minuten bei Raumtemperatur inkubiert. Nach Entfernen des Fc-Rezeptor-Blockpuffers wurde einmal mit PBS gewaschen.
   2.5 20 µl Bindungspuffer (100 mM NaCl, 100 µM MgCl₂, 100 µM MnCl₂, 100 µM CaCl₂, 1 mg/ml BSA in 50 mM HEPES, pH 7,5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).
   2.6 U937-Zellen wurden 20 Minuten in Fc-Rezeptor-Blockpuffer inkubiert und anschließend in einer Konzentration von 1 x 10⁶/ml und in einer Menge von 100 µl pro weil zupipettiert (Endvolumen 125µl/well).
   2.7 Die Platten wurden in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht und ausgeschlagen. Der Vorgang wurde wiederholt.
   2.8 Anschließend wurden 50 µl/well einer Färbelösung (16,7 µg/ml Hoechst Farbstoff 33258, 4 % Formaldehyd, 0,5 % Triton-X-100 in PBS) 15 Minuten auf den Platten inkubiert.
   2.9 Die Platten wurden ausgeschlagen, in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht. Der Vorgang wurde wiederholt. Anschließend wurde mit der Flüssigkeit in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1(1-3)-IgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration IC₅₀ berechnet, die zu einer Hemmung der Adhäsion um 50 % führt.

Es wurden die folgenden Testergebnisse erhalten:

| Beispiel | U937/VCAM-1 Zelladhäsinostest IC₅₀ (µM) |
|---|---|
| 1 | 30 |
| 2 | 27,7 |
| 3 | 2,8 |
| 4 | 14 |
| 5 | 9 |
| 6 | 6,5 |
| 7 | 20 |

## Patentansprüche

1. Verbindungen der Formel I, 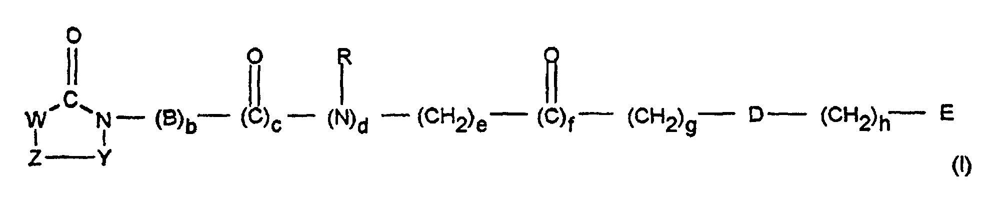 worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor ein- oder mehrfach substituiert sein können;
R⁰ für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-(C₁-C₈)-alkyl-CO, (C₆-C₁₂)-Bicycloalkyl-CO, -(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 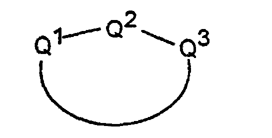 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, Het-CO, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Ayyl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ für Wasserstoff, R^{12a}, R^{12a}-CO, H-CO, R^{12a}-O-CO, R^{12b}-CO, R^{12b}-CS, R^{12a}-S(O)₂ oder R^{12b}-S(O)₂ steht;
R^{12a} (C₁-C₁₈)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
R^{12b} Amino, Di-((C₁-C₁₈)-alkyl)-amino oder R^{12a}-NH bedeutet;
R¹³ Wasserstoff, (C₁-C₆)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder (C₃-C₈)-Cycloalkyl-(C₁-C₈)-alkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein, zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
R²² (C₁-C₈)-Alkyl, Hydroxy-(C₁-C₈)-alkyl, -alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
R²⁸ für einen der Reste R²¹-, R²¹N(R²¹)-, R²¹C(O)-, R²²O-C(O)-, R²¹N(R²¹)-C(O)- oder R²¹N(R²¹)―C(=N(R²¹))― steht;
R²⁹ für einen der Reste R²²-, R²¹N(R²¹)-, R²¹C(O)-, R²²O-C(O)-, R²¹N(R²¹)-C(O)- oder R²¹N(R²¹)―C(=N(R²¹))― steht;
Het für den Rest eines über ein Stickstoffatom gebundenen 5- bis 10-gliedrigen, monocyclischen oder polycyclischen Heterocyclus steht, der aromatisch oder teilweise ungesättigt oder gesättigt sein kann und der ein, zwei, drei oder vier gleiche oder verschiedene zusätzliche Ring-Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring-Stickstoffatomen gleiche oder verschiedene Reste R^{h}, R^{h}CO oder R^{h}O-CO als Substituenten stehen können und R^{h} für Wasserstoff, (C₁-C₈)-Alkyl, (C₃-C₈)-Cycloalkyl, (C₃-C₈)-Cycloalkyl-(C₁-C₈)-alkyl, gegebenenfalls substituiertes (C₆-C₁₄)-Aryl oder im Arylrest gegebenenfalls substituiertes (C₆-C₁₄)-Aryl-(C₁-C₈)-alkyl steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze;
wobei aber, wenn gleichzeitig W für 4-Cyanophenyl-C(R¹³) steht, Y für eine Carbonylgruppe steht, Z für NR^{0a} steht, B für eine unsubstituierte Methylengruppe steht, R für R^{a} steht, b, c und d für 1 stehen und e, f und g für 0 stehen, dann nicht D für C(R^{2a})(R^{3a}) stehen kann, wobei
R^{0a}, R^{a} und R^{2a} unabhängig voneinander für Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl stehen und
R^{3a} für Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder 2-, 3- oder 4-Pyridyl steht.

2. Verbindungen der Formel I gemäß Anspruch 1, worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R und R⁰ unabhängig voneinander Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeuten, wobei Alkylreste durch Fluor ein- oder mehrfach substituiert sein können;
R¹ einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(OR²¹)-N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 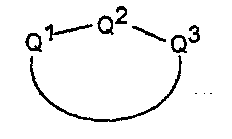 steht, in der
Q¹ für-C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl, R⁹NHS(O)₂ oder den Rest R¹⁵ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino, den Rest R¹⁵ oder den Rest R¹⁵-O- bedeutet;
R¹³ Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Ayyl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
R²² (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
R²⁸ für einen der Reste R²¹-, R²¹N(R²¹)-, R²¹C(O)-, R²²O-C(O)-, R²¹N(R²¹)-C(O)- oder R²¹N(R²¹)―C(=N(R²¹))― steht;
R²⁹ für einen der Reste R²²-, R²¹N(R²¹)-, R²¹C(O)-, R²²O-C(O)-, R²¹N(R²¹)-C(O)- oder R²¹N(R²¹)―C(=N(R²¹))― steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

3. Verbindungen der Formel I gemäß Anspruch 1 und/oder 2, worin R¹ für für einen der Reste
-S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 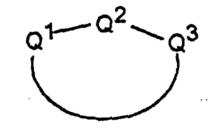 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
in allen ihren stereoisomeren Formen und Mischungen davon in allen Verhältnissen, sowie ihre physiologisch verträglichen Salze.

4. Verbindungen der Formel I gemäß einem oder mehreren der Ansprüche 1 bis 3, worin R⁰ für (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl, bevorzugt für unsubstituiertes oder im Arylrest einfach oder mehrfach substituiertes Biphenylylmethyl, Naphthylmethyl oder Benzyl steht;
in allen ihren stereoisomeren Formen und Mischungen davon in allen Verhältnissen, sowie ihre physiologisch verträglichen Salze.

5. Verbindungen der Formel I gemäß einem oder mehreren der Ansprüche 1, 3 und 4, worin gleichzeitig
W für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰ CO bedeutet;
R Wasserstoff, (C₁-C₆)-Alkyl oder Benzyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)₂-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)₂-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 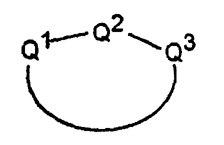 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff oder (C₁-C₈)-Alkyl steht;
R³ für (C₁-C₈)-Alkyl, gegebenenfalls substituiertes (C₆-C₁₄)-Aryl, (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹ NH,
R⁴CO, COOR⁴, CONHR⁴, CSNHR⁴, COOR¹⁵ und CONHR¹⁵ steht;
und e, g und h unabhängig voneinander für die Zahlen 0, 1, 2 oder 3 stehen;
in allen ihren stereoisomeren Formen und Mischungen davon in allen Verhältnissen, sowie ihre physiologisch verträglichen Salze.

6. Verbindungen der Formel I gemäß einem oder mehreren der Ansprüche 1 bis 5, worin W für R¹-A-C(R¹³) und R¹³ für (C₁-C₆)-Alkyl, im Arylrest gegebenenfalls substituiertes (C₆-C₁₄)-Aryl-(C₁-C₈)-alkyl oder (C₃-C₈)-Cycloalkyl steht;
in allen ihren stereoisomeren Formen und Mischungen davon in allen Verhältnissen, sowie ihre physiologisch verträglichen Salze.

7. Verbindungen der Formel I gemäß einem oder mehreren der Ansprüche 1 und 3 bis 6, worin R³ für gegebenenfalls substituiertes (C₆-C₁₄)-Aryl, für COOR⁴, für R¹¹NH oder für CONHR⁴ steht, wobei -NHR⁴ für den Rest einer α-Aminosäure, für deren ω-Amino-(C₂-C₈)-alkylamid, für deren (C₁-C₈)-Alkylester, für deren (C₆-C₁₄)-Aryl-(C₁-C₄)-alkylester oder für deren Derivat steht, in dem die Carbonsäuregruppe in die Gruppe Het-CO überführt ist, bevorzugt für den Rest der α-Aminosäuren Valin, Lysin, Phenylglycin, Phenylalanin oder Tryptophan oder deren (C₁-C₈)-Alkylester, (C₆-C₁₄)-Aryl-(C₁-C₄)-alkylester oder Het-CO-Derivat, steht;
in allen ihren stereoisomeren Formen und Mischungen davon in allen Verhältnissen, sowie ihre physiologisch verträglichen Salze.

8. Verbindungen der Formel I gemäß einem oder mehreren der Ansprüche 1 und 3 bis 7, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl oder Methylenphenylmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ oder Cyano steht;
R² für Wasserstoff steht;
R³ für den Rest CONHR⁴ steht;
R⁴ für Methyl steht, das durch Hydroxycarbonyl und einen Rest aus der Reihe (C₁-C₄)-Alkyl, Phenyl und Benzyl substituiert ist, oder für Methyl steht, das durch (C₁-C₈)-Alkoxycarbonyl, bevorzugt (C₁-C₄)-Alkoxycarbonyl, und einen Rest aus der Reihe (C₁-C₄)-Alkyl, Phenyl und Benzyl substituiert ist, oder für Methyl steht, das durch Het-CO und einen Rest aus der Reihe (C₁-C₄)-Alkyl, Phenyl und Benzyl substituiert ist;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

9. Verbindungen der Formel I gemäß einem oder mehreren der Ansprüche 1, 3, 4 und 5, worin gleichzeitig
W für R¹-A-CH=C und darin A für einen Phenylenrest oder einen Methylenphenylrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl, Methylenphenyl, Methylenphenylmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff oder (C₁-C₆)-Alkyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 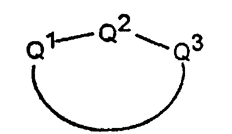 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -NR(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff oder (C₁-C₈)-Alkyl steht;
R³ für CONHR¹⁵ oder CONHR⁴ steht, wobei R⁴ hierin für einen unsubstituierten oder durch einen oder mehrere (C₆-C₁₄)-Arylreste substituierten (C₁-C₈)-Alkylrest steht;
R¹⁵ für R¹⁶-(C₁-C₆)-Alkyl oder R¹⁶ steht, wobei R¹⁶ für einen 7- bis 12-gliedrigen verbrückten bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann, und insbesondere R¹⁵ für einen Adamantylrest oder einen Adamantylmethylrest steht;
und e, g und h unabhängig voneinander für die Zahlen 0, 1, 2 oder 3 stehen und b, c und d für 1 stehen;
in allen ihren stereoisomeren Formen und Mischungen davon in allen Verhältnissen, sowie ihre physiologisch verträglichen Salze.

10. Verbindungen der Formel I gemäß einem oder mehreren der Ansprüche 1, 3, 4, 5, 6 und 9, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl oder Methylenphenylmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -O-C(O)-R²¹, -O-C(O)-OR²², -O-C(O)-N(R²¹)-R²⁸,
-N(R²⁹)-C(O)-OR²², -N(R²⁸)-C(O)-N(R²¹)-R²⁸, -N(R²⁸)-C(S)-N(R²¹)-R²⁸ oder Cyano steht;
R² für Wasserstoff steht;
R³ für CONHR¹⁵ oder CONHR⁴ steht, wobei R⁴ hierin für einen unsubstituierten oder durch einen oder mehrere (C₆-C₁₀)-Arylreste substituierten (C₁-C₆)-Alkylrest steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

11. Verbindungen der Formel I gemäß einem oder mehreren der Ansprüche 1 und 3 bis 6, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl, Methylenphenylmethyl steht;
B für einen unsubstituierten oder substituierten Methylenrest oder Ethylenrest steht;
D für C(R²)(R³) steht;
E für R¹⁰ CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-R²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 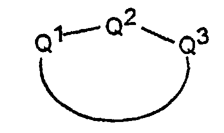 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen unsubstituierten oder substituierten Phenylrest oder Naphthylrest steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

12. Verbindungen der Formel I gemäß einem oder mehreren der Ansprüche 1 und 3 bis 6, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl, Methylenphenyl, Methylenphenylmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für einen der Reste -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)₂-N(R²¹)-R²⁸,
-N(R²⁹)-C(O)-OR²², -N(R²⁸)-C(S)-R²¹,
-N(R²⁸)-C(O)-N(R²¹)R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R^{28,}
-C(S)-R²¹, -C(S)-N(R²¹)-R²⁸ oder Cyano steht oder für den Rest eines gegebenenfalls substituierten, 5- bis 14-gliedrigen, mono- oder polycyclischen, gesättigten oder ungesättigten heterocyclischen Rings der Formel 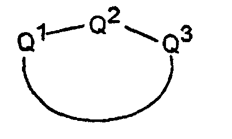 steht, in der
Q¹ für-C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes RingKohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² für Wasserstoff steht;
R³ für R¹¹NH steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und
Isopropoxy steht;
R¹³ für (C₁-C₆)-Alkyl, (C₃-C₇)-Cycloalkyl oder Benzyl, insbesondere Methyl, steht;
b, c, d und e für 1 stehen und f und g für 0 stehen;
h für 0 steht;
in allen ihren stereoisomeren Formen und Mischungen davon in allen Verhältnissen, sowie ihre physiologisch verträglichen Salze.

13. Verbindungen der Formel I gemäß einem oder mehreren der Ansprüche 1, 3, 4, 5, 6 und 12, worin
worin R¹¹ für R^{12a}, R^{12a}-CO, R^{12a}-O-CO, R^{12b}-CO, R^{12b}-CS oder R^{12a}-S(O)₂ steht;
R^{12a} für (C₁-C₁₀)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes
Heteroaryl-(C₁-C₈)-alkyl oder den Rest R¹⁵ steht;
R^{12b} für R^{12a}-NH steht;
in allen ihren stereoisomeren Formen und Mischungen davon in allen Verhältnissen, sowie ihre physiologisch verträglichen Salze.

14. Verbindungen der Formel I gemäß einem oder mehreren der Ansprüche 1 und 2 bis 13, worin ein für die Gruppe B stehender substituierter Methylenrest oder substituierter Ethylenrest als Substituenten einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl und im Heteroarylrest gegebenenfalls substituiertes Heteroaryl-(C₁-C₄)-alkyl trägt; in allen ihren stereoisomeren Formen und Mischungen davon in allen Verhältnissen, sowie ihre physiologisch verträglichen Salze.

15. Verbindungen der Formel I gemäß einem oder mehreren der Ansprüche 1 und 2 bis 14, worin B für einen unsubstituierten Methylenrest steht oder für einen Methylenrest steht, der durch einen (C₁-C₈)-Alkylrest substituiert ist;
in allen ihren stereoisomeren Formen und Mischungen davon in allen Verhältnissen, sowie ihre physiologisch verträglichen Salze.

16. Verbindungen der Formel I gemäß einem oder mehreren der Ansprüche 1 bis 15, worin R¹ für einen der Reste -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ oder Cyano steht;
in allen ihren stereoisomeren Formen und Mischungen davon in allen Verhältnissen, sowie ihre physiologisch verträglichen Salze.

17. Verfahren zur Herstellung von Verbindungen der Formel I gemäß einem oder mehreren der Ansprüche 1 bis 16, **dadurch gekennzeichnet, daß** man eine Fragmentkondensation einer Verbindung der Formel II 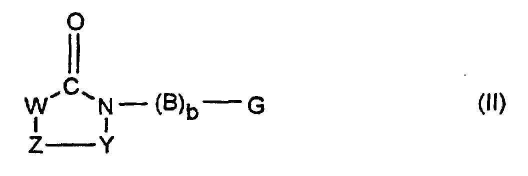 mit einer Verbindung der Formel III 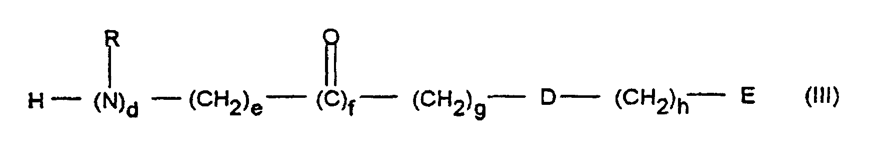 ausführt, wobei W, Y, Z, B, D, E und R sowie b, d, e, f, g und h wie in den Ansprüchen 1 bis 16 angegeben definiert sind und G für Hydroxycarbonyl, (C₁-C₆)-Alkoxycarbonyl, aktivierte Carbonsäurederivate, wie Säurechloride, Aktivester oder gemischte Anhydride, oder für Isocyanato steht.

18. Verbindungen der Formel lb, 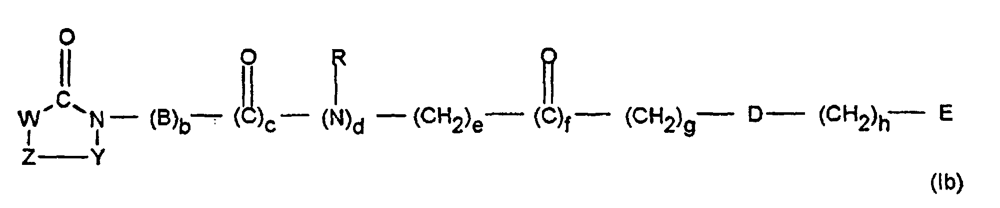 worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₁₂)(C₃-C₁₂)-Cycloalkylen, (C₁-C₆)-Alkylen-(C₃-C₁₂)-cycloalkyl, Phenylen, Phenylen-(C₁-C₆)-alkyl, (C₁-C₆)-Alkylen-phenyl, (C₁-C₆)-Alkylen-phenyl-(C₁-C₆)-alkyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet oder für eine direkte Bindung steht;
B einen zweiwertigen Rest aus der Reihe (C₁-C₆)-Alkylen, (C₂-C₆)-Alkenylen, Phenylen, Phenylen-(C₁-C₃)-alkyl, -alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor ein- oder mehrfach substituiert sein können;
R⁰ für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ einen der Reste -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²² -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
Cyano, Halogen, Nitro oder Methylendioxy bedeutet oder für den Rest eines gegebenenfalls substituierten, 5- bis 14-gliedrigen, mono- oder polycyclischen, gesättigten oder ungesättigten heterocyclischen Rings der Formel 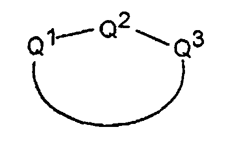 steht, in der
Q¹ für -C(R²¹)₂-, =C(R²¹)-, -N(R²⁸)-, -O- oder -S- steht;
Q² für -S(O)- oder -S(O)₂- steht;
Q³ für -C(R²¹)₂-, =C(R²¹)-, -N(R²⁸)-, -O-, -S-, -C(R²¹)(-)- oder -N(-)- steht,
wobei der heterocyclische Ring über die freie Bindung in den für Q³ stehenden Gruppen -C(R²¹)(-)- oder -N(-)- oder über ein beliebiges anderes Ring-Kohlenstoffatom an die Gruppe A gebunden sein kann und wobei, wenn der heterocyclische Ring an ein in der Gruppe A enthaltenes Ringsystem gebunden ist, der heterocyclische Ring auch über zwei benachbarte Atome an das Ringsystem in der Gruppe A anelliert sein kann;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R⁴, CONHR⁴, CSNHR⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, Het-CO, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und den Rest R⁵ substituiert sein kann;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterozyklus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ für Wasserstoff, R^{12a}, R^{12a}-CO, H-CO, R^{12a}-O-CO, R^{12b}-CO, R^{12b}-CS, R^{12a}-S(O)₂ oder R^{12b}-S(O)₂ steht;
R^{12a} (C₁-C₁₈)-Alkyl, (C₂-C₈)-Alkenyl, (C₂-C₈)-Alkinyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl oder den Rest R¹⁵ bedeutet;
R^{12b} Amino, Di-((C₁-C₁₈)-alkyl)-amino oder R^{12a}-NH bedeutet;
R¹³ Wasserstoff, (C₁-C₆)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl oder (C₃-C₈)-Cycloalkyl-(C₁-C₈)-alkyl bedeutet;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein,zwei, drei oder vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
R²¹ Wasserstoff, (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²¹ bei mehrfachem Auftreten gleich oder verschieden sein können;
R²² (C₁-C₈)-Alkyl, Hydroxy-(C₁-C₈)-alkyl, (C₂-C₈)-Alkenyl, (C₃-C₁₂)-Cycloalkyl, (C₃-C₁₂)-Cycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl oder im Heteroarylrest gegebenenfalls substituiertes Heteroaryl-(C₁-C₈)-alkyl bedeutet, wobei Alkylreste durch Fluor einfach oder mehrfach substituiert sein können und die Reste R²² bei mehrfachem Auftreten gleich oder verschieden sein können;
R²⁸ für einen der Reste R²¹-, R²¹N(R²¹)-, R²¹C(O)-, R²²O-C(O)-, R²¹N(R²¹)-C(O)- oder R²¹N(R²¹)―C(=N(R²¹))― steht;
R²⁹ für einen der Reste R²²-, R²¹N(R²¹)-, R²¹C(O)-, R²²O-C(O)-, R²¹N(R²¹)-C(O)- oder R²¹N(R²¹)―C(=N(R²¹))― steht;
Het für den Rest eines über ein Stickstoffatom gebundenen 5- bis 10-gliedrigen, monocyclischen oder polycyclischen Heterocyclus steht, der aromatisch oder teilweise ungesättigt oder gesättigt sein kann und der ein, zwei, drei oder vier gleiche oder verschiedene zusätzliche Ring-Heteratome aus der Reihe Sauerstoff, Stickstoff und Schwefel enthalten kann und der an Kohlenstoffatomen und an zusätzlichen Ring-Stickstoffatomen gegebenenfalls substituiert sein kann, wobei an zusätzlichen Ring-Stickstoffatomen gleiche oder verschiedene Reste R^{h}, R^{h}CO oder R^{h}O-CO als Substituenten stehen können und R^{h} für Wasserstoff, (C₁-C₈)-Alkyl, (C₃-C₈)-Cycloalkyl, (C₃-C₈)-Cycloalkyl-(C₁-C₈)-alkyl, gegebenenfalls substituiertes (C₆-C₁₄)-Aryl oder im Arylrest gegebenenfalls substituiertes (C₆-C₁₄)-Aryl-(C₁-C₈)-alkyl steht;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze;
zur Verwendung als Arzneimittel.

19. Verbindungen der Formel Ib gemäß Anspruch 18 und/oder ihre physiologisch verträglichen Salze als Mittel zur Entzündungshemmung.

20. Verwendung von Verbindungen der Formel Ib gemäß Anspruch 18 und/oder ihren physiologisch verträglichen Salzen zur Herstellung von Arzneimitteln zur Behandlung oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen.

21. Verwendung von Verbindungen der Formel Ib gemäß Anspruch 18 und/oder ihren physiologisch verträglichen Salzen zur Herstellung von Arzneimitteln zur Behandlung oder Prophylaxe der rheumatoiden Arthritis, der inflammatorischen bowel disease, des systemischen Lupus erythematosus, von inflammatorischen Erkrankungen des zentralen Nervensystems, von Asthma, von Allergien, von cardiovaskulären Erkrankungen, von Arteriosklerose, von Restenosen, von Diabetes, zur Verhinderung der Schädigung von Organtransplantaten, zur Hemmung von Tumorwachstum oder Tumormetastasierung, zur Therapie der Malaria oder zur Entzündungshemmung.

22. Pharmazeutische Zubereitung, **dadurch gekennzeichnet, daß** sie eine oder mehrere Verbindungen der Formel Ib gemäß Anspruch 18 und/oder deren physiologisch verträgliche Salze neben pharmazeutisch einwandfreien Trägerstoffen und/oder Zusatzstoffen enthält.

## Claims

1. A compound of the formula I 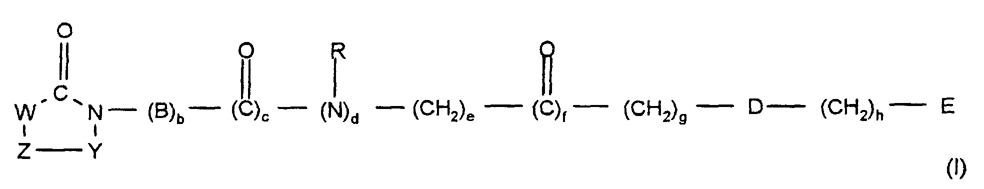 in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁-C₆)-alkylenephenyl-(C₁-C₆)-alkyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur, or is a direct bond;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl, where the bivalent (C₁-C₆)-alkylene radical can be unsubstituted or substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³), N(R³) or CH=C(R³);
E is tetrazolyl, (R⁸O)₂P(O), HOS(O)₂, R⁹NHS(O)₂ or R¹⁰CO;
R is hydrogen, (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be mono- or polysubstituted by fluorine;
R⁰ is hydrogen, (C₁-C₈)-alkyl, (C₃-C₁₂)-cycloalkyl, (C₃-C₁₂)-cycloalkyl-(C₁-C₈)-alkyl, (C₆-C₁₂)-bicycloalkyl, (C₆-C₁₂)-bicycloalkyl-(C₁-C₈)-alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO, optionally substituted (C₆-C₁₄)-aryl-CO, (C₆-C₁₄)-aryl-(C₁-C₈)-alkyl-CO optionally substituted in the aryl radical, optionally substituted heteroaryl-CO, heteroaryl-(C₁-C₈)-alkyl-CO optionally substituted in the heteroaryl radical, (C₁-C₈)-alkyl-S(O)ₙ, (C₃-C₁₂)-cycloalkyl-S(O)ₙ, (C₃-C₁₂)-cycloalkyl-(C₁-C₈)-alkyl-S(O)ₙ, (C₆-C₁₂)-bicycloalkyl-S(O)ₙ, (C₆-C₁₂)-bicycloalkyl-(C₁-C₈)-alkyl-S(O)ₙ, (C₆-C₁₂)-tricycloalkyl-S(O)ₙ, (C₆-C₁₂)-tricycloalkyl-(C₁-C₈)-alkyl-S(O)ₙ, optionally substituted (C₆-C₁₄)-aryl-S(O)ₙ, (C₆-C₁₄)-aryl-(C₁-C₈)-alkyl-S(O)ₙ optionally substituted in the aryl radical, optionally substituted heteroaryl-S(O)ₙ or heteroaryl-(C₁-C₈)-alkyl-S(O)ₙ optionally substituted in the heteroaryl radical, where n is 1 or 2;
R¹ is one of the radicals -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂ -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
cyano, halogen, nitro or methylenedioxy or the radical of an optionally substituted, 5- to 14-membered, mono- or polycyclic, saturated or unsaturated heterocyclic ring of the formula 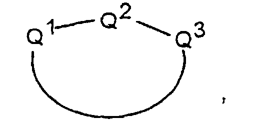 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di((C₁-C₁₈)-alkyl)aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, Het-CO, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl and the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)-arylcarbonyl, (C₆-C₁₄)-aryl-(C₁-C₈)-alkylcarbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di((C₁-C₁₈)-alkyl)amino;
R¹¹ is hydrogen, R^{12a}, R^{12a}-CO, H-CO, R^{12a}-O-CO, R^{12b}-CO, R^{12b}-CS, R^{12a}-S(O)₂ or R^{12b}-S(O)₂;
R^{12a} is (C₁-C₁₈)-alkyl, (C₂-C₈)-alkenyl, (C₂-C₈)-alkynyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical or the radical R¹⁵;
R^{12b} is amino, di((C₁-C₁₈)-alkyl)amino or R^{12a}-NH;
R¹³ is hydrogen, (C₁-C₆)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl or (C₃-C₈)-cycloalkyl-(C₁-C₈)-alkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one, two, three or four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₈)-alkyl, hydroxy-(C₁-C₈)-alkyl, (C₂-C₈)-alkenyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be monosubstituted or polysubstituted by fluorine and the radicals R²¹ can be identical or different if they occur two or more times;
R²² is (C₁-C₈)-alkyl, hydroxy-(C₁-C₈)-alkyl, (C₂-C₈)-alkenyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be monosubstituted or polysubstituted by fluorine and the radicals R²² can be identical or different if they occur two or more times;
R²⁸ is one of the radicals R²¹-, R²¹N(R²¹)-, R²¹C(O)-, R²²O-C(O)-, R²¹N(R²¹)-C(O)- or R²¹N(R²¹)-C(=N(R²¹))-;
R²⁹ is one of the radicals R²²-, R²¹N(R²¹)-, R²¹C(O)-, R²²O-C(O)-, R²¹N(R²¹)-C(O)- or R²¹N(R²¹)-C(=N(R²¹))-;
Het is the radical of a 5- to 10-membered, monocyclic or polycyclic heterocycle bonded via a nitrogen atom, which can be aromatic or partially unsaturated or saturated and which can contain one, two, three or four identical or different additional ring heteroatoms from the group consisting of oxygen, nitrogen and sulfur and which can be optionally substituted on carbon atoms and on additional ring nitrogen atoms, where there can be identical or different radicals R^{h}, R^{h}CO or R^{h}O-CO as substituents on additional ring nitrogen atoms and R^{h} is hydrogen, (C₁-C₈)-alkyl, (C₃-C₈)-cycloalkyl, (C₃ C₈)-cycloalkyl-(C₁-C₈)-alkyl, optionally substituted (C₆-C₁₄)-aryl or (C₆-C₁₄)-aryl-(C₁-C₈)-alkyl optionally substituted in the aryl radical;
b, c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts;
where, however, if simultaneously W is 4-cyanophenyl-C(R¹³), Y is a carbonyl group, Z is NR^{0a}, B is an unsubstituted methylene group, R is R^{a}, b, c and d are 1 and e, f and g are 0, then D cannot be C(R^{2a})(R^{3a}), where
R^{0a}, R^{a} and R^{2a} independently of one another are hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl and
R^{3a} is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl or 2-, 3- or 4-pyridyl.

2. A compound of the formula I as claimed in claim 1, in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁-C₆)-alkylenephenyl-(C₁-C₆)-alkyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur, or is a direct bond;
B is a bivalent radical from the group consisting of (C₁-C₆)-alkylene, (C₂-C₆)-alkenyfene, phenylene, phenylene-(C₁-C₃)-alkyl, (C₁-C₃)-alkylenephenyl;
D is C(R²)(R³), N(R³) or CH=C(R³);
E is tetrazolyl, (R⁸O)₂P(O), HOS(O)₂, R⁹NHS(O)₂ or R¹⁰CO;
R and R⁰ independently of one another are hydrogen, (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be mono- or polysubstituted by fluorine;
R¹ is one of the radicals -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸ -N(R²⁸)-P(O)(OR²¹)₂, -N(R²⁸)-P(O)(OR²¹)-N(R²¹)-R²⁸ -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
cyano, halogen, nitro or methylenedioxy or the radical of an optionally substituted, 5- to 14-membered, mono- or polycyclic, saturated or unsaturated heterocyclic ring of the formula 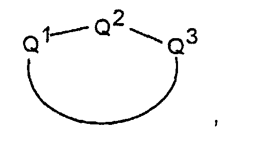 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di((C₁-C₁₈)-alkyl)aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl and the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)-arylcarbonyl, (C₆-C₁₄)-aryl-(C₁-C₈)-alkylcarbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl. (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di((C₁-C₁₈)-alkyl)amino;
R¹¹ is hydrogen, (C₁-C₁₈)-alkyl, R¹²CO, optionally substituted (C₆-C₁₄)-aryl-S(O)₂, (C₁-C₁₈)-alkyl-S(O)₂, (C₆-C₁₄)-aryl-(C₁-C₈)-alkyl optionally substituted in the aryl radical, R⁹NHS(O)₂ or the radical R¹⁵;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di((C₁-C₁₈)-alkyl)amino, the radical R¹⁵ or the radical R¹⁵-O-;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₆)-alkyl, hydroxy-(C₁-C₈)-alkyl, (C₂-C₈)-alkenyl, (C₃-C₁₂)-cycloalkyl, (C₃-C₁₂)-cycloalkyl-(C₁-C₈)-alkyl, optionally substituted (C₆-C₁₄)-aryl (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be monosubstituted or polysubstituted by fluorine and the radicals R²¹ can be identical or different if they occur two or more times;
R²² is (C₁-C₈)-alkyl, hydroxy-(C₁-C₈)-alkyl, (C₂-C₈)-alkenyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be monosubstituted or polysubstituted by fluorine and the radicals R²² can be identical or different if they occur two or more times;
R²⁸ is one of the radicals R²¹-, R²¹N(R²¹)-, R²¹C(O)-, R²²O-C(O)-, R²¹N(R²¹)-C(O)- or R²¹N(R²¹)-C(=N(R²¹))-
R²⁹ is one of the radicals R²²-, R²¹N(R²¹)-, R²¹C(O)-, R²²O-C(O)-, R²¹N(R²¹)-C(O)- or R²¹N(R²¹)-C(=N(R²¹))-;
b, c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts.

3. A compound of the formula I as claimed in claim 1 and/or 2, in which R¹ is one of the radicals
-S(O)-N(R²¹)-R²⁸, -S(O)₂-N(R²¹)-R²⁸, -O-C(O)-R²¹, -O-C(O)-OR²², -O-C(O)-N(R²¹)-R²⁸, -O-C(S)-N(R²¹)-R²⁸, -O-S(O)₂-N(R²¹)-R²⁸, -O-S(O)-N(R²¹)-R²⁸, -N(R²⁹)-C(O)-OR²², -N(R²⁸)-C(S)-R²¹, -N(R²⁸)-C(O)-N(R²¹)-R²⁸, -N(R²⁸)-C(S)-N(R²¹)-R²⁸ -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ or cyano or the radical of an optionally substituted, 5- to 14-membered, mono- or polycyclic, saturated or unsaturated heterocyclic ring of the formula 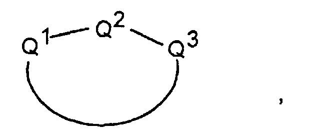 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
in all its stereoisomeric forms and mixtures thereof in all ratios, or its physiologically tolerable salts.

4. A compound of the formula I as claimed in one or more of claims 1 to 3, in which R⁰ is (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, preferably biphenylylmethyl, naphthylmethyl or benzyl, each of which is unsubstituted or monosubstituted or polysubstituted in the aryl radical; in all its stereoisomeric forms and mixtures thereof in all ratios, or its physiologically tolerable salts.

5. A compound of the formula I as claimed in one or more of claims 1, 3 and 4, in which simultaneously
W is R¹-A-CH=C and therein A is a phenylene radical or a
methylenephenyl radical or W is R¹-A-C(R¹³) and therein A is a bivalent radical from the group consisting of methylene, ethylene, trimethylene, tetramethylene, pentamethylene, cyclohexylene, phenylene, phenylenemethyl, methylenephenyl, methylenephenylmethyl;
B is a bivalent radical from the group consisting of methylene, ethylene, trimethylene, tetramethylene, vinylene, phenylene, or substituted methylene or ethylene;
E is R¹⁰CO;
R is hydrogen, (C₁-C₆)-alkyl or benzyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ or cyano or the radical of an optionally substituted, 5- to 14-membered, mono- or polycyclic, saturated or unsaturated heterocyclic ring of the formula 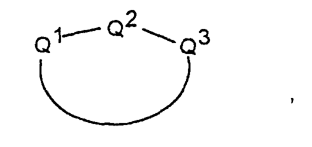 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen or (C₁-C₈)-alkyl;
R³ is (C₁-C₈)-alkyl, optionally substituted (C₆-C₁₄)-aryl, (C₆-C₁₄)-aryl-(C₁-C₈)-alkyl, (C₃-C₈)-cycloalkyl, (C₂-C₈)-alkenyl, (C₂-C₈)-alkynyl, pyridyl,
R¹¹NH, R⁴CO, COOR⁴, CONHR⁴, CSNHR⁴, COOR¹⁵ and CONHR¹⁵;
and e, g and h independently of one another are the numbers 0, 1, 2 or 3;
in all its stereoisomeric forms and mixtures thereof in all ratios, or its physiologically tolerable salts.

6. A compound of the formula I as claimed in one or more of claims 1 to 5, in which W is R¹-A-C(R¹³) and R¹³ is (C₁-C₆)-alkyl, (C₆-C₁₄)-aryl-(C₁-C₈)-alkyl optionally substituted in the aryl radical or (C₃-C₈)-cycloalkyl;
in all its stereoisomeric forms and mixtures thereof in all ratios, or its physiologically tolerable salts.

7. A compound of the formula I as claimed in one or more of claims 1 and 3 to 6, in which R³ is optionally substituted (C₆-C₁₄)-aryl, COOR⁴, R¹¹NH or CONHR⁴, where -NHR⁴ is the radical of an α-amino acid, its ω-amino-(C₂-C₈)-alkylamide, its (C₁-C₈)-alkyl ester, its (C₆-C₁₄)-aryl-(C₁-C₄)-alkyl ester or its derivative in which the carboxylic acid group is converted into the group Het-CO, preferably the radical of the α-amino acids valine, lysine, phenylglycine, phenylalanine or tryptophan or their (C₁-C₈)-alkyl esters, (C₆-C₁₄)-aryl-(C₁-C₄)-alkyl esters or Het-CO derivative;
in all its stereoisomeric forms and mixtures thereof in all ratios, or its physiologically tolerable salts.

8. A compound of the formula I as claimed in one or more of claims 1 and 3 to 7, in which simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl or methylenephenylmethyl;
B is an unsubstituted or substituted methylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ or cyano;
R² is hydrogen;
R³ is the radical CONHR⁴;
R⁴ is methyl which is substituted by hydroxycarbonyl and a radical from the group consisting of (C₁-C₄)-alkyl, phenyl and benzyl, or is methyl which is substituted by (C₁-C₈)-alkoxycarbonyl, preferably (C₁-C₄)-alkoxycarbonyl, and a radical from the group consisting of (C₁-C₄)-alkyl, phenyl and benzyl, or is methyl which is substituted by Het-CO and a radical from the group consisting of (C₁-C₄)-alkyl, phenyl and benzyl;
R¹⁰ is hydroxyl or (C₁-C₈)-alkoxy, preferably (C₁-C₄)-alkoxy;
R¹³ is (C₁-C₆)-alkyl, (C₃-C₇)-cycloalkyl or benzyl, in particular methyl;
b, c and d are 1 and e, f and g are 0;
h is 1 or 2, preferably 1;
in all its stereoisomeric forms and mixtures thereof in all ratios, or its physiologically tolerable salts.

9. A compound of the formula I as claimed in one or more of claims 1, 3, 4 and 5, in which simultaneously
W is R¹-A-CH=C and therein A is a phenylene radical or a methylenephenyl radical or W is R¹-A-C(R¹³) and therein A is a bivalent radical from the group consisting of methylene, ethylene, trimethylene, tetramethylene, pentamethylene, cyclohexylene, phenylene, phenylenemethyl, methylenephenyl, methylenephenylmethyl;
B is a bivalent radical from the group consisting of methylene, ethylene, trimethylene, tetramethylene, vinylene, phenylene or substituted methylene or ethylene;
E is R¹⁰CO;
R is hydrogen or (C₁-C₆)-alkyl;
R⁰ is (C₁-C₈)-alkyl, (C₃-C₈)-cycloalkyl, optionally substituted (C₆-C₁₄)-aryl
or (C₆-C₁₄)-aryl-(C₁-C₈)-alkyl optionally substituted in the aryl radical;
R¹ is one of the radicals -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ or cyano or the radical of an optionally substituted, 5- to 14-membered, mono- or polycyclic, saturated or unsaturated heterocyclic ring of the formula 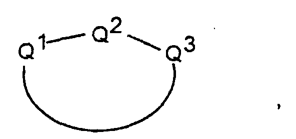 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen or (C₁-C₈)-alkyl;
R³ is CONHR¹⁵ or CONHR⁴, where R⁴ herein is a (C₁-C₈)-alkyl radical which is unsubstituted or substituted by one or more (C₆-C₁₄)-aryl radicals;
R¹⁵ is R¹⁶-(C₁-C₆)-alkyl or R¹⁶, where R¹⁶ is a 7- to 12-membered bridged bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo, and in particular R¹⁵ is an adamantyl radical or an adamantylmethyl radical;
and e, g and h independently of one another are the numbers 0, 1, 2 or 3 and b, c and d are 1;
in all its stereoisomeric forms and mixtures thereof in all ratios, or its physiologically tolerable salts.

10. A compound of the formula I as claimed in one or more of claims 1, 3, 4, 5, 6 and 9, in which simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl or methylenephenylmethyl;
B is an unsubstituted or substituted methylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ or cyano;
R² is hydrogen;
R³ is CONHR¹⁵ or CONHR⁴, where R⁴ herein is a (C₁-C₆)-alkyl radical
which is unsubstituted or substituted by one or more (C₆-C₁₀)-aryl radicals;
R¹⁰ is hydroxyl or (C₁-C₈)-alkoxy, preferably (C₁-C₄)-alkoxy;
R¹³ is (C₁-C₆)-alkyl, (C₃-C₇)-cycloalkyl or benzyl, in particular methyl;
R¹⁵ is an adamantyl radical or an adamantylmethyl radical;
b, c and d are 1 and e, f and g are 0;
h is 1 or 2, preferably 1;
in all its stereoisomeric forms and mixtures thereof in all ratios, or its physiologically tolerable salts.

11. A compound of the formula I as claimed in one or more of claims 1 and 3 to 6, in which simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl, methylenephenylmethyl;
B is an unsubstituted or substituted methylene radical or ethylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl which is optionally substituted in the aryl radical;
R¹ is one of the radicals -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸,
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ or cyano or the radical of an optionally substituted, 5- to 14-membered, mono- or polycyclic, saturated or unsaturated heterocyclic ring of the formula 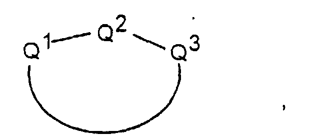 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen;
R³ is an unsubstituted phenyl radical or naphthyl radical, a phenyl radical or naphthyl radical substituted by one, two or three identical or different radicals from the group consisting of (C₁-C₄)-alkyl, (C₁-C₄)-alkoxy, hydroxyl, halogen, trifluoromethyl, nitro, methylenedioxy, ethylenedioxy, hydroxycarbonyl, (C₁-C₄)-alkoxycarbonyl, aminocarbonyl, cyano, phenyl, phenoxy and benzyloxy, a pyridyl radical, a (C₁-C₄)-alkyl radical, a (C₂-C₄)-alkenyl radical, a (C₂-C₄)-alkynyl radical or a (C₅-C₆)-cycloalkyl radical, and in particular R³ is an unsubstituted or substituted phenyl radical or naphthyl radical;
R¹⁰ is hydroxyl or (C₁-C₈)-alkoxy, in particular (C₁-C₄)-alkoxy, and preferably R¹⁰ is a radical from the group consisting of hydroxyl, methoxy, ethoxy, propoxy and isopropoxy;
R¹³ is (C₁-C₆)-alkyl, (C₃-C₇)-cycloalkyl or benzyl, in particular methyl;
b, c and d are 1 and e, f and g are 0;
h is 1 or 2, preferably 1;
in all its stereoisomeric forms and mixtures thereof in all ratios, or its physiologically tolerable salts.

12. A compound of the formula I as claimed in one or more of claims 1 and 3 to 6, in which simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl, methylenephenyl, methylenephenylmethyl;
B is an unsubstituted or substituted methylene radical or ethylene radical;
D is C(R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is one of the radicals -S(O)-N(R²¹)-R²⁸, -S(O)₂-N(R²¹)-R²⁸,
-O-C(O)-R²¹, -O-C(O)-OR²², -O-C(O)-N(R²¹)-R²⁸, -O-C(S)-N(R²¹)-R²⁸,
-O-S(O)₂-N(R²¹)-R²⁸, -O-S(O)-N(R²¹)-R²⁸,
-N(R²⁹)-C(O)-OR²², -N(R²⁸)-C(S)-R²¹,
-N(R²⁸)-C(O)-N(R²¹)-R²⁸, -N(R²⁸)-C(S)-N(R²¹)-R²⁸
-N(R²⁸)-S(O)₂-R²², -N(R²⁸)-S(O)-R²², -N(R²⁸)-S(O)₂-OR²¹, -N(R²⁸)-S(O)-OR²¹,
-N(R²⁸)-S(O)₂-N(R²¹)-R²⁸, -N(R²⁸)-S(O)-N(R²¹)-R²⁸,
-C(S)-R²¹, -C(S)-N(R²¹)-R²⁸ or cyano or the radical of an optionally substituted, 5- to 14-membered, mono- or polycyclic, saturated or unsaturated heterocyclic ring of the formula 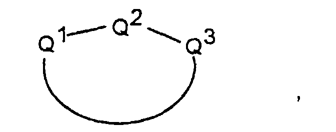 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-,
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen;
R³ is R¹¹NH;
R¹⁰ is hydroxyl or (C₁-C₈)-alkoxy, in particular (C₁-C₄)-alkoxy, and
preferably R¹⁰ is a radical from the group consisting of hydroxyl, methoxy, ethoxy, propoxy and isopropoxy;
R¹³ is (C₁-C₆)-alkyl, (C₃-C₇)-cycloalkyl or benzyl, in particular methyl;
b, c, d and e are 1 and f and g are 0;
h is 0;
in all its stereoisomeric forms and mixtures thereof in all ratios, or its physiologically tolerable salts.

13. A compound of the formula I as claimed in one or more of claims 1, 3, 4, 5, 6 and 12, in which
R¹¹ is R^{12a}, R^{12a}-CO, R^{12a}-O-CO, R^{12b}-CO, R^{12b}-CS or R^{12a}-S(O)₂;
R^{12a} is (C₁-C₁₀)-alkyl, (C₂-C₈)-alkenyl, (C₂-C₈)-alkynyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical or the radical R¹⁵;
R^{12b} is R^{12a}-NH;
in all its stereoisomeric forms and mixtures thereof in all ratios, or its physiologically tolerable salts.

14. A compound of the formula I as claimed in one or more of claims 1 and 2 to 13, in which a substituted methylene radical or substituted ethylene radical representing the group B carries as a substituent a radical from the group consisting of (C₁-C₈)-alkyl, (C₂-C₆)-alkenyl, (C₂-C₆)-alkynyl, (C₃-C₈)-cycloalkyl, in particular (C₅-C₆)-cycloalkyl, (C₃-C₈)-cycloalkyl-(C₁-C₄)-alkyl, in particular (C₅-C₆)-cycloalkyl-(C₁-C₄)-alkyl, optionally substituted (C₆-C₁₀)-aryl, (C₆-C₁₀)-aryl-(C₁-C₄)-alkyl optionally substituted in the aryl radical, optionally substituted heteroaryl and
heteroaryl-(C₁-C₄)-alkyl optionally substituted in the heteroaryl radical; in all its stereoisomeric forms and mixtures thereof in all ratios, or its physiologically tolerable salts.

15. A compound of the formula I as claimed in one or more of claims 1 and 2 to 14, in which B is an unsubstituted methylene radical or a methylene radical which is substituted by a (C₁-C₈)-alkyl radical;
in all its stereoisomeric forms and mixtures thereof in all ratios, or its physiologically tolerable salts.

16. A compound of the formula I as claimed in one or more of claims 1 to 15, in which R¹ is one of the radicals -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ or cyano;
in all its stereoisomeric forms and mixtures thereof in all ratios, or its physiologically tolerable salts.

17. A process for the preparation of compounds of the formula I as claimed in one or more of claims 1 to 16, which comprises carrying out a fragment condensation of a compound of the formula II 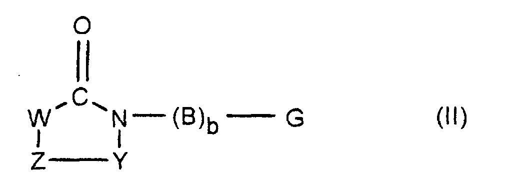 with a compound of the formula III 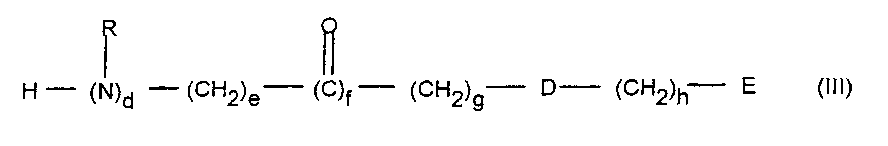 where W, Y, Z, B, D, E and R, and b, d, e, f, g and h are defined as indicated in claims 1 to 16 and G is hydroxycarbonyl, (C₁-C₆)-alkoxycarbonyl, activated carboxylic acid derivatives, such as acid chlorides, active esters or mixed anhydrides, or isocyanato.

18. A compound of the formula Ib 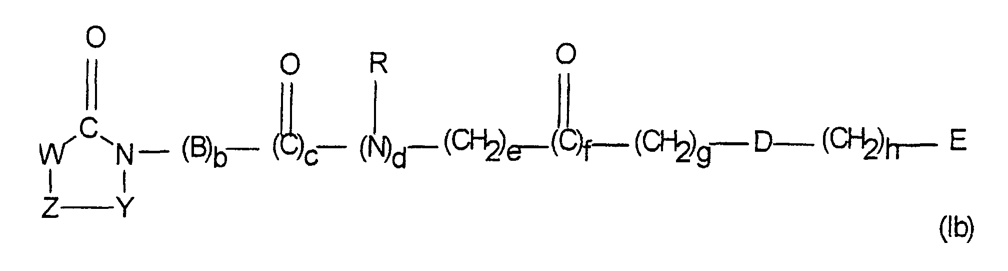 in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₁₂)-cycloalkylene, (C₁-C₆)-alkylene-(C₃-C₁₂)-cycloalkyl, phenylene, phenylene-(C₁-C₆)-alkyl, (C₁-C₆)-alkylenephenyl, (C₁-C₆)-alkylenephenyl-(C₁-C₆)-alkyl, phenylene-(C₂-C₆)-alkenyl or a bivalent radical of a 5- or 6-membered, saturated or unsaturated ring which can contain 1 or 2 nitrogen atoms and can be mono- or disubstituted by (C₁-C₆)-alkyl or doubly bonded oxygen or sulfur or is a direct bond;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl, where the bivalent (C₁-C₆)-alkylene radical can be unsubstituted or substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³), N(R³) or CH=C(R³);
E is tetrazolyl, (R⁸O)₂P(O), HOS(O)₂, R⁹NHS(O)₂ or R¹⁰CO;
R is hydrogen, (C₁-C₈)-alkyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be mono- or polysubstituted by fluorine;
R⁰ is hydrogen, (C₁-C₈)-alkyl, (C₃-C₁₂)-cycloalkyl, (C₃-C₁₂)-cycloalkyl-(C₁-C₈)-alkyl, (C₆-C₁₂)-bicycloalkyl, (C₆-C₁₂)-bicycloalkyl-(C₁-C₈)-alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO, optionally substituted (C₆-C₁₄)-aryl-CO, (C₆-C₁₄)-aryl-(C₁-C₈)-alkyl-CO optionally substituted in the aryl radical, optionally substituted heteroaryl-CO, heteroaryl-(C₁-C₈)-alkyl-CO optionally substituted in the heteroaryl radical, (C₁-C₈)-alkyl-S(O)ₙ, (C₃-C₁₂)-cycloalkyl-S(O)ₙ, (C₃-C₁₂)-cycloalkyl-(C₁-C₈)-alkyl-S(O)ₙ, (C₆-C₁₂)-bicycloalkyl-S(O)ₙ, (C₆-C₁₂)-bicycloalkyl-(C₁-C₈)-alkyl-S(O)ₙ, (C₆-C₁₂)-tricycloalkyl-S(O)ₙ, (C₆-C₁₂)-tricycloalkyl-(C₁-C₈)-alkyl-S(O)ₙ, optionally substituted (C₆-C₁₄)-aryl-S(O)ₙ, (C₆-C₁₄)-aryl-(C₁-C₈)-alkyl-S(O)ₙ optionally substituted in the aryl radical, optionally substituted heteroaryl-S(O)ₙ or heteroaryl-(C₁-C₈)-alkyl-S(O)ₙ optionally substituted in the heteroaryl radical, where n is 1 or 2;
R¹ is one of the radicals -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)-R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O)₂-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)-R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R^{28,} -N(R²⁸)-P(O)(OR²¹)₂, -N(R²⁸)-P(O)(OR²¹)-N(R²¹)-R²⁸ -N(R²⁸)-P(O)(N(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²²)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸,
cyano, halogen, nitro or methylenedioxy or the radical of an optionally substituted, 5- to 14-membered, mono- or polycyclic, saturated or unsaturated heterocyclic ring of the formula 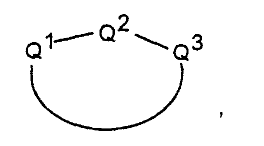 in which
Q¹ is -C(R²¹)₂-, =C(R²¹)-, -N(R²⁸)-, -O- or -S-;
Q² is -S(O)- or -S(O)₂-;
Q³ is -C(R²¹)₂-, =C(R²¹)-, -N(R²⁸)-, -O-, -S-, -C(R²¹)(-)- or -N(-)-
where the heterocyclic ring can be bonded to the group A via the free bond in the groups -C(R²¹)(-)- or -N(-)- representing Q³ or via any other desired ring carbon atom and where, if the heterocyclic ring is bonded to a ring system contained in the group A, the heterocyclic ring can also be fused in the group A via two adjacent atoms on the ring system;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R⁴, CONHR⁴, CSNHR⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di((C₁-C₁₈)-alkyl)aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, Het-CO, optionally substituted (C₃-C₈)-cycloalkyl, HOS(O)₂-(C₁-C₃)-alkyl, R⁹NHS(O)₂-(C₁-C₃)-alkyl, (R⁸O)₂P(O)-(C₁-C₃)-alkyl, tetrazolyl-(C₁-C₃)-alkyl, halogen, nitro, trifluoromethyl and the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)-arylcarbonyl, (C₆-C₁₄)-aryl-(C₁-C₈)-alkylcarbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di((C₁-C₁₈)-alkyl)amino;
R¹¹ is hydrogen, R^{12a}, R^{12a}-CO, H-CO, R^{12a}-O-CO, R^{12b}-CO, R^{12b}-CS, R^{12a}-S(O)₂ or R^{12b}-S(O)₂;
R^{12a} is (C₁-C₁₈)-alkyl, (C₂-C₈)-alkenyl, (C₂-C₈)-alkynyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical or the radical R¹⁵;
R^{12b} is amino, di((C₁-C₁₈)-alkyl)-amino or R^{12a}-NH;
R¹³ is hydrogen, (C₁-C₆)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl or (C₃-C₈)-cycloalkyl-(C₁-C₈)-alkyl;
R¹⁵ is R¹⁶-(C₁-C₆)-alkyl or R¹⁶;
R¹⁶ is a 6- to 24-membered, bicyclic or tricyclic radical which is saturated or partially unsaturated and which can also contain one, two, three or four identical or different heteroatoms from the group consisting of nitrogen, oxygen and sulfur and which can also be substituted by one or more identical or different substituents from the group consisting of (C₁-C₄)-alkyl and oxo;
R²¹ is hydrogen, (C₁-C₈)-alkyl, hydroxy-(C₁-C₈)-alkyl, (C₂-C₈)-alkenyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be monosubstituted or polysubstituted by fluorine and the radicals R²¹ can be identical or different if they occur two or more times;
R²² is (C₁-C₈)-alkyl, hydroxy-(C₁-C₈)-alkyl, (C₂-C₈)-alkenyl, (C₃-C₁₂)-cycloalkyl, (C₃-C₁₂)-cycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl or heteroaryl-(C₁-C₈)-alkyl optionally substituted in the heteroaryl radical, where alkyl radicals can be monosubstituted or polysubstituted by fluorine and the radicals R²² can be identical or different if they occur two or more times;
R²⁸ is one of the radicals R²¹-, R²¹N(R²¹)-, R²¹C(O)-, R²²O-C(O)-, R²¹N(R²¹)-C(O)- or R²¹N(R²¹)-C(=N(R²¹))-;
R²⁹ is one of the radicals R²²-, R²¹N(R²¹)-, R²¹C(O)-, R²²O-C(O)-, R²¹N(R²¹)-C(O)- or R²¹N(R²¹)-C(=N(R²¹))-;
Het is the radical of a 5- to 10-membered, monocyclic or polycyclic heterocycle bonded via a nitrogen atom, which can be aromatic or partially unsaturated or saturated and which can contain one, two, three or four identical or different additional ring heteroatoms from the group consisting of oxygen, nitrogen and sulfur and which can be optionally substituted on carbon atoms and on additional ring nitrogen atoms, where there can be identical or different radicals R^{h}, R^{h}CO or R^{h}O-CO as substituents on additional ring nitrogen atoms and R^{h} is hydrogen, (C₁-C₈)-alkyl, (C₃-C₈)-cycloalkyl, (C₃-C₈)-cycloalkyl-(C₁-C₈)-alkyl, optionally substituted (C₆-C₁₄)-aryl or (C₆-C₁₄)-aryl-(C₁-C₈)-alkyl optionally substituted in the aryl radical;
b, c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts;
for use as a pharmaceutical.

19. A compound of the formula lb as claimed in claim 18 and/or its physiologically tolerable salts as an agent for the suppression of inflammation.

20. The use of compounds of the formula Ib as claimed in claim 18 and/or their physiologically tolerable salts for the production of pharmaceuticals for the treatment or prophylaxis of illnesses in which leucocyte adhesion and/or leucocyte migration exhibits an undesired extent, or of illnesses in which VLA-4-dependent adhesion processes play a part.

21. The use of compounds of the formula lb as claimed in claim 18 and/or their physiologically tolerable salts for the production of pharmaceuticals for the treatment or prophylaxis of rheumatoid arthritis, inflammatory bowel disease, systemic lupus erythematosus, inflammatory disorders of the central nervous system, asthma, allergies, cardiovascular disorders, arteriosclerosis, restenoses, diabetes, for the prevention of damage to organ transplants, for the inhibition of tumor growth or tumor metastasis, for the therapy of malaria or for the inhibition of inflammation.

22. A pharmaceutical preparation which comprises one or more compounds of the formula lb as claimed in claim 18 and/or their physiologically tolerable salts in addition to pharmaceutically innocuous excipients and/or additives.

## Revendications

1. Composés de formule I 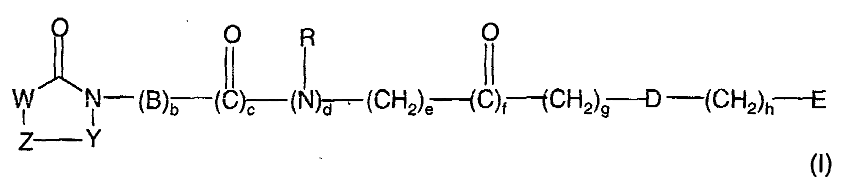 où
W représente R¹-A-C(R¹³) ou R¹-A-CH=C ;
Y représente un groupe carbonyle, thiocarbonyle ou méthylène ;
Z représente N(R⁰), l'oxygène, le soufre ou un groupe méthylène ;
A représente un reste divalent de la série alkylène en C₁-C₆, cycloalkylène en C₃₋C₁₂, alkylène en C₁₋C₆-cycloalkyle en C₃-C₁₂, phénylène, phénylène-alkyle en C₁-C₆, alkylène en C₁-C₆-phényle, alkylène en C₁-C₆-phényl-alkyle en C₁-C₆, phénylène-alcényle en C₂-C₆ ou un reste divalent d'un cycle saturé ou insaturé à 5 ou 6 chaînons, qui peut contenir 1 ou 2 atomes d'azote et qui peut être substitué 1 ou 2 fois par alkyle en C₁-C₆ ou l'oxygène ou le soufre doublement lié, ou représente une liaison directe ;
B représente un reste divalent de la série alkylène en C₁-C₆, alcénylène en C₂-C₆, phénylène, phénylène-alkyle en C₁-C₃, alkylène en C₁-C₃-phényle, où le reste alkylène en C₁-C₆ divalent peut être non substitué ou substitué par un reste de la série alkyle en C₁-C₈, alcényle en C₂-C₈, alcynyle en C₂-C₈, cycloalkyle en C₃-C₁₀, cycloalkyle en C₃-C₁₀-alkyle en C₁-C₆, aryle en C₆-C₁₄ éventuellement substitué, aryle en C₆-C₁₄-alkyle en C₁-C₆ éventuellement substitué dans la partie aryle, hétéroaryle éventuellement substitué et hétéroaryl-alkyle en C₁-C₆ éventuellement substitué dans le reste hétéroaryle ;
D représente C(R²)(R³), N(R³) ou CH=C(R³) ;
E représente tétrazolyle, (R⁸O)₂P(O), HOS(O)₂, R⁹NHS(O)₂ ou R¹⁰CO ;
R représente l'hydrogène, alkyle en C₁-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où les restes alkyle peuvent être substitués une ou plusieurs fois par le fluor ;
R⁰ représente l'hydrogène, alkyle en C₁-C₈, cycloalkyle en C₃-C₁₂, cycloalkyle en C₃-C₁₂-alkyle en C₁-C₈, bicycloalkyle en C₆-C₁₂, bicycloalkyle en C₆-C₁₂-alkyle en C₁-C₈, tricycloalkyle en C₆-C₁₂, tricycloalkyle en C₆-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué, hétéroaryl-alkyle en C₁-C₈ éventuellement substitué dans le reste hétéroaryle, CHO, alkyle en C₁-C₈-CO, cycloalkyle en C₃-C₁₂-CO, cycloalkyle en C₃-C₁₂-alkyle en C₁-C₈-CO, bicycloalkyle en C₆-C₁₂-CO, bicycloakyle en C₆-C₁₂-alkyle en C₁-C₈-CO, tricycloalkyle en C₆-C₁₂-CO, tricycloalkyle en C₆-C₁₂-alkyle en C₁-C₈-CO, aryle en C₆-C₁₄-CO éventuellement substitué, aryle en C₆-C₁₄-alkyle en C₁-C₈-CO éventuellement substitué dans le reste aryle, hétéroaryl-CO éventuellement substitué, hétéroaryl-alkyle en C₁-C₈-CO éventuellement substitué dans le reste hétéroaryle, alkyle en C₁-C₈-S(O)ₙ, cycloalkyle en C₃-C₁₂-S(O)ₙ, cycloalkyle en C₃-C₁₂-alkyle en C₁-C₈-S(O)ₙ, bicycloalkyle en C₆-C₁₂-S(O)ₙ, bicycloalkyle en C₆-C₁₂-alkyle en C₁-C₈-S(O)ₙ, tricycloalkyle en C₆-C₁₂-S(O)ₙ, tricycloalkyle en C₆-C₁₂-alkyle en C₁-C₈-S(O)ₙ, aryle en C₆-C₁₄-S(O)ₙ éventuellement substitué, aryle en C₆-C₁₄-alkyle en C₁-C₈-S(O)ₙ éventuellement substitué dans le reste aryle, hétéroaryl-S(O)ₙ éventuellement substitué, ou hétéroaryl-alkyle en C₁-C₈-S(O)ₙ éventuellement substitué dans le reste aryle, où n représente 1 ou 2 ;
R¹ représente l'un des restes -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)R²⁸, -S(O)₂-N(R²¹)-R²⁸, -S-C(O)-R²¹, -S-C(O)-OR²², -S-C(S)-SR²², -S-C(O)- N(R²¹)-R²⁸, -S-C(S)-N(R²¹)-R²⁸, -O-C(O)-R²¹, -O-C(S)-R²¹, -O-C(O)- OR²², -O-C(O)-N(R²¹)-R²⁸, -O-C(S)-N(R²¹)-R²⁸, -O-S(O₂)-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S-(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N)(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²¹)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸, cyano, halogène, nitro ou méthylènedioxy ou représente le reste d'un cycle hétérocyclique mono- ou polycyclique, saturé ou insaturé à 5 à 14 chaînons, éventuellement substitué, de formule 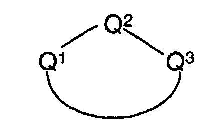
où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène, alkyle en C₁-C₈, aryle en C₆-C₁₄ éventuellement substitué, aryle en C₆-C₁₄-alkyle en C₁-C₈ éventuellement substitué dans le reste aryle ou cycloalkyle en C₃-C₈ ;
R³ représente l'hydrogène, alkyle en C₁-C₈, aryle en C₆-C₁₄ éventuellement substitué, aryle en C₆-C₁₄-alkyle en C₁-C₈ éventuellement substitué dans le reste aryle, cycloakyle en C₃-C₈, alcényle en C₂-C₈, alcynyle en C₂-C₈, alcényle en C₂-C₈-carbonyle, alcynyle en C₂-C₈-carbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵;
R⁴ représente l'hydrogène ou alkyle en C₁-C₂₈, qui peut éventuellement être substitué une ou plusieurs fois par des restes identiques ou différents de la série hydroxyle, hydroxycarbonyle, aminocarbonyle, mono- ou di(alkyle en C₁-C₁₈)aminocarbonyle, aminoalkyle en C₂-C₈-aminocarbonyle, aminoalkyle en C₁-C₃-phényl-alkyle en C₁-C₃-aminocarbonyle, alkyle en C₁-C₁₈-carbonylamino-alkyle en C₁-C₃-phényl-alkyle en C₁-C₃-aminocarbonyle, alkyle en C₁-C₁₈-carbonylamino-alkyle en C₂-C₁₈-aminocarbonyle, aryle en C₆-C₁₄-alcoxy en C₁-C₈-carbonyle, qui peut aussi être substitué dans le reste aryle, amino, mercapto, alcoxy en C₁-C₁₈, alcoxy en C₁-C₁₈-carbonyle, Het-CO, cycloalkyle en C₃-C₈ éventuellement substitué, HOS(O)₂-alkyle en C₁-C₃, R⁹NHS(O)₂-alkyle en C₁-C₃, (R⁸O)₂P(O)-alkyle en C₁-C₃, tétrazolyl-alkyle en C₁-C₃, halogène, nitro, trifluorométhyle et le reste R⁵ ;
R⁵ représente aryle en C₆-C₁₄ éventuellement substitué, aryle en C₆-C₁₄-alkyle en C₁-C₈ éventuellement substitué dans le reste aryle, un cycle hétérocyclique mono- ou bicyclique à 5 à 12 chaînons qui peut être aromatique, partiellement hydrogéné ou totalement hydrogéné, qui peut contenir 2 ou 3 hétéroatomes identiques ou différents de la série de l'azote, de l'oxygène et du soufre, un reste R⁶ ou un reste R⁶CO-, où le reste aryle et, indépendamment de celui-ci, le reste hétérocyclique peuvent être substitués ou une plusieurs fois par des restes identiques ou différents de la série alkyle en C₁-C₁₈, alcoxy en C₁-C₁₈, halogène, nitro, amino ou trifluorométhyle ;
R⁶ représente R⁷R⁸N, R⁷O ou R⁷S ou une chaîne latérale d'aminoacide, un reste naturel ou non naturel d'aminoacide, d'iminoacide, d'azaaminoacide N-alkylé par alkyle en C₁-C₈ ou N-alkylé par aryle en C₆-C₁₄-alkyle en C₁-C₈ ou un reste de dipeptide qui peut aussi être substitué dans la partie aryle et/ou dans lequel la liaison peptidique peut être réduite en -NH-CH₂-, ainsi que leurs esters et amides, où l'hydrogène ou hydroxyméthyle peut éventuellement remplacer des groupes fonctionnels libres et/ou des groupes fonctionnels libres peuvent être protégés par des groupes protecteurs courants en chimie des peptides ;
R⁷ représente l'hydrogène, alkyle en C₁-C₁₈, aryle en C₆-C₁₄-alkyle en C₁-C₈, alkyle en C₁-C₁₈-carbonyle, alcoxy en C₁-C₁₈-carbonyle, aryle en C₆-C₁₄-carbonyle, aryle en C₆-C₁₄-alkyle en C₁-C₈-carbonyle ou aryle en C₆-C₁₄-alkyle en C₁-C₁₈-oxycarbonyle, où les groupes alkyle peuvent éventuellement être substitués par un groupe amino et/ou les restes aryle peuvent éventuellement être substitués une ou plusieurs fois, de préférence une fois, par des restes identiques ou différents de la série alkyle en C₁-C₈, alcoxy en C₁-C₈, halogène, nitro, amino et trifluorométhyle, un reste naturel ou non naturel d'aminoacide, d'iminoacide, d'azaaminoacide éventuellement N-alkylé par alkyle en C₁-C₈ ou N-alkylé par aryle en C₆-C₁₄-alkyle en C₁-C₈ ou un reste de dipeptide, qui peut aussi être substitué dans le reste aryle et/ou dans lequel la liaison peptidique peut être réduite en -NH-CH₂- ;
R⁸ représente l'hydrogène, alkyle en C₁-C₁₈, aryle en C₆-C₁₄ éventuellement substitué ou aryle en C₆-C₁₄-alkyle en C₁-C₈ qui peut aussi être substitué dans le reste aryle ;
R⁹ représente l'hydrogène, aminocarbonyle, alkyle en C₁-C₁₈-aminocarbonyle, cycloalkyle en C₃-C₈-aminocarbonyle, aryle en C₆-C₁₄-aminocarbonyle éventuellement substitué, alkyle en C₁-C₁₈, aryle en C₆-C₁₄ éventuellement substitué ou cycloalkyle en C₃-C₈ ;
R¹⁰ représente hydroxyle, alcoxy en C₁-C₁₈, aryle en C₆-C₁₄-alcoxy en C₁-C₈, qui peut aussi être substitué dans le reste aryle, aryloxy en C₆-C₁₄ éventuellement substitué, amino ou mono- ou di-(alkyle en C₁-C₁₈)-amino ;
R¹¹ représente l'hydrogène, R^{12a}, R^{12a}-CO, H-CO, R^{12a}-O-CO, R^{12b}-CO, R^{12b}-CS, R^{12a}-S(O)₂ ou R^{12b}-S(O)₂ ;
R^{12a} représente alkyle en C₁-C₁₈, alcényle en C₂-C₈, alcynyle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué, hétéroaryl-alkyle en C₁-C₈ éventuellement substitué dans le reste hétéroaryle ou le reste R¹⁵ ;
R^{12b} représente amino, di-(alkyle en C₁-C₁₈)amino ou R^{12a}-NH ;
R¹³ représente l'hydrogène, alkyle en C₁-C₆, aryle en C₆-C₁₄ éventuellement substitué, aryle en C₆-C₁₄-alkyle en C₁-C₈ éventuellement substitué dans le reste aryle, cycloakyle en C₃-C₈ ou cycloalkyle en C₃-C₈-alkyle en C₁-C₈ ;
R¹⁵ représente R¹⁶-alkyle en C₁-C₆ ou le reste R¹⁶ ;
R¹⁶ représente un reste bicyclique ou tricyclique à 6 à 24 chaînons qui est saturé ou partiellement insaturé et qui peut aussi contenir 1, 2, 3 ou 4 hétéroatomes identiques ou différents de la série de l'azote, de l'oxygène et du soufre et qui peut aussi être substitué par un ou plusieurs substituants identiques ou différents de la série alkyle en C₁-C₄ et oxo ;
R²¹ représente l'hydrogène, alkyle en C₁-C₈, hydroxyalkyle en C₁-C₈, alcényle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où des restes alkyle peuvent être substitués une ou plusieurs fois par le fluor et les restes R²¹, quand ils apparaissent plusieurs fois, peuvent être identiques ou différents ;
R²² représente alkyle en C₁-C₈, hydroxyalkyle en C₁-C₈, alcényle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où des restes alkyle peuvent être substitués une ou plusieurs fois par le fluor et les restes R²², quand ils apparaissent plusieurs fois, peuvent être identiques ou différents ;
R²⁸ représente l'un des restes R²¹-, R²¹N(R²¹)-, R²¹C(O)-, R²²O-C(O)-, R²¹N(R²¹)-C(O)- ou R²¹N(R²¹)-C(=N(R²¹)) ;
R²⁹ représente l'un des restes R²²-, R²¹N(R²¹)-, R²¹C(O)-, R²²O-C(O)-, R²¹N(R²¹)-C(O)- ou R²¹N(R²¹)-C(=N(R²¹))- ;
Het représente le reste d'un hétérocycle monocyclique ou polycyclique à 5 à 10 chaînons lié par le biais d'un atome d'azote, qui peut être aromatique ou partiellement insaturé ou saturé et qui peut contenir 1, 2, 3 ou 4 hétéroatomes cycliques supplémentaires identiques ou différents de la série de l'oxygène, de l'azote et du soufre et qui peut éventuellement être substitué sur des atomes de carbone et sur des atomes d'azote cycliques supplémentaires, où des restes R^{h}, R^{h}CO ou R^{h}O-CO identiques ou différents peuvent être situés comme substituants sur des atomes d'azote cycliques supplémentaires et R^{h} représente l'hydrogène, alkyle en C₁-C₈, cycloalkyle en C₃-C₈, cycloalkyle en C₃-C₈-alkyle en C₁-C₈, aryle en C₆-C₁₄ éventuellement substitué ou aryle en C₆-C₁₄-alkyle en C₁-C₈ éventuellement substitué dans le reste aryle ;
b, c, d et f représentent indépendamment les uns des autres 0 ou 1 mais ne peuvent pas tous représenter simultanément 0 ;
e, g et h représentent indépendamment les uns des autres 0, 1, 2, 3, 4, 5 ou 6 ;
sous toutes leurs formes stéréoisomériques et leurs mélanges en toutes proportions, ainsi que leurs sels physiologiquement acceptables ;
où, cependant, quand, simultanément, W représente 4-cyanophényl -C(R¹³), Y représente un groupe carbonyle, Z représente NR^{0a}, B représente un groupe méthylène non substitué, R représente R^{a}, b, c et d représentent 1 et e, f et g représentent 0, D ne peut pas représenter C(R^{2a})(R^{3a}), où
R^{0a}, R^{a} et R^{2a} représentent indépendamment les uns des autres l'hydrogène, alkyle en C₁-C₈, aryle en C₆-C₁₄ éventuellement substitué, aryle en C₆-C₁₄-alkyle en C₁-C₈ éventuellement substitué dans le reste aryle ou cycloakyle en C₃-C₈ et
R^{3a} représente l'hydrogène, alkyle en C₁-C₈, aryle en C₆-C₁₄ éventuellement substitué, aryle en C₆-C₁₄-alkyle en C₁-C₈ éventuellement substitué dans le reste aryle, cycloakyle en C₃-C₈ ou 2-, 3- ou 4-pyridyle.

2. Composés de formule I selon la revendication 1 où
W représente R¹-A-C(R¹³) ou R¹-A-CH=C ;
Y représente un groupe carbonyle, thiocarbonyle ou méthylène ;
Z représente N(R⁰), l'oxygène, le soufre ou un groupe méthylène ;
A représente un reste divalent de la série alkylène en C₁₋C₆, cycloalkylène en C₃₋C₁₂, alkylène en C₁₋C₆-cycloalkyle en C₃-C₁₂, phénylène, phénylène-alkyle en C₁-C₆, alkylène en C₁-C₆-phényle, alkylène en C₁-C₆-phényl-alkyle en C₁-C₆, phénylène-alcényle en C₂-C₆ ou un reste divalent d'un cycle saturé ou insaturé à 5 ou 6 chaînons, qui peut contenir 1 ou 2 atomes d'azote et qui peut être substitué 1 ou 2 fois par alkyle en C₁-C₆ ou l'oxygène ou le soufre doublement lié, ou représente une liaison directe ;
B représente un reste divalent de la série alkylène en C₁-C₆, alcénylène en C₂-C₆, phénylène, phénylène-alkyle en C₁-C₃, alkylène en C₁-C₃-phényle ;
D représente C(R²)(R³), N(R³) ou CH=C(R³) ;
E représente tétrazolyle, (R⁸O)₂P(O), HOS(O)₂, R⁹NHS(O)₂ ou R¹⁰CO ;
R et R⁰ représentent indépendamment l'un de l'autre l'hydrogène, alkyle en C₁-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où les restes alkyle peuvent être substitués une ou plusieurs fois par le fluor ;
R¹ représente l'un des restes -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O₂)-OR²¹, -O-S(O)-OR²¹, -O-S(O)₂-N(R²¹)-R^{28,} -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S-(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N)(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²¹)₂, -P(O)(OR²¹)_{2,} -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸, cyano, halogène, nitro ou méthylènedioxy ou représente le reste d'un cycle hétérocyclique mono- ou polycyclique, saturé ou insaturé à 5 à 14 chaînons, éventuellement substitué, de formule 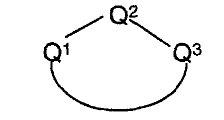
où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène, alkyle en C₁-C₈, aryle en C₆-C₁₄ éventuellement substitué, aryle en C₆-C₁₄-alkyle en C₁-C₈ éventuellement substitué dans le reste aryle ou cycloalkyle en C₃-C₈ ;
R³ représente l'hydrogène, alkyle en C₁-C₈, aryle en C₆-C₁₄ éventuellement substitué, aryle en C₆-C₁₄-alkyle en C₁-C₈ éventuellement substitué dans le reste aryle, cycloakyle en C₃-C₈, alcényle en C₂-C₈, alcynyle en C₂-C₈, alcényle en C₂-C₈-carbonyle, alcynyle en C₂-C₈-carbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵ ;
R⁴ représente l'hydrogène ou alkyle en C₁-C₂₈, qui peut éventuellement être substitué une ou plusieurs fois par des restes identiques ou différents de la série hydroxyle, hydroxycarbonyle, aminocarbonyle, mono- ou di(alkyle en C₁-C₁₈)aminocarbonyle, aminoalkyle en C₂-C₈-aminocarbonyle, aminoalkyle en C₁-C₃-phényl-alkyle en C₁-C₃-aminocarbonyle, alkyle en C₁-C₁₈-carbonylamino-alkyle en C₁-C₃-phényl-alkyle en C₁-C₃-aminocarbonyle, alkyle en C₁-C₁₈-carbonylamino-alkyle en C₂-C₁₈-aminocarbonyle, aryle en C₆-C₁₄-alcoxy en C₁-C₈-carbonyle, qui peut aussi être substitué dans le reste aryle, amino, mercapto, alcoxy en C₁-C₁₈, alcoxy en C₁-C₁₈-carbonyle, cycloalkyle en C₃-C₈ éventuellement substitué, HOS(O)₂-alkyle en C₁-C₃, R⁹NHS(O)₂-alkyle en C₁-C₃, (R⁸O)₂P(O)-alkyle en C₁-C₃, tétrazolylalkyle en C₁-C₃, halogène, nitro, trifluorométhyle et le reste R⁵ ;
R⁵ représente aryle en C₆-C₁₄ éventuellement substitué, aryle en C₆-C₁₄-alkyle en C₁-C₈ éventuellement substitué dans le reste aryle, un cycle hétérocyclique mono- ou bicyclique à 5 à 12 chaînons qui peut être aromatique, partiellement hydrogéné ou totalement hydrogéné et qui peut contenir 1, 2 ou 3 hétéroatomes identiques ou différents de la série de l'azote, de l'oxygène et du soufre, un reste R⁶ ou un reste R⁶CO-, où le reste aryle et, indépendamment de celui-ci, le reste hétérocyclique peuvent être substitués ou une plusieurs fois par des restes identiques ou différents de la série alkyle en C₁-C₁₈, alcoxy en C₁-C₁₈, halogène, nitro, amino ou trifluorométhyle ;
R⁶ représente R⁷R⁸N, R⁷O ou R⁷S ou une chaîne latérale d'aminoacide, un reste naturel ou non naturel d'aminoacide, d'iminoacide, d'azaaminoacide éventuellement N-alkylé par alkyle en C₁-C₈ ou N-alkylé par aryle en C₆-C₁₄-alkyle en C₁-C₈ ou un reste de dipeptide qui peut aussi être substitué dans la partie aryle et/ou dans lequel la liaison peptidique peut être réduite en -NH-CH₂-, ainsi que leurs esters et amides, où l'hydrogène ou hydroxyméthyle peut éventuellement remplacer des groupes fonctionnels libres et/ou des groupes fonctionnels libres peuvent être protégés par des groupes protecteurs courants en chimie des peptides ;
R⁷ représente l'hydrogène, alkyle en C₁-C₁₈, aryle en C₆-C₁₄-alkyle en C₁-C₈, alkyle en C₁-C₁₈-carbonyle, alcoxy en C₁-C₁₈-carbonyle, aryle en C₆-C₁₄-carbonyle, aryle en C₆-C₁₄-alkyle en C₁-C₈-carbonyle ou aryle en C₆-C₁₄-alkyle en C₁-C₁₈-oxycarbonyle, où les groupes alkyle peuvent éventuellement être substitués par un groupe amino et/ou les restes aryle peuvent éventuellement être substitués une ou plusieurs fois, de préférence une fois, par des restes identiques ou différents de la série alkyle en C₁-C₈, alcoxy en C₁-C₈, halogène, nitro, amino et trifluorométhyle, un reste naturel ou non naturel d'aminoacide, d'iminoacide, d'azaaminoacide éventuellement N-alkylé par alkyle en C₁-C₈ ou N-alkylé par aryle en C₆-C₁₄-alkyle en C₁-C₈ ou un reste de dipeptide, qui peut aussi être substitué dans le reste aryle et/ou dans lequel la liaison peptidique peut être réduite en -NH-CH₂-;
R⁸ représente l'hydrogène, alkyle en C₁-C₁₈, aryle en C₆-C₁₄ éventuellement substitué ou aryle en C₆-C₁₄-alkyle en C₁-C₈ qui peut aussi être substitué dans le reste aryle ;
R⁹ représente l'hydrogène, aminocarbonyle, alkyle en C₁-C₁₈-aminocarbonyle, cycloalkyle en C₃-C₈-aminocarbonyle, aryle en C₆-C₁₄-aminocarbonyle éventuellement substitué, alkyle en C₁-C₁₈, aryle en C₆-C₁₄ éventuellement substitué ou cycloalkyle en C₃-C₈ ;
R¹⁰ représente hydroxyle, alcoxy en C₁-C₁₈, aryle en C₆-C₁₄-alcoxy en C₁-C₈, qui peut aussi être substitué dans le reste aryle, aryloxy en C₆-C₁₄ éventuellement substitué, amino ou mono- ou di-(alkyle en C₁-C₁₈)-amino ;
R¹¹ représente l'hydrogène, alkyle en C₁-C₁₈, , R¹²CO, aryle en C₆-C₁₄-S(O)₂ éventuellement substitué, alkyle en C₁-C₁₈-S(O)₂, aryle en C₆-C₁₄-alkyle en C₁-C₈ éventuellement substitué dans le reste aryle, R⁹NHS(O)₂ ou le reste R¹⁵ ;
R¹² représente l'hydrogène, alkyle en C₁-C₁₈, alcényle en C₂-C₈, alcynyle en C₂-C₈, aryle en C₆-C₁₄ éventuellement substitué, alcoxy en C₁-C₁₈, aryle en C₆-C₁₄-alcoxy en C₁-C₈ qui peut aussi être substitué dans le reste aryle, aryloxy en C₆-C₁₄, amino ou mono- ou di-(alkyle en C₁-C₁₈)amino, le reste R¹⁵ ou le reste ou le reste R¹⁵-O- ;
R¹³ représente l'hydrogène, alkyle en C₁-C₆, aryle en C₆-C₁₄-alkyle en C₁-C₈ éventuellement substitué dans le reste aryle ou cycloakyle en C₃-C₈ ;
R¹⁵ représente R¹⁶-alkyle en C₁-C₆ ou le reste R¹⁶ ;
R¹⁶ représente un reste bicyclique ou tricyclique à 6 à 24 chaînons qui est saturé ou partiellement insaturé et qui peut aussi contenir 1 à 4 hétéroatomes identiques ou différents de la série de l'azote, de l'oxygène et du soufre et qui peut aussi être substitué par un ou plusieurs substituants identiques ou différents de la série alkyle en C₁-C₄ et oxo ;
R²¹ représente l'hydrogène, alkyle en C₁-C₈, hydroxyalkyle en C₁-C₈, alcényle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où des restes alkyle peuvent être substitués une ou plusieurs fois par le fluor et les restes R²¹, quand ils apparaissent plusieurs fois, peuvent être identiques ou différents ;
R²² représente alkyle en C₁-C₈, hydroxyalkyle en C₁-C₈, alcényle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où des restes alkyle peuvent être substitués une ou plusieurs fois par le fluor et les restes R²², quand ils apparaissent plusieurs fois, peuvent être identiques ou différents ;
R²⁸ représente l'un des restes R²¹-, R²¹N(R²¹)-, R²¹C(O)-, R²²O-C(O)-, R²¹N(R²¹)-C(O)- ou R²¹N(R²¹)-C(=N(R²¹)) ;
R²⁹ représente l'un des restes R²²-, R²¹N(R²¹)-, R²¹C(O)-, R²²O-C(O)-, R²¹N(R²¹)-C(O)- ou R²¹N(R²¹)-C(=N(R²¹))-
b, c, d et f représentent indépendamment les uns des autres 0 ou 1 mais ne peuvent pas tous représenter simultanément 0 ;
e, g et h représentent indépendamment les uns des autres 0, 1, 2, 3, 4, 5 ou 6 ;
sous toutes leurs formes stéréoisomériques et leurs mélanges en toutes proportions, ainsi que leurs sels physiologiquement acceptables.

3. Composés de formule I selon la revendication 1 et/ou 2 où R¹ représente l'un des restes
-S(O)-N(R²¹)-R²⁸, -S(O)₂-N(R²¹)-R²⁸, -O-C(O)-R²¹, -O-C(O)-OR²², -O-C(O)-N(R²¹)-R²⁸, -O-C(S)-N(R²¹)-R²⁸, -O-S(O)₂-N(R²¹)-R^{28,} -O-S(O)-N(R²¹)-R²⁸, -N(R²⁹)-C(O)-OR²², -N(R²⁸)-C(S)-OR²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -C(S)-R²¹, -C(S)-N(R²¹)-R²⁸ ou cyano ou représente le reste d'un cycle hétérocyclique mono- ou polycyclique, saturé ou insaturé à 5 à 14 chaînons, éventuellement substitué, de formule 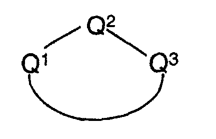 où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
sous toutes leurs formes stéréoisomériques et leurs mélanges en toutes proportions, ainsi que leurs sels physiologiquement acceptables.

4. Composés de formule I selon une ou plusieurs des revendications 1 à 3 où R⁰ représente alkyle en C₁-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, de préférence biphénylylméthyle, naphtylméthyle ou benzyle non substitué ou substitué une fois ou plusieurs fois dans le reste aryle ;
sous toutes leurs formes stéréoisomériques et leurs mélanges en toutes proportions, ainsi que leurs sels physiologiquement acceptables.

5. Composés de formule I selon une ou plusieurs des revendications 1, 3 et 4 où simultanément
W représente R¹-A-CH=C et dans celui-ci A représente un reste phénylène ou un reste méthylènephényle ou W représente R¹-A-C(R¹³) et dans celui-ci A représente un reste divalent de la série méthylène, éthylène, triméthylène, tétraméthylène, pentaméthylène, cyclohexylène, phénylène, phénylèneméthyle, méthylènephényle, méthylènephénylméthyle ;
B représente un reste divalent de la série méthylène, éthylène, triméthylène, tétraméthylène, vinylène, phénylène ou représente méthylène ou éthylène substitué ;
E représente R¹⁰CO ;
R représente l'hydrogène, alkyle en C₁-C₆ ou benzyle ;
R⁰ représente alkyle en C₁-C₈, cycloalkyle en C₃-C₈, aryle en C₆-C₁₄ éventuellement substitué ou aryle en C₆-C₁₄-alkyle en C₁-C₈ éventuellement substitué dans le reste aryle ;
R¹ représente l'un des restes
-S(O)-N(R²¹)-R²⁸, -S(O)₂-N(R²¹)-R²⁸, -O-C(O)-R²¹, -O-C(O)-OR²², -O-C(O)-N(R²¹)-R²⁸, -O-C(S)-N(R²¹)-R²⁸, -O-S(O)₂-N(R²¹)-R^{28,} -O-S(O)-N(R²¹)-R²⁸, -N(R²⁹)-C(O)-OR²², -N(R²⁸)-C(S)-OR²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -C(S)-R²¹, -C(S)-N(R²¹)-R²⁸ ou cyano ou représente le reste d'un cycle hétérocyclique mono- ou polycyclique, saturé ou insaturé à 5 à 14 chaînons, éventuellement substitué, de formule 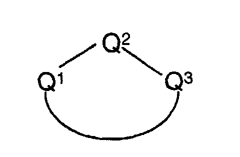 où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène ou alkyle en C₁-C₈;
R³ représente alkyle en C₁-C₈, aryle en C₆-C₁₄ éventuellement substitué, aryle en C₆-C₁₄-alkyle en C₁-C₈, cycloakyle en C₃-C₈, alcényle en C2-C₈, alcynyle en C₂-C₈, pyridyle, R¹¹NH, R⁴CO, COOR⁴, CONHR⁴, CSNHR⁴, COOR¹⁵ et CONHR¹⁵ ;
et e, g et h représentent indépendamment les uns des autres les nombres 0, 1, 2 ou 3 ;
sous toutes leurs formes stéréoisomériques et leurs mélanges en toutes proportions, ainsi que leurs sels physiologiquement acceptables.

6. Composés de formule I selon une ou plusieurs des revendications 1 à 5 où W représente R¹-A-C(R¹³) et R¹³ représente alkyle en C₁-C₆, aryle en C₆-C₁₄-alkyle en C₁-C₈ éventuellement substitué dans le reste aryle ou cycloakyle en C₃-C₈;
sous toutes leurs formes stéréoisomériques et leurs mélanges en toutes proportions, ainsi que leurs sels physiologiquement acceptables.

7. Composés de formule I selon une ou plusieurs des revendications 1 à 3 où R³ représente aryle en C₆-C₁₄ éventuellement substitué, COOR⁴, R¹¹NH ou CONHR⁴, où -NHR⁴ représente le reste d'un α-aminoacide, son ω-amino-alkyle en C₂-C₈-amide, son ester d'alkyle en C₁-C₈, son aryle en C₆-C₁₄-alkyle en C₁-C₄-ester ou son dérivé où le groupe acide carboxylique est converti en le groupe Het-CO, de préférence le reste des α-aminoacides valine, lysine, phénylglycine, phénylalanine ou tryptophane ou de leur ester d'alkyle en C₁-C₈, de leur aryle en C₆-C₁₄-alkyle en C₁-C₄-ester ou de leur dérivé Het-CO ;
sous toutes leurs formes stéréoisomériques et leurs mélanges en toutes proportions, ainsi que leurs sels physiologiquement acceptables.

8. Composés de formule I selon une ou plusieurs des revendications 1 et 3 à 7 où simultanément
W représente R¹-A-C(R¹³) ;
Y représente un groupe carbonyle ;
Z représente N(R⁰) ;
A représente éthylène, triméthylène, tétraméthylène, pentaméthylène, cyclohexylène, phénylène, phénylèneméthyle, méthylènephényle ou méthylènephénylméthyle ;
B représente un reste méthylène non substitué ou substitué ;
D représente C(R²)(R³) ;
E représente R¹⁰CO ;
R représente l'hydrogène ou alkyle en C₁-C₄, en particulier l'hydrogène, méthyle ou éthyle ;
R⁰ représente alkyle en C₁-C₈, cycloalkyle en C₃-C₈, aryle en C₆-C₁₄ éventuellement substitué ou aryle en C₆-C₁₄-alkyle en C₁-C₈ éventuellement substitué dans le reste aryle ;
R¹ représente l'un des restes -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ ou cyano ;
R² représente l'hydrogène ;
R³ représente le reste CONHR⁴ ;
R⁴ représente méthyle, qui est substitué par hydroxycarbonyle et un reste de la série alkyle en C₁-C₄, phényle et benzyle, ou représente méthyle, qui est substitué par alcoxy en C₁-C₈-carbonyle, de préférence alcoxy en C₁-C₄-carbonyle, et un reste de la série alkyle en C₁-C₄, phényle et benzyle, ou représente méthyle, qui est substitué par Het-CO et un reste de la série alkyle en C₁-C₄, phényle et benzyle ;
R¹⁰ représente hydroxyle ou alcoxy en C₁-C₈, de préférence alcoxy en C₁-C₄;
R¹³ représente alkyle en C₁-C₆, cycloalkyle en C₃-C₇ ou benzyle, en particulier méthyle ;
b, c et d représentent 1 et e, f et g représentent 0 ;
h représente 1 ou 2, de préférence 1 ;
sous toutes leurs formes stéréoisomériques et leurs mélanges en toutes proportions, ainsi que leurs sels physiologiquement acceptables.

9. Composés de formule I selon une ou plusieurs des revendications 1, 3, 4 et 5 où simultanément
W représente R¹-A-CH=C et dans celui-ci A représente un reste phénylène ou un reste méthylènephényle ou W représente R¹-A-C(R¹³) et dans celui-ci A représente un reste divalent de la série méthylène, éthylène, triméthylène, tétraméthylène, pentaméthylène, cyclohexylène, phénylène, phénylèneméthyle, méthylènephényle, méthylènephénylméthyle ;
B représente un reste divalent de la série méthylène, éthylène, triméthylène, tétraméthylène, vinylène, phénylène ou représente méthylène ou éthylène substitué ;
E représente R¹⁰CO ;
R représente l'hydrogène ou alkyle en C₁-C₆ ;
R⁰ représente alkyle en C₁-C₈, cycloalkyle en C₃-C₈, aryle en C₆-C₁₄ éventuellement substitué ou aryle en C₆-C₁₄-alkyle en C₁-C₈ éventuellement substitué dans le reste aryle ;
R¹ représente l'un des restes -S(O)-N(R²¹)-R²⁸, -S(O)₂-N(R²¹)-R²⁸, -O-C(O)-R²¹, -O-C(O)-OR²², -O-C(O)-N(R²¹)-R²⁸, -O-C(S)-N(R²¹)-R²⁸, -O-S(O)₂-N(R²¹)-R^{28,} -O-S(O)-N(R²¹)-R²⁸, -N(R²⁹)-C(O)-OR²², -N(R²⁸)-C(S)-OR²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -C(S)-R²¹, -C(S)-N(R²¹)-R²⁸ ou cyano ou représente le reste d'un cycle hétérocyclique mono- ou polycyclique, saturé ou insaturé à 5 à 14 chaînons, éventuellement substitué, de formule 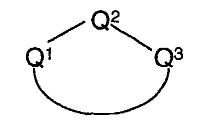 où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène ou alkyle en C₁-C₈ ;
R³ représente CONHR¹⁵ ou CONHR⁴, où R⁴ représente un reste alkyle en C₁-C₈ non substitué ou substitué par un ou plusieurs restes aryle en C₆-C₁₄ ;
R¹⁵ représente R¹⁶-alkyle en C₁-C₆ ou le reste R¹⁶, où R¹⁶ représente un reste bicyclique ou tricyclique ponté à 7 à 12 chaînons qui est saturé ou partiellement insaturé et qui peut aussi contenir 1 à 4 hétéroatomes identiques ou différents de la série de l'azote, de l'oxygène et du soufre et qui peut aussi être substitué par un ou plusieurs substituants identiques ou différents de la série alkyle en C₁-C₄ et oxo, et en particulier R¹⁵ représente un reste adamantyle ou un reste adamantylméthyle ;
et e, g et h représentent indépendamment les uns des autres les nombres
0, 1, 2 ou 3 et b, c et d représentent 1 ;
sous toutes leurs formes stéréoisomériques et leurs mélanges en toutes proportions, ainsi que leurs sels physiologiquement acceptables.

10. Composés de formule I selon une ou plusieurs des revendications 1, 3, 4, 5, 6 et 9 où simultanément
W représente R¹-A-C(R¹³) ;
Y représente un groupe carbonyle ;
Z représente N(R⁰) ;
A représente éthylène, triméthylène, tétraméthylène, pentaméthylène, cyclohexylène, phénylène, phénylèneméthyle, méthylènephényle ou méthylènephénylméthyle ;
B représente un reste méthylène substitué ou non substitué ;
D représente C(R²)(R³) ;
E représente R¹⁰CO ;
R représente l'hydrogène ou alkyle en C₁-C₄, en particulier l'hydrogène, méthyle ou éthyle ;
R⁰ représente alkyle en C₁-C₈, cycloalkyle en C₃-C₈, aryle en C₆-C₁₄ éventuellement substitué ou aryle en C₆-C₁₄-alkyle en C₁-C₈ éventuellement substitué dans le reste aryle ;
R¹ représente l'un des restes -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ ou cyano ;
R² représente l'hydrogène ;
R³ représente CONHR¹⁵ ou CONHR⁴, où R⁴ représente un reste alkyle en C₁-C₆ non substitué ou substitué par un ou plusieurs restes aryle en C₆-C₁₀ ;
R¹⁰ représente hydroxyle ou alcoxy en C₁-C₈, de préférence alcoxy en C₁-C₄;
R¹³ représente alkyle en C₁-C₆, cycloalkyle en C₃-C₇ ou benzyle, en particulier méthyle ;
R¹⁵ représente un reste adamantyle ou un reste adamantylméthyle ;
b, c et d représentent 1 et e, f et g représentent 0 ;
h représente 1 ou 2, de préférence 1 ;
sous toutes leurs formes stéréoisomériques et leurs mélanges en toutes proportions, ainsi que leurs sels physiologiquement acceptables.

11. Composés de formule I selon une ou plusieurs des revendications 1 et 3 à 6 où simultanément
W représente R¹-A-C(R¹³) ;
Y représente un groupe carbonyle ;
Z représente N(R⁰) ;
A représente éthylène, triméthylène, tétraméthylène, pentaméthylène, cyclohexylène, phénylène, phénylèneméthyle, méthylènephényle, méthylènephénylméthyle ;
B représente un reste méthylène substitué ou non substitué ou un reste éthylène ;
D représente C(R²)(R³) ;
E représente R¹⁰CO ;
R représente l'hydrogène ou alkyle en C₁-C₄, en particulier l'hydrogène, méthyle ou éthyle ;
R⁰ représente alkyle en C₁-C₈, cycloalkyle en C₃-C₈, aryle en C₆-C₁₄ éventuellement substitué ou aryle en C₆-C₁₄-alkyle en C₁-C₈ éventuellement substitué dans le reste aryle ;
R¹ représente l'un des restes -S(O)-N(R²¹)-R²⁸, -S(O)₂-N(R²¹)-R²⁸, -O-C(O)-R²¹, -O-C(O)-OR²², -O-C(O)-N(R²¹)-R²⁸, -O-C(S)-N(R²¹)-R²⁸, -O-S(O)₂-N(R²¹)-R^{28,} -O-S(O)-N(R²¹)-R²⁸, -N(R²⁹)-C(O)-OR²², -N(R²⁸)-C(S)-OR²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -C(S)-R21, -C(S)-N(R²¹)-R²⁸ ou cyano ou représente le reste d'un cycle hétérocyclique mono- ou polycyclique, saturé ou insaturé à 5 à 14 chaînons, éventuellement substitué, de formule 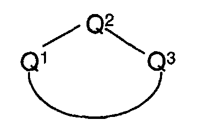 où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène ;
R³ représente un reste phényle ou un reste naphtyle non substitué, un reste phényle ou un reste naphtyle substitué par un deux ou trois restes identiques ou différents de la série alkyle en C₁-C₄, alcoxy en C₁-C₄, hydroxyle, halogène, trifluorométhyle, nitro, méthylènedioxy, éthylènedioxy, hydroxycarbonyle, alcoxy en C₁-C₄-carbonyle, aminocarbonyle, cyano, phényle, phénoxy et benzyloxy, un reste pyridyle, un reste alkyle en C₁-C₄, un reste alcényle en C₂-C₄, un reste alcynyle en C₂-C₄ ou un reste cycloalkyle en C₅-C₆, et en particulier R³ représente un reste phényle ou un reste naphtyle non substitué ou substitué ;
R¹⁰ représente hydroxyle ou alcoxy en C₁-C₈, en particulier alcoxy en C₁-C₄, et de préférence R¹⁰ représente un reste de la série hydroxyle, méthoxy, éthoxy, propoxy et isopropoxy ;
R¹³ représente alkyle en C₁-C₆, cycloalkyle en C₃-C₇ ou benzyle, en particulier méthyle ;
b, c et d représentent 1 et e, f et g représentent 0 ;
h représente 1 ou 2, de préférence 1 ;
sous toutes leurs formes stéréoisomériques et leurs mélanges en toutes proportions, ainsi que leurs sels physiologiquement acceptables.

12. Composés de formule I selon une ou plusieurs des revendications 1 et 3 à 6 où simultanément
W représente R¹-A-C(R¹³) ;
Y représente un groupe carbonyle ;
Z représente N(R⁰) ;
A représente éthylène, triméthylène, tétraméthylène, pentaméthylène, cyclohexylène, phénylène, phénylèneméthyle, méthylènephényle, méthylènephénylméthyle ;
B représente un reste méthylène substitué ou non substitué ou un reste éthylène ;
D représente C(R²)(R³) ;
E représente R¹⁰CO ;
R représente l'hydrogène ou alkyle en C₁-C₄, en particulier l'hydrogène, méthyle ou éthyle ;
R⁰ représente alkyle en C₁-C₈, cycloalkyle en C₃-C₈, aryle en C₆-C₁₄ éventuellement substitué ou aryle en C₆-C₁₄-alkyle en C₁-C₈ éventuellement substitué dans le reste aryle ;
R¹ représente l'un des restes -S(O)-N(R²¹)-R²⁸, -S(O)₂-N(R²¹)-R²⁸, -O-C(O)-R²¹, -O-C(O)-OR²², -O-C(O)-N(R²¹)-R²⁸, -O-C(S)-N(R²¹)-R²⁸, -O-S(O)₂-N(R²¹)-R^{28,} -O-S(O)-N(R²¹)-R²⁸, -N(R²⁹)-C(O)-OR²², -N(R²⁸)-C(S)-OR²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -C(S)-R²¹, -C(S)-N(R²¹)-R²⁸ ou cyano ou représente le reste d'un cycle hétérocyclique mono- ou polycyclique, saturé ou insaturé à 5 à 14 chaînons, éventuellement substitué, de formule 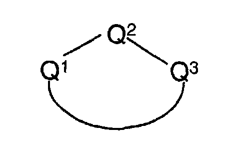 où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène ;
R³ représente R¹¹NH ;
R¹⁰ représente hydroxyle ou alcoxy en C₁-C₈, en particulier alcoxy en C₁-C₄, et de préférence R¹⁰ représente un reste de la série hydroxyle, méthoxy, éthoxy, propoxy et isopropoxy ;
R¹³ représente alkyle en C₁-C₆, cycloalkyle en C₃-C₇ ou benzyle, en particulier méthyle ;
b, c, d et e représentent 1 et f et g représentent 0 ;
h représente 0 ;
sous toutes leurs formes stéréoisomériques et leurs mélanges en toutes proportions, ainsi que leurs sels physiologiquement acceptables.

13. Composé de formule I selon une ou plusieurs des revendications 1, 3, 4, 5, 6 et 12 où
R¹¹ représente R^{12a}, R^{12a}-CO, R^{12a}-O-CO, R^{12b}-CO, R^{12b}-CS ou R^{12a}-S(O)₂ ;
R^{12a} représente alkyle en C₁-C₁₀, alcényle en C₂-C₈, alcynyle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué, hétéroaryl-alkyle en C₁-C₈ éventuellement substitué dans le reste hétéroaryle ou le reste R¹⁵ ;
R^{12b} représente R^{12a}-NH ;
sous toutes leurs formes stéréoisomériques et leurs mélanges en toutes proportions, ainsi que leurs sels physiologiquement acceptables.

14. Composés de formule I selon une ou plusieurs des revendications 1 et 2 à 13 où un reste méthylène substitué ou un reste éthylène substitué représentant le groupe B porte comme substituant un reste de la série alkyle en C₁-C₈, alcényle en C₂-C₆, alcynyle en C₂-C₆, cycloalkyle en C₃-C₈, en particulier cycloalkyle en C₅-C₆, cycloalkyle en C₃-C₈-alkyle en C₁-C₄, en particulier cycloalkyle en C₅-C₆- alkyle en C₁-C₄, aryle en C₆-C₁₀ éventuellement substitué, aryle en C₆-C₁₀-alkyle en C₁-C₄ éventuellement substitué dans le reste aryle, hétéroaryle éventuellement substitué et hétéroaryl-alkyle en C₁-C₄ éventuellement substitué dans le reste hétéroaryle ;
sous toutes leurs formes stéréoisomériques et leurs mélanges en toutes proportions, ainsi que leurs sels physiologiquement acceptables.

15. Composés de formule I selon une ou plusieurs des revendications 1 et 2 à 14 où B représente un reste méthylène non substitué ou un reste méthylène qui est substitué par un reste alkyle en C₁-C₈;
sous toutes leurs formes stéréoisomériques et leurs mélanges en toutes proportions, ainsi que leurs sels physiologiquement acceptables.

16. Composés de formule I selon une ou plusieurs des revendications 1 à 15 où R¹ représente l'un des restes -O-C(O)-R²¹, -O-C(O)-OR²², -O-C(O)-N(R²¹)-R²⁸, -N(R²⁹)-C(O)-OR²², -N(R²⁸)-C(O)-N(R²¹)-R²⁸, -N(R²⁸)-C(S)-N(R²¹)-R²⁸ ou cyano;
sous toutes leurs formes stéréoisomériques et leurs mélanges en toutes proportions, ainsi que leurs sels physiologiquement acceptables.

17. Procédé de préparation de composés de formule I selon une ou plusieurs des revendications 1 à 16 **caractérisé en ce que** l'on réalise une condensation de fragments d'un composé de formule II 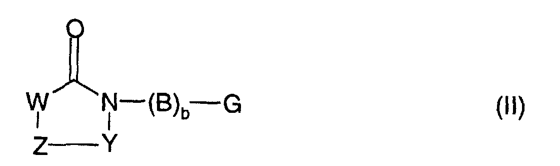 avec un composé de formule III 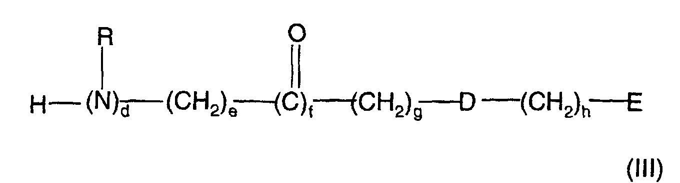 où W, Y, Z, B, D, E et R ainsi que b, d, e, f, g et h sont définis de la manière indiquée dans les revendications 1 à 16 et G représente hydroxycarbonyle, alcoxy en C₁-C₆-carbonyle, des dérivés d'acide carboxylique activés, comme des chlorures d'acide, des esters actifs ou des anhydrides mixtes, ou représente isocyanato.

18. Composés de formule Ib 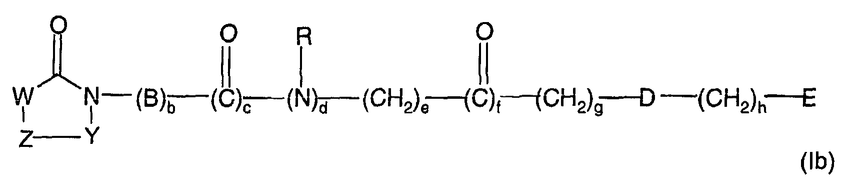 où
W représente R¹-A-C(R¹³) ou R¹-A-CH=C ;
Y représente un groupe carbonyle, thiocarbonyle ou méthylène ;
Z représente N(R⁰), l'oxygène, le soufre ou un groupe méthylène ;
A représente un reste divalent de la série alkylène en C₁-C₆, cycloalkylène en C₃-C₁₂, alkylène en C₁-C₆-cycloalkyle en C₃-C₁₂, phénylène, phénylène-alkyle en C₁-C₆, alkylène en C₁-C₆-phényle, alkylène en C₁-C₆-phényl-alkyle en C₁-C₆, phénylène-alcényle en C₂-C₆ ou un reste divalent d'un cycle saturé ou insaturé à 5 ou 6 chaînons, qui peut contenir 1 ou 2 atomes d'azote et qui peut être substitué 1 ou 2 fois par alkyle en C₁-C₆ ou l'oxygène ou le soufre doublement lié, ou représente une liaison directe ;
B représente un reste divalent de la série alkylène en C₁-C₆, alcénylène en C₂-C₆, phénylène, phénylène-alkyle en C₁-C₃, alkylène en C₁-C₃-phényle, où le reste alkylène en C₁-C₆ divalent peut être non substitué ou substitué par un reste de la série alkyle en C₁-C₈, alcényle en C₂-C₈, alcynyle en C₂-C₈, cycloalkyle en C₃-C₁₀, cycloalkyle en C₃-C₁₀-alkyle en C₁-C₆, aryle en C₆-C₁₄ éventuellement substitué, aryle en C₆-C₁₄-alkyle en C₁-C₆ éventuellement substitué dans la partie aryle, hétéroaryle éventuellement substitué et hétéroaryl-alkyle en C₁-C₆ éventuellement substitué dans le reste hétéroaryle ;
D représente C(R²)(R³), N(R³) ou CH=C(R³) ;
E représente tétrazolyle, (R⁸O)₂P(O), HOS(O)₂, R⁹NHS(O)₂ ou R¹⁰CO ;
R représente l'hydrogène, alkyle en C₁-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où les restes alkyle peuvent être substitués une ou plusieurs fois par le fluor ;
R⁰ représente l'hydrogène, alkyle en C₁-C₈, cycloalkyle en C₃-C₁₂, cycloalkyle en C₃-C₁₂-alkyle en C₁-C₈, bicycloalkyle en C₆-C₁₂, bicycloalkyle en C₆-C₁₂-alkyle en C₁-C₈, tricycloalkyle en C₆-C₁₂, tricycloalkyle en C₆-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué, hétéroaryl-alkyle en C₁-C₈ éventuellement substitué dans le reste hétéroaryle, CHO, alkyle en C₁-C₈-CO, cycloalkyle en C₃-C₁₂-CO, cycloalkyle en C₃-C₁₂-alkyle en C₁-C₈-CO, bicycloalkyle en C₆-C₁₂-CO, bicycloakyle en C₆-C₁₂-alkyle en C₁-C₈-CO, tricycloalkyle en C₆-C₁₂-CO, tricycloalkyle en C₆-C₁₂-alkyle en C₁-C₈-CO, aryle en C₆-C₁₄-CO éventuellement substitué, aryle en C₆-C₁₄-alkyle en C₁-C₈-CO éventuellement substitué dans le reste aryle, hétéroaryl-CO éventuellement substitué, hétéroaryl-alkyle en C₁-C₈-CO éventuellement substitué dans le reste hétéroaryle, alkyle en C₁-C₈-S(O)ₙ, cycloalkyle en C₃-C₁₂-S(O)ₙ, cycloalkyle en C₃-C₁₂-alkyle en C₁-C₈-S(O)ₙ, bicycloalkyle en C₆-C₁₂-S(O)ₙ, bicycloalkyle en C₆-C₁₂-alkyle en C₁-C₈-S(O)ₙ, tricycloalkyle en C₆-C₁₂-S(O)ₙ, tricycloalkyle en C₆-C₁₂-alkyle en C₁-C₈-S(O)ₙ, aryle en C₆-C₁₄-S(O)ₙ éventuellement substitué, aryle en C₆-C₁₄-alkyle en C₁-C₈-S(O)ₙ éventuellement substitué dans le reste aryle, hétéroaryl-S(O)ₙ éventuellement substitué, ou hétéroaryl-alkyle en C₁-C₈-S(O)ₙ éventuellement substitué dans le reste aryle, où n représente 1 ou 2 ;
R¹ représente l'un des restes -S-R²¹, -S-S-R²¹, -S(O)-R²², -S(O)₂-R²², -S-OR²¹, -S(O)-OR²¹, -S(O)₂-OR²¹, -S-N(R²¹)-R²⁸, -S(O)-N(R²¹)R²⁸, -S(O)₂-N(R²¹)-R²⁸, -S-C(O)-R²¹, -S-C(O)-OR²², -S-C(S)-SR²², -S-C(O)-N(R²¹)-R²⁸, -S-C(S)-N(R²¹)-R²⁸, -O-C(O)-R²¹, -O-C(S)-R²¹, -O-C(O)-OR²², -O-C(O)-N(R²¹)-R²⁸, -O-C(S)-N(R²¹)-R²⁸, -O-S(O₂)-OR²¹, -O-S(O)-OR²¹, -O-S(O)₂-N(R²¹)-R²⁸, -O-S(O)-N(R²¹)-R²⁸, -O-S(O)₂-R²², -O-S(O)-R²², -O-P(O)(OR²¹)₂, -O-P(O)(OR²¹)-N(R²¹)-R²⁸, -O-P(O)(N(R²¹)-R²⁸)₂, -N(R²⁹)-C(O)-OR²², -N(R²⁸)-C(O)-SR²², -N(R²⁸)-C(S)-OR²², -N(R²⁸)-C(S)-SR²², -N(R²⁸)-C(S)R²¹, -N(R²⁸)-C(O)-N(R²¹)-R²⁸, -N(R²⁸)-C(S)-N(R²¹)-R²⁸, -N(R²⁸)-S(O)₂-R²², -N(R²⁸)-S-(O)-R²², -N(R²⁸)-S(O)₂-OR²¹, -N(R²⁸)-S(O)-OR²¹, -N(R²⁸)-S(O)₂-N(R²¹)-R²⁸, -N(R²⁸)-S(O)-N(R²¹)-R²⁸, -N(R²⁸)-P(O)(OR²¹)₂, -N(R²⁸)-P(O)(OR²¹)-N(R²¹)-R²⁸, -N(R²⁸)-P(O)(N)(R²¹)-R²⁸)₂, -N(R²⁸)-P(O)(R²²)-OR²¹, -N(R²⁸)-P(O)(R²²)-N(R²¹)-R²⁸, -N(R²⁸)-P(O)(R²¹)₂, -P(O)(OR²¹)₂, -P(O)(OR²¹)-N(R²¹)-R²⁸, -P(O)(N(R²¹)-R²⁸)₂, -P(O)(R²²)-OR²¹, -P(O)(R²²)-N(R²¹)-R²⁸, -P(O)(R²²)₂, -C(S)-R²¹, -C(S)-SR²¹, -C(S)-N(R²¹)-R²⁸, cyano, halogène, nitro ou méthylènedioxy ou représente le reste d'un cycle hétérocyclique mono- ou polycyclique, saturé ou insaturé à 5 à 14 chaînons, éventuellement substitué, de formule 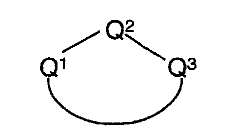
où
Q¹ représente -C(R²¹)₂-, =C(R²¹)-, -N(R²⁸)-, -O- ou -S-,
Q² représente -S(O)- ou -S(O)₂- ;
Q³ représente -C(R²¹)₂-, =C(R²¹)-, -N-(R²⁸)-, -O-, -S-, -C(R²¹)(-)- ou -N(-)-, où le cycle hétérocyclique peut être lié au groupe A par la liaison libre dans les groupes -C(R²¹)(-)- ou -N(-)- représentant Q³ ou par un autre atome de carbone cyclique quelconque et où, quand le cycle hétérocyclique est lié à un système cyclique contenu dans le groupe A, le cycle hétérocyclique peut aussi être condensé au système cyclique dans le groupe A par deux atomes voisins ;
R² représente l'hydrogène, alkyle en C₁-C₈, aryle en C₆-C₁₄ éventuellement substitué, aryle en C₆-C₁₄-alkyle en C₁-C₈ éventuellement substitué dans le reste aryle ou cycloalkyle en C₃-C₈ ;
R³ représente l'hydrogène, alkyle en C₁-C₈, aryle en C₆-C₁₄ éventuellement substitué, aryle en C₆-C₁₄-alkyle en C₁-C₈ éventuellement substitué dans le reste aryle, cycloakyle en C₃-C₈, alcényle en C₂-C₈, alcynyle en C₂-C₈, alcényle en C₂-C₈-carbonyle, alcynyle en C₂-C₈-carbonyle, pyridyle, R¹¹NH, R⁴CO, COOR⁴, CON(CH₃)R⁴, CONHR⁴, CSNHR⁴, COOR¹⁵, CON(CH₃)R¹⁵ ou CONHR¹⁵ ;
R⁴ représente l'hydrogène ou alkyle en C₁-C₂₈, qui peut éventuellement être substitué une ou plusieurs fois par des restes identiques ou différents de la série hydroxyle, hydroxycarbonyle, aminocarbonyle, mono- ou di(alkyle en C₁-C₁₈)aminocarbonyle, aminoalkyle en C₂-C₈-aminocarbonyle, aminoalkyle en C₁-C₃-phényl-alkyle en C₁-C₃-aminocarbonyle, alkyle en C₁-C₁₈-carbonylamino-alkyle en C₁-C₃-phényl-alkyle en C₁-C₃-aminocarbonyle, alkyle en C₁-C₁₈-carbonylamino-alkyle en C₂-C₁₈-aminocarbonyle, aryle en C₆-C₁₄-alcoxy en C₁-C₈-carbonyle, qui peut aussi être substitué dans le reste aryle, amino, mercapto, alcoxy en C₁-C₁₈, alcoxy en C₁-C₁₈-carbonyle, Het-CO, cycloalkyle en C₃-C₈ éventuellement substitué, HOS(O)₂-alkyle en C₁-C₃, R⁹NHS(O)₂-alkyle en C₁-C₃, (R⁸O)₂P(O)-alkyle en C₁-C₃, tétrazolyl-alkyle en C₁-C₃, halogène, nitro, trifluorométhyle et le reste R⁵ ;
R⁵ représente aryle en C₆-C₁₄ éventuellement substitué, aryle en C₆-C₁₄-alkyle en C₁-C₈ éventuellement substitué dans le reste aryle, un cycle hétérocyclique mono- ou bicyclique à 5 à 12 chaînons qui peut être aromatique, partiellement hydrogéné ou totalement hydrogéné, qui peut contenir 2 ou 3 hétéroatomes identiques ou différents de la série de l'azote, de l'oxygène et du soufre, un reste R⁶ ou un reste R⁶CO-, où le reste aryle et, indépendamment de celui-ci, le reste hétérocyclique peuvent être substitués ou une plusieurs fois par des restes identiques ou différents de la série alkyle en C₁-C₁₈, alcoxy en C₁-C₁₈, halogène, nitro, amino ou trifluorométhyle ;
R⁶ représente R⁷R⁸N, R⁷O ou R⁷S ou une chaîne latérale d'aminoacide, un reste naturel ou non naturel d'aminoacide, d'iminoacide, d'azaaminoacide N-alkylé par alkyle en C₁-C₈ ou N-alkylé par aryle en C₆-C₁₄-alkyle en C₁-C₈ ou un reste de dipeptide qui peut aussi être substitué dans la partie aryle et/ou dans lequel la liaison peptidique peut être réduite en -NH-CH₂-, ainsi que leurs esters et amides, où l'hydrogène ou hydroxyméthyle peut éventuellement remplacer des groupes fonctionnels libres et/ou des groupes fonctionnels libres peuvent être protégés par des groupes protecteurs courants en chimie des peptides ;
R⁷ représente l'hydrogène, alkyle en C₁-C₁₈, aryle en C₆-C₁₄-alkyle en C₁-C₈, alkyle en C₁-C₁₈-carbonyle, alcoxy en C₁-C₁₈-carbonyle, aryle en C₆-C₁₄-carbonyle, aryle en C₆-C₁₄-alkyle en C₁-C₈-carbonyle ou aryle en C₆-C₁₄-alkyle en C₁-C₁₈-oxycarbonyle, où les groupes alkyle peuvent éventuellement être substitués par un groupe amino et/ou les restes aryle peuvent éventuellement être substitués une ou plusieurs fois, de préférence une fois, par des restes identiques ou différents de la série alkyle en C₁-C₈, alcoxy en C₁-C₈, halogène, nitro, amino et trifluorométhyle, un reste naturel ou non naturel d'aminoacide, d'iminoacide, d'azaaminoacide éventuellement N-alkylé par alkyle en C₁-C₈ ou N-alkylé par aryle en C₆-C₁₄-alkyle en C₁-C₈ ou un reste de dipeptide, qui peut aussi être substitué dans le reste aryle et/ou dans lequel la liaison peptidique peut être réduite en -NH-CH₂- ;
R⁸ représente l'hydrogène, alkyle en C₁-C₁₈, aryle en C₆-C₁₄ éventuellement substitué ou aryle en C₆-C₁₄-alkyle en C₁-C₈ qui peut aussi être substitué dans le reste aryle ;
R⁹ représente l'hydrogène, aminocarbonyle, alkyle en C₁-C₁₈-aminocarbonyle, cycloalkyle en C₃-C₈-aminocarbonyle, aryle en C₆-C₁₄-aminocarbonyle éventuellement substitué, alkyle en C₁-C₁₈, aryle en C₆-C₁₄ éventuellement substitué ou cycloalkyle en C₃-C₈;
R¹⁰ représente hydroxyle, alcoxy en C₁-C₁₈, aryle en C₆-C₁₄-alcoxy en C₁-C₈, qui peut aussi être substitué dans le reste aryle, aryloxy en C₆-C₁₄ éventuellement substitué, amino ou mono- ou di-(alkyle en C₁-C₁₈)-amino ;
R¹¹ représente l'hydrogène, R^{12a}, R^{12a}-CO, H-CO, R^{12a}-O-CO, R^{12b}-CO, R^{12b}-CS, R^{12a}-S(O)₂ ou R^{12b}-S(O)₂ ;
R^{12a} représente alkyle en C₁-C₁₈, alcényle en C₂-C₈, alcynyle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué, hétéroaryl-alkyle en C₁-C₈ éventuellement substitué dans le reste hétéroaryle ou le reste R¹⁵ ;
R^{12b} représente amino, di-(alkyle en C₁-C₁₈)amino ou R^{12a}-NH ;
R¹³ représente l'hydrogène, alkyle en C₁-C₆, aryle en C₆-C₁₄ éventuellement substitué, aryle en C₆-C₁₄-alkyle en C₁-C₈ éventuellement substitué dans le reste aryle, cycloakyle en C₃-C₈ ou cycloalkyle en C₃-C₈-alkyle en C₁-C₈ ;
R¹⁵ représente R¹⁶-alkyle en C₁-C₆ ou le reste R¹⁶ ;
R¹⁶ représente un reste bicyclique ou tricyclique à 6 à 24 chaînons qui est saturé ou partiellement insaturé et qui peut aussi contenir 1, 2, 3 ou 4 hétéroatomes identiques ou différents de la série de l'azote, de l'oxygène et du soufre et qui peut aussi être substitué par un ou plusieurs substituants identiques ou différents de la série alkyle en C₁-C₄ et oxo ;
R²¹ représente l'hydrogène, alkyle en C₁-C₈, hydroxyalkyle en C₁-C₈, alcényle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où des restes alkyle peuvent être substitués une ou plusieurs fois par le fluor et les restes R²¹, quand ils apparaissent plusieurs fois, peuvent être identiques ou différents ;
R²² représente alkyle en C₁-C₈, hydroxyalkyle en C₁-C₈, alcényle en C₂-C₈, cycloalkyle en C₃-C₁₂, cycloalkyle en C₃-C₁₂-alkyle en C₁-C₈, aryle en C₆-C₁₄ éventuellement substitué, aryle en C₆-C₁₄-alkyle en C₁-C₈ éventuellement substitué dans le reste aryle, hétéroaryle éventuellement substitué ou hétéroaryl-alkyle en C₁-C₈ éventuellement substitué dans le reste hétéroaryle, où des restes alkyle peuvent être substitués une ou plusieurs fois par le fluor et les restes R²², quand ils apparaissent plusieurs fois, peuvent être identiques ou différents ;
R²⁸ représente l'un des restes R²¹-, R²¹N(R²¹)-, R²¹C(O)-, R²²O-C(O)-, R²¹N(R²¹)-C(O)- ou R²¹N(R²¹)-C(=N(R²¹));
R²⁹ représente l'un des restes R²²-, R²¹N(R²¹)-, R²¹C(O)-_{,} R²²O-C(O)-, R²¹N(R²¹)-C(O)- ou R²¹N(R²¹)-C(=N(R²¹))-
Het représente le reste d'un hétérocycle monocyclique ou polycyclique à 5 à 10 chaînons lié par le biais d'un atome d'azote, qui peut être aromatique ou partiellement insaturé ou saturé et qui peut contenir 1, 2, 3 ou 4 hétéroatomes cycliques supplémentaires identiques ou différents de la série de l'oxygène, de l'azote et du soufre et qui peut éventuellement être substitué sur des atomes de carbone et sur des atomes d'azote cycliques supplémentaires, où des restes R^{h}, R^{h}CO ou R^{h}O-CO identiques ou différents peuvent être situés comme substituants sur des atomes d'azote cycliques supplémentaires et R^{h} représente l'hydrogène, alkyle en C₁-C₈, cycloalkyle en C₃-C₈, cycloalkyle en C₃-C₈-alkyle en C₁-C₈, aryle en C₆-C₁₄ éventuellement substitué ou aryle en C₆-C₁₄-alkyle en C₁-C₈ éventuellement substitué dans le reste aryle;
b, c, d et f représentent indépendamment les uns des autres 0 ou 1 mais ne peuvent pas tous représenter simultanément 0 ;
e, g et h représentent indépendamment les uns des autres 0, 1, 2, 3, 4, 5 ou 6 ;
sous toutes leurs formes stéréoisomériques et leurs mélanges en toutes proportions, ainsi que leurs sels physiologiquement acceptables ; destinés à être utilisés comme médicaments.

19. Composés de formule Ib selon la revendication 18 et/ou leurs sels physiologiquement acceptables comme agents pour inhiber les inflammations.

20. Utilisation de composés de formule Ib selon la revendication 18 et/ou de leurs sels physiologiquement acceptables pour la production de médicaments pour le traitement ou la prophylaxie de maladies dans lesquelles l'adhésion des leucocytes et/ou la migration des leucocytes présente une ampleur indésirable, ou de maladies dans lesquelles les processus d'adhésion dépendants de VLA-4 jouent un rôle.

21. Utilisation de composés de formule Ib selon la revendication 18 et/ou de leurs sels physiologiquement acceptables pour la production de médicaments pour le traitement ou la prophylaxie de la polyarthrite rhumatoïde, des affections intestinales inflammatoires non spécifiques, du lupus érythémateux aigu disséminé, des maladies inflammatoires du système nerveux central, de l'asthme, des allergies, des maladies cardiovasculaires, de l'artériosclérose, les resténoses, du diabète, pour éviter une lésion des greffes d'organes, pour inhiber la croissance tumorale ou les métastases tumorales, pour la thérapie de la malaria ou pour inhiber les inflammations.

22. Préparation pharmaceutique **caractérisée en ce qu'**elle contient un ou plusieurs composés de formule Ib selon la revendication 18 et/ou leurs sels physiologiquement acceptables ainsi que des supports et/ou additifs pharmaceutiquement irréprochables.
